(19) Europäisches Patentamt
European Patent Office
Office européen des brevets

(11) EP 4 036 232 A1

(12) EUROPEAN PATENT APPLICATION
published in accordance with Art. 153(4) EPC

(43) Date of publication:
**03.08.2022 Bulletin 2022/31**

(21) Application number: **20866111.6**

(22) Date of filing: **18.09.2020**

(51) International Patent Classification (IPC):
***C12N 15/113*** (2010.01) ***C12N 15/85*** (2006.01)
***A61P 7/04*** (2006.01)

(52) Cooperative Patent Classification (CPC):
**A61P 7/04; C12N 15/113; C12N 15/85**

(86) International application number:
**PCT/CN2020/116227**

(87) International publication number:
**WO 2021/052470 (25.03.2021 Gazette 2021/12)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.09.2019 CN 201910891508**

(71) Applicant: **Ractigen Therapeutics**
**Nantong, Jiangsu 226400 (CN)**

(72) Inventors:
- **LI, Longcheng**
**Nantong City, Jiangsu 226400 (CN)**
- **KANG, Moorim**
**Nantong City, Jiangsu 226400 (CN)**

(74) Representative: **Høiberg P/S**
**Adelgade 12**
**1304 Copenhagen K (DK)**

# (54) NUCLEIC ACID MOLECULE FOR TREATING IMMUNE THROMBOCYTOPENIA AND APPLICATION THEREOF

(57) The present invention relates to a small activating nucleic acid molecules and uses thereof for treating diseases and conditions, such as thrombocytopenia, related to THPO protein deficiency or insufficiency. As described herein, small activating nucleic acid molecules can be double-stranded or single-stranded RNA molecules targeting the promoter region of the *Thpo*/*THPO* gene through an RNA activation mechanism and comprise a first nucleic acid strand and a second nucleic acid strand. The double-stranded RNA molecule targeting the promoter region of the *Thpo*/*THPO* gene comprises two nucleic acid strands of 16 to 35 nucleotides in length, wherein one nucleic acid strand has at least 75% homology or complementarity to a target selected from the promoter region of the *Thpo*/*THPO* gene. The present invention also relates to pharmaceutical compositions and formulations comprising the small activating nucleic acid molecules and methods for up-regulating the expression of the *Thpo*/*THPO* gene in a cell and treating diseases and conditions, related to THPO protein deficiency or insufficiency , by administering small activating nucleic acid molecules, pharmaceutical compositions, and formulations thereof.



Processed by Luminess, 75001 PARIS (FR)

**Description**

TECHNICAL FIELD

The present invention belongs to the technical field of nucleic acids, particularly as it relates to double-stranded nucleic acid molecules associated with gene activation, e.g., small activating nucleic acid molecules, uses of the small activating nucleic acid molecules in activating/up-regulating the transcription of the Thrombopoietin (*THPO, TPO*) gene, and uses thereof for treating diseases and conditions, such as thrombocytopenia, related to THPO protein deficiency or insufficiency.

BACKGROUND

Thrombocyte is one of the blood components in peripheral blood derived from nucleus-free megakaryocytes in the bone marrow (Machlus, Thon, and Italiano 2014). The main function of the thrombocyte is to promote hemostasis, accelerate coagulation, and maintain the integrity of capillary walls, while playing an important role in physiological hemostasis and pathological thrombosis (Laki 1972).

Thrombocytopenia is a disease in which the number of platelets in peripheral blood is abnormally reduced, and characterized clinically by ecchymosis, mucosal bleeding, or intracranial bleeding, all of which endanger the lives of patients with this disease. Thrombocytopenia is considered to be present when platelet concentrations are lower than $100 \times 10^9$/L by direct blood platelet count (Kuter 2009). Spontaneous bleeding, manifesting as subcutaneous purpuraa, occurs frequently when platlet concentrations are lower than $50 \times 10^9$/L. When the platelet concentrations are lower than $20 \times 10^9$/L, a patient's condition can be life-threatening, especially due to trauma or sudden intracranial bleeding, gastrointestinal bleeding, and the like.

Thrombocytopenia can be classified into 3 categories basd on the pathogenesis: myelo-thrombocytopenia, increased peripheral platelet destruction, and splenic sequestration (Seo et al. 2017). Factors causing a reduction in platelets comprise congenital (hereditary) and acquired thrombocytopenia. It has been found currently that hereditary thrombocytopenia can be caused by at least mutations in genes such as *MPL, GP1BA, GP1BB, GP9, MYH9, FLI1, NBEAL2, WAS, GATA1, PRKACG, GFI1b, STIM1, FYB, SLFN14, ETV6, DIAPH1* and *SRC.* The thrombocytopenia caused by acquired factors include thrombocytopenia caused by aplastic anemia, myelodysplastic syndromes, leukemia, drugs, infection, tumor diseases, radiotherapy, bone marrow transplantation, and chronic liver diseases, and immune thrombocytopenia (ITP). Factors causing increased platelet destruction comprise thrombotic thrombocytopenia, heparin-induced thrombocytopenia, drug-induced thrombocytopenia, ITP, and thrombotic thrombocytopenia purpura (TPP) (Hassan and Waller 2015). Thrombocytopenia may also be caused by splenomegaly sequestration, transfusion of banked blood with low platelet content, and the like.

Thrombopoietin (THPO), also known as Megakaryocyte Growth and Development Factor (MGDF) or C-MPL ligand, is a hormone-likeglycoprotein encoded by the human *THPO* gene and mainly produced and secreted by the liver and kidney (de Sauvage et al. 1994). The human *THPO* gene is located on the long arm of chromosome 3 (3q26.3-3q27.2) and encodes a precursor protein containing 353 amino acids and has a molecular weight of 36 kDa. After the removal of the signal peptide containing 21 amino acids, the remaining 332 amino acids are glycosylated to form a 95 kDa glycoprotein which is then released into circulation to bind the C-MPL receptor on megakaryocytes and platelets stimulating the formation and differentiation of megakaryocytes, while promoting thrombopoiesis (Bartley et al. 1994). The mouse *Thpo* gene (also known as *Ml, Tpo, Mgdf, Mpllg*) is homologous to the human *THPO* gene and encodes mouse thrombopoietin. C-MPL was first discovered in mice infected with Myeloproliferative Leukemia Virus (MPLV), and then the human *C-MPL* gene was cloned (Mignotte et al. 1994).

The first-line clinical treatment for thrombocytopenia is primarily intravenous injections of glucocorticoids (high doses of dexamethasone or prednisone) and gamma globulin (IVIg). If first-line therapy fails or cannot be minatained, the second-line therapy such as drug therapy or splenectomy would then be chosen. Drug therapy is the commonly used second-line treatment method at present, because splenectomy is susceptible to infection, long-term relapse, prolonged hospitalization and high death risk (Provan et al. 2010).

The drugs currently used to treat thrombocytopenia are mainly thrombopoietic agents that stimulate megakaryocytes to grow, maturate, and produce platelet by supplementing THPO or simulating the function thereof. Such thrombopoietic agents include two major classes, recombinant human thrombopoietin (rhTHPO) and C-MPL receptor agonist (C-MPL-RA). Initially, peptides with homology to endogenous THPO (first generation thrombopoietic agents) had shown good results in early clinical studies in patients with thrombocytopenia. However, later clinical studies have found that the body can produce neutralizing antibodies that cross-react with endogenous THPO, resulting in persistent thrombocytopenia (Basser 2002; Solberg 2005). In addition, since recombinant THPO produced *in vitro* cannot be completely glycosylated, its thrombopoietic function is limited. Second generation thrombopoietic agents are primarily C-MMPL-RAs, which include Eltrombopag and Romiplostim approved by the FDA in 2008 for the ITP treatment, and Avatrombopag

approved by the FDA and marketed for the treatment of ITP in 2018. C-MPL agonist drugs have some curative effects, however, they have been associated with various complications and withdrawal reactions.

Although the aforementioned drugs have some curative effects by supplementing or mimicking the function of THPO to promote thrombopoiesis or simulate the function thereof, these drugs have certain defects, and all belong to second-line treatments and are all mainly used for treating ITP. In order to effectively treat thrombocytopenia caused by various reasons, there is a need to develop treatment methods based on a novel mechanism.

## SUMMARY

One objective of the present invention is to provide a small activating nucleic acid molecule based on an RNA activation mechanism, which promotes thrombopoiesis or treats a disease and condition related to THPO protein deficiency or insufficiency or thrombocytopenia caused by various reasons, such as drug-induced thrombocytopenia and immune thrombocytopenia, by activating/up-regulating *THPO* gene transcription to increase protein expression of THPO.

Another objective of the present invention is to provide a composition or formulation comprising the small activating nucleic acid molecule.

Another objective of the present invention is to provide a use ofa small activating nucleic acid molecule or a composition or formulation comprising same in the preparation of a drug for activating/up-regulating the expression of the *THPO* gene in a cell.

Yet another objective of the present invention is to provide a method for activating/up-regulating the expression of the *Thpo*/*THPO* gene in a cell.

Still another objective of the present invention is to provide a use of a small activating nucleic acid molecule or a composition or formulation thereof in the preparation of a drug for treating thrombocytopenia and/or a disease or a condition related to THPO protein deficiency or insufficiency, or in a method of treating thrombocytopenia and/or a disease or a condition related to THPO protein deficiency or insufficiency.

Still yet another objective of the present invention is to provide an isolated small activating nucleic acid molecule target site of the *Thpo*/*THPO* gene, wherein the target site comprises or is selected from any continuous sequence of 16 to 35 nucleotides in length in any sequence set forth in SEQ ID NO: 2 to SEQ ID NO: 4 and SEQ ID NO: 601 to SEQ ID NO: 605, or a sequence having at least 75%, e.g., at least about 79%, about 80%, about 85%, about 90%, about 95%, about 99% or 100% homology to a sequence consisting of any of the aforementioned continuous 16 to 35 nucleotides in length.

### Technical Solution

In one aspect of the present invention, a small activating nucleic acid (e.g., a small activating RNA molecule, saRNA) molecule activating/up-regulating the expression of the *Thpo* gene in a mouse cell is provided, wherein one strand of the small activating nucleic acid molecule has at least 75% homology or complementarity to any nucleic acid sequence of 16 to 35 nucleotides in length in a promoter region of a mouse *Thpo* gene, thereby activating or up-regulating the expression of the gene, wherein the promoter region comprises 500 nucleotides upstream of a transcription start site. Specifically, one strand of the small activating nucleic acid molecule comprises or is selected from a nucleic acid sequence having at least 75% (e.g., at least about 79%, about 80%, about 85%, about 90%, about 95%, about 99% or 100%) homology or complementarity to a continuous sequence of 16 to 35 nucleotides in length at positions -493 to -356 (hotspot 1, SEQ ID NO: 2), positions -273 to -183 (hotspot 2, SEQ ID NO: 3), or positions -164 to -80 (hotspot 3, SEQ ID NO: 4) upstream of the transcription start site of the *Thpo* gene promoter. More specifically, one strand of the small activating nucleic acid molecule of the present invention has at least 75% (e.g., at least about 79%, about 80, about 85%, about 90%, about 95%, about 99% or about 100%) homology or complementarity to any nucleotide sequence selected from SEQ ID NO: 200 to SEQ ID NO: 296. In one specific embodiment, one strand of the small activating nucleic acid molecule of the present invention comprises a nucleic acid sequence having at least 75% (e.g., at least about 79%, about 80%, about 85%, about 90%, about 95%, about 99% or about 100%) homology or complementarity to any nucleotide sequence selected from SEQ ID NO: 200 to SEQ ID NO: 296. In another embodiment, one strand of the small activating nucleic acid molecule of the present invention consists of a nucleic acid sequence having at least 75% (e.g., at least about 79%, about 80%, about 85%, about 90%, about 95%, about 99% or about 100%) homology or complementarity to any nucleotide sequence selected from SEQ ID NO: 200 to SEQ ID NO: 296. In yet another embodiment, one strand of the small activating nucleic acid molecule of the present invention is a nucleic acid sequence having at least 75% (e.g., at least about 79%, about 80%, about 85%, about 90%, about 95%, about 99% or about 100%) homology or complementarity to any nucleotide sequence selected from SEQ ID NO: 200 to SEQ ID NO: 296.

The small activating nucleic acid molecule of the present invention comprises a double-stranded small activating nucleic acid molecule, such as the small activating RNA (saRNA) molecule, targeting the promoter region of a mouse *Thpo* gene comprising a first nucleic acid strand and a second nucleic acid strand, wherein the first nucleic acid strand

has at least 75% (e.g., at least about 79%, about 80%, about 85%, about 90%, about 95%, about 99% or about 100%) homology or complementarity to any continuous sequence of 16 to 35 nucleotides in length in positions -493 to -356 (hotspot 1, SEQ ID NO: 2), positions -273 to -183 (hotspot 2, SEQ ID NO: 3) or positions -164 to -80 (hotspot 3, SEQ ID NO: 4) away from the transcription start site of the *Thpo* gene promoter, and the first nucleic acid strand and the second nucleic acid strand can complementarily form a double-stranded nucleic acid structure capable of activating the expression of the *Thpo* gene in a cell.

In another aspect of the present invention, a small activating nucleic acid (such as a small activating RNA molecule, saRNA) molecule activating/up-regulating the expression of the *THPO* gene in a mouse cell is provided, wherein one strand of the small activating nucleic acid molecule has at least 75% homology or complementarity to any nucleic acid sequence of 16 to 35 nucleotides in length in a promoter region of the human *THPO* gene, thereby activating or up-regulating the expression of the gene, wherein the promoter region comprises 500 nucleotides upstream of a transcription start site. Specifically, one strand of the small activating nucleic acid molecule comprises or is selected from a nucleic acid sequence having at least 75% (e.g., at least about 79%, about 80%, about 85%, about 90%, about 95%, about 99% or 100%) homology or complementarity to a continuous sequence of 16 to 35 nucleotides in length at positions -1339 to -1044 (hotspot 1, SEQ ID NO: 601), positions -1027 to -903 (hotspot 2, SEQ ID NO: 602), positions -861 to -754 (hotspot 3, SEQ ID NO: 603), positions -728 to -611 (hotspot 4, SEQ ID NO: 604), or positions - 593 to -1 (hotspot 5, SEQ ID NO: 605) upstream of the transcription start site of the *THPO* gene promoter. More specifically, one strand of the small activating nucleic acid molecule of the present invention has at least 75% (e.g., at least about 79%, about 80, about 85%, about 90%, about 95%, about 99% or about 100%) homology or complementarity to any nucleotide sequence selected from SEQ ID NO: 606 to SEQ ID NO: 1047. In one specific embodiment, one strand of the small activating nucleic acid molecule of the present invention comprises a nucleic acid sequence having at least 75% (e.g., at least about 79%, about 80%, about 85%, about 90%, about 95%, about 99% or about 100%) homology or complementarity to any nucleotide sequence selected from SEQ ID NO: 606 to SEQ ID NO: 1047. In another embodiment, one strand of the small activating nucleic acid molecule of the present invention consists of a nucleic acid sequence having at least 75% (e.g., at least about 79%, about 80%, about 85%, about 90%, about 95%, about 99% or about 100%) homology or complementarity to any nucleotide sequence selected from SEQ ID NO: 606 to SEQ ID NO: 1047. In yet another embodiment, one strand of the small activating nucleic acid molecule of the present invention is a nucleic acid sequence having at least 75% (e.g., at least about 79%, about 80%, about 85%, about 90%, about 95%, about 99% or about 100%) homology or complementarity to any nucleotide sequence selected from SEQ ID NO: 606 to SEQ ID NO: 1047.

A small activating nucleic acid molecule of the present invention comprises a double-stranded small activating nucleic acid molecule, such as the small activating RNA (saRNA) molecule, targeting the promoter region of the human *THPO* gene comprising a first nucleic acid strand and a second nucleic acid strand, wherein the first nucleic acid strand has at least 75% (e.g., at least about 79%, about 80%, about 85%, about 90%, about 95%, about 99% or about 100%) homology or complementarity to any continuous sequence of 16 to 35 nucleotides in length at positions -1339 to -1044 (hotspot 1, SEQ ID NO: 601), positions -1027 to -903 (hotspot 2, SEQ ID NO: 602), positions -861 to -754 (hotspot 3, SEQ ID NO: 603), positions -728 to -611 (hotspot 4, SEQ ID NO: 604) or positions -593 to -1 (hotspot 5, SEQ ID NO: 605) upstream of the transcription start site of the *THPO* gene promoter, and the first nucleic acid strand and the second nucleic acid strand can complementarily form a double-stranded nucleic acid structure capable of activating the expression of the human *THPO* gene in a cell.

The first nucleic acid strand and the second nucleic acid strand of a small activating nucleic acid molecule of the present invention may be present on either two different nucleic acid strands or on the same nucleic acid strand. When the first nucleic acid strand and the second nucleic acid strand are located on two different strands, at least one strand of the small activating nucleic acid molecule may have overhangs at the 5' terminus and/or the 3' terminus, e.g. overhangs of 0 to 6 nucleotides in length at 3' terminus, such that the overhangs are of 0, 1, 2, 3, 4, 5 or 6 nucleotides in length. Preferably, both strands of the small activating nucleic acid molecule of the present invention have overhangs; more preferably, the 3' terminus of both strands of the small activating nucleic acid molecule can have overhangs of 0 to 6 nucleotides in length, e.g., overhangs of 0, 1, 2, 3, 4, 5 or 6 nucleotides in length; most preferably overhangs of 2 or 3 nucleotides in length. Preferably, the nucleotide of the overhang is dT.

A small activating nucleic acid molecule of the present invention can also comprise a small activating nucleic acid molecule capable of forming a double-stranded region hairpin structure, e.g., a single-stranded small activating RNA molecule. In one embodiment, a small activating nucleic acid molecule of the present invention comprises a single-stranded small activating RNA molecule targeting the promoter region of the *THPO* gene, wherein the single-stranded small activating nucleic acid molecule can form a double-stranded region hairpin structure. Preferably, when the first nucleic acid strand and the second nucleic acid strand are present on the same nucleic acid strand, a small activating nucleic acid molecule of the present invention can be a hairpin single-stranded nucleic acid molecule, wherein the first nucleic acid strand and the second nucleic acid strand have complementary regions capable of forming a double-stranded nucleic acid structure, and the double-stranded nucleic acid structure can promote the expression of the *THPO* gene in

a cell with, for example, a RNA activation mechanism.

In the aforementioned small activating nucleic acid molecules, the first nucleic acid strand and the second nucleic acid strand can have 16 to 35 nucleotides (e.g., 16, 17, 18, 19, 20, 21, 22, 23, 24, 25, 26, 27, 28, 29, 30, 31, 32, 33, 34 or 35 nucleotides) in length.

In one embodiment, the first nucleic acid strand of a small activating nucleic acid molecule of the present invention has at least 75% (e.g., at least about 79%, about 80%, about 85%, about 90%, about 95%, about 99% or about 100%) identity or homology to any nucleotide sequence selected from SEQ ID NOs: 6-102, and the second nucleic acid strand of the small activating nucleic acid molecule has at least 75% (e.g., at least about 79%, about 80%, about 85%, about 90%, about 95%, about 99% or about 100%) identity or homology to any nucleotide sequence selected from SEQ ID NOs: 103-199. In one embodiment, the first nucleic acid strand of a small activating nucleic acid molecule of the present invention comprises a nucleic acid sequence having at least 75% (e.g., at least about 79%, about 80%, about 85%, about 90%, about 95%, about 99% or about 100%) identity or homology to any nucleotide sequence selected from SEQ ID NOs: 6-102, or consists of a nucleic acid sequence having at least 75% (e.g., at least about 79%, about 80%, about 85%, about 90%, about 95%, about 99% or about 100%) identity or homology to any nucleotide sequence selected from SEQ ID NOs: 6-142; the second nucleic acid strand of the small activating nucleic acid molecule of the present invention comprises a nucleic acid sequence having at least 75% (e.g., at least about 79%, about 80%, about 85%, about 90%, about 95%, about 99% or about 100%) identity or homology to any nucleotide sequence selected from SEQ ID NOs: 103-199, or consists of a nucleic acid sequence having at least 75% (e.g., at least about 79%, about 80%, about 85%, about 90%, about 95%, about 99% or about 100%) identity or homology to any nucleotide sequence selected from SEQ ID NOs: 103-199. In a specific embodiment, the first nucleic acid strand of a small activating nucleic acid molecule of the present invention can comprise or be selected from any nucleotide sequence set forth in SEQ ID NOs: 6-102, and the second strand can comprise or be selected from any nucleotide sequence set forth in SEQ ID NOs: 103-199. In one embodiment, the small activating nucleic acid molecule described herein can be synthesized, transcribed *in vitro,* or expressed by a vector.

In another embodiment, the first nucleic acid strand of a small activating nucleic acid molecule of the present invention has at least 75% (e.g., at least about 79%, about 80%, about 85%, about 90%, about 95%, about 99% or about 100%) identity or homology to any nucleotide sequence selected from SEQ ID NO: 1048 to SEQ ID NO: 1489, and the second nucleic acid strand of a small activating nucleic acid molecule has at least 75% (e.g., at least about 79%, about 80%, about 85%, about 90%, about 95%, about 99% or about 100%) identity or homology to any nucleotide sequence selected from SEQ ID NO: 1490 to SEQ ID NO: 1931. In one embodiment, the first nucleic acid strand of a small activating nucleic acid molecule of the present invention comprises a nucleic acid sequence having at least 75% (e.g., at least about 79%, about 80%, about 85%, about 90%, about 95%, about 99% or about 100%) identity or homology to any nucleotide sequence selected from SEQ ID NO: 1048 to SEQ ID NO: 1489, or consists of a nucleic acid sequence having at least 75% (e.g., at least about 79%, about 80%, about 85%, about 90%, about 95%, about 99% or about 100%) identity or homology to any nucleotide sequence selected from SEQ ID NO: 1048 to SEQ ID NO: 1489; the second nucleic acid strand of a small activating nucleic acid molecule of the present invention comprises a nucleic acid sequence having at least 75% (e.g., at least about 79%, about 80%, about 85%, about 90%, about 95%, about 99% or about 100%) identity or homology to any nucleotide sequence selected from SEQ ID NO: 1490 to SEQ ID NO: 1931, or consists of a nucleic acid sequence having at least 75% (e.g., at least about 79%, about 80%, about 85%, about 90%, about 95%, about 99% or about 100%) identity or homology to any nucleotide sequence selected from SEQ ID NO: 1490 to SEQ ID NO: 1931. In a specific embodiment, the first nucleic acid strand of a small activating nucleic acid molecule of the present invention can comprise or be selected from any nucleotide sequence set forth in SEQ ID NO: 1048 to SEQ ID NO: 1489, and the second strand can comprise or be selected from any nucleotide sequence set forth in SEQ ID NO: 1490 to SEQ ID NO: 1931. In one embodiment, the small activating nucleic acid molecule described herein can be synthesized, transcribed *in vitro,* or expressed by a vector.

All the nucleotides in the small activating nucleic acid molecule described herein can be natural non-chemically modified nucleotides or can comprise at least one modification. In one embodiment, the modification in the small activating nucleic acid molecule described herein can comprise chemical modification, for example, at least one nucleotide can have a chemical modification, and the chemical modification used in the present invention can comprise or be selected from one or more combinations of the following modifications:

(1) modification of a phosphodiester bond of nucleotides in the nucleotide sequence of the small activating nucleic acid molecule;

(2) modification of 2'-OH of a ribose in the nucleotide sequence of the small activating nucleic acid molecule;

(3) modification of a base in the nucleotide sequence of the small activating nucleic acid molecule; and

(4) at least one nucleotide in the nucleotide sequence of the small activating nucleic acid molecule being a locked nucleic acid.

The chemical modifications described herein is well-known to those skilled in the art, and the modification of the phosphodiester bond refers to the modification of oxygen in the phosphodiester bond, including, but not limited to, phosphorothioate modification and boranophosphate modification. Both modifications can stabilize an saRNA structure and maintain high specificity and high affinity for base pairing.

The ribose modification refers to the modification of 2'-OH in pentose of a nucleotide, i.e., the introduction of some substituents into hydroxyl positions of the ribose, for example, including, but not limited to, 2'-fluoro modification, 2'-oxymethyl modification, 2'-oxyethylidene methoxy modification, 2,4'-dinitrophenol modification, locked nucleic acid (LNA), 2'-amino modification, 2'-deoxy modification, and the like.

The base modification refers to the modification of the base of a nucleotide, for example, including, but not limited to, 5'-bromouracil modification, 5'-iodouracil modification, N-methyluracil modification, 2,6-diaminopurine modification, and the like.

These modifications can increase the bioavailability of the small activating nucleic acid molecule, improve affinity to a target sequence, and enhance resistance to nuclease hydrolysis in a cell.

In addition, in order to promote the access of a small activating nucleic acid molecule into a cell, on the basis of the aforementioned modifications, a lipophilic group, such as cholesterol,) can be introduced on the terminus of the first nucleic acid strand and/or the second nucleic acid strand of the small activating nucleic acid molecule to facilitate the interaction with the promoter region of a gene in the cell nucleus, as the cell membrane and nuclear membrane are composed of lipid bilayers.

After contacting and entering a cell, the small activating nucleic acid molecule of the present invention can effectively activate or up-regulate the expression of the *Thpo*/*THPO* gene in the cell, preferably by at least 10%.

Another aspect of the present invention also relates to a nucleic acid encoding a small activating nucleic acid molecule of the invention. In one embodiment, the nucleic acid is a DNA molecule.

Another aspect of the present invention provides a cell comprising the aforementioned small activating nucleic acid molecule or the nucleic acid encoding the small activating nucleic acid molecule described herein. In one embodiment, a small activating nucleic acid molecule of the present invention can be a double-stranded small activating nucleic acid molecule, such as a double-stranded small activating RNA (saRNA) molecule, which targets the promoter region of the *Thpo*/*THPO* gene comprising a first nucleic acid strand and a second nucleic acid strand. In another embodiment, a small activating nucleic acid molecule of the present invention can be a single-stranded small activating nucleic acid molecule, such as a hairpin-structured single-stranded nucleic acid, targeting the promoter region of the *Thpo*/*THPO* gene, wherein the hairpin-structured single-stranded nucleic acid produces a double-stranded small activating RNA (saRNA) molecule in a cell after being introduced into the cell.

Another aspect of the present invention provides a composition (e.g., a pharmaceutical composition). The composition comprises a small activating nucleic acid molecule of the present invention or a nucleic acid encoding the small activating nucleic acid molecule described herein, and optionally, a pharmaceutically acceptable carrier. In one embodiment, the pharmaceutically acceptable carrier can comprise or be selected from a liposome, a high-molecular polymer, and a polypeptide.

In another aspect of the present invention, a formulation is provided which comprises a small activating nucleic acid molecule described in the present invention, a nucleic acid encoding the small activating nucleic acid molecule described in the present invention, or a cell comprising a small activating nucleic acid molecule of the present invention or a nucleic acid encoding a small activating nucleic acid molecule of the present invention, or a composition comprising a small activating nucleic acid molecule of the present invention.

In another aspect of the present invention, a kit is provided which comprises a small activating nucleic acid molecule of the present invention, a nucleic acid encoding a small activating nucleic acid molecule described herein, or a cell comprising a small activating nucleic acid molecule of the present invention or a nucleic acid encoding a small activating nucleic acid molecule of the present invention, or a composition comprising a small activating nucleic acid molecule of the present invention.

Another aspect of the present invention relates to uses of the small activating nucleic acid molecule described in the present invention, a nucleic acid encoding the small activating nucleic acid molecule of the present invention, a cell comprising the small activating nucleic acid molecule of the present invention, or the nucleic acid encoding the small activating nucleic acid molecule of the present invention, or a composition comprising the small activating nucleic acid molecule of the present invention in the preparation of a drug or formulation for activating/up-regulating the expression of the *Thpo*/*THPO* gene in a cell.

Another aspect of the present invention also relates to a method for activating/up-regulating the expression of the *Thpo*/*THPO* gene in a cell. The method comprises administering the small activating nucleic acid molecule described in the present invention, a nucleic acid encoding the small activating nucleic acid molecule of the present invention or

a composition or formulation comprising the small activating nucleic acid molecule of the present invention to a cell. In one embodiment, the method for activating/up-regulating the expression of the *Thpo*/*THPO* gene in the cell comprises administering the small activating nucleic acid molecule described in the present invention, the nucleic acid encoding the small activating nucleic acid molecule of the present invention, or the composition or formulation comprising the small activating nucleic acid molecule of the present invention to the cell. The aforementioned cell comprises a mammal cell, e.g., a cell from a human body, such as a human embryo liver cell, a human hepatoma cell, such as an HepG2 cell, and a human liver cell, or a cell from a mouse, such as a mouse embryonic liver cell, such as embryonic cell line BNL.CL2, a mouse liver cancer cell, such as the liver cancer cell line LPC-H12, and a mouse primary liver cell. The aforementioned cell can be *in vitro,* or can be present in a mammalian body, such as a mouse or human body.

The small activating nucleic acid molecule of the present invention can be directly introduced into a cell, or can be produced in the cell after a nucleotide sequence encoding the small activating nucleic acid molecule of the present invention is introduced into the cell. The cell is preferably a mammalian cell, more preferably a mouse or human cell. The aforementioned cell may be *in vitro,* such as a cell line or a cell strain, or may present in a mammalian body, such as a mouse or human body, and more specifically, such as a liver cell from a mouse or human body. The human body may be a patient suffering from a disease or condition related to insufficient or decreased expression of the THPO protein, or a patient with thrombocytopenia due to various causes. The small activating nucleic acid molecule of the present invention can be administered at a sufficient dose to treat the disease or condition related to a deficiency in the amount of THPO protein or insufficient or decreased expression of the THPO protein. Specifically, the disease or condition related to a deficiency in the amount of THPO protein or insufficient or decreased expression of the THPO protein may be thrombocytopenia.

Another aspect of the present invention provides an isolated small activating nucleic acid molecule acting site of the *Thpo*/*THPO* gene, which has any continuous sequence of 16 to 35 nucleotides in length in the promoter region of the *Thpo*/*THPO* gene, and preferably, the acting site comprises or is selected from any continuous sequence of 16 to 35 nucleotides in length in any of the sequences set forth in SEQ ID NOs: 2-4 and SEQ ID NOs: 601-605. Specifically, the acting site may comprise or be selected from any nucleotide sequence set forth in SEQ ID NOs: 200-296 and SEQ ID NOs: 606-1047.

Another aspect of the present invention relates to a method for treating a disease or condition related to insufficient or decreased expression of the THPO protein in a subject such as a human or mouse or thrombocytopenia, which comprises administering a small activating nucleic acid molecule of the present invention, a nucleic acid encoding a small activating nucleic acid molecule of the present invention, a cell comprising a small activating nucleic acid molecule of the present invention, or a nucleic acid encoding a small activating nucleic acid molecule of the present invention, or a composition comprising a small activating nucleic acid molecule of the present invention at a curative dose to the subject. In one embodiment, a method for treating thrombocytopenia and/or a disease or condition related to insufficient or decreased expression of THPO protein in a subject in the present invention can comprise administering a small activating nucleic acid molecule of the present invention, a nucleic acid encoding the small activating nucleic acid molecule of the present invention, a cell comprising the small activating nucleic acid molecule of the present invention, or a nucleic acid enencoding a small activating nucleic acid molecule of the present invention, or a composition comprising the small activating nucleic acid molecule of the present invention, and other formulations at a curative dose to the subject, wherein the other formulations comprises, such as a low-molecular-weight compound, antibody, polypeptide or protein, etc. The subject can be a mammal, e.g., a mouse or a human. In the present invention, thrombocytopenia is caused by various factors, including, but not limited to, myelo-thrombocytopenia, increased peripheral platelet destruction, and splenic sequestration. Factors causing a reduction in platelets comprise congenital (hereditary) and acquired thrombocytopenia. In one embodiment, factors causing acquired thrombocytopenia include, but are not limited to, thrombocytopenia caused by aplastic anemia, myelodysplastic syndromes, leukemia, drugs, infection, tumor diseases, radiotherapy, bone marrow transplantation, and chronic liver diseases, and immune thrombocytopenia (ITP). Factors causing increased platelet destruction include, but are not limited to, thrombotic thrombocytopenia, heparin-induced thrombocytopenia, drug-induced thrombocytopenia, ITP, and thrombotic thrombocytopenia purpura. In one embodiment, thrombocytopenia is related to a disease or condition related to insufficient or decreased expression of the THPO protein. In another embodiment, thrombocytopenia is related to insufficient or decreased expression of the THPO protein. Thrombocytopenia can comprise drug-induced thrombocytopenia, immune thrombocytopenia, thrombotic thrombocytopenia purpura, and the like. The thrombocytopenia described herein can be caused by various factors, such as myelo-thrombocytopenia, increased peripheral platelet destruction and splenomegaly sequestration, such as tumor radiotherapy, tumor chemotherapy, bone marrow transplantation, chronic liver disease, drug-induced thrombocytopenia, immune thrombocytopenia, thrombotic thrombocytopenic purpura, and hereditary thrombocytopenia. Thrombocytopenia is generally characterized clinically by ecchymosis, changes in megakaryocytes in the bone marrow, mucosal bleeding or intracranial bleeding, all of which will endanger the lives of patients with severe thrombocytopenia. When the platelet concentrations are lower than 100 $\times$ $10^9$/L by direct blood platelet count, thrombocytopenia is considered to be present (Kuter 2009). When the platelet concentrations are lower than 50 $\times$ $10^9$/L, spontaneous bleeding, commonly manifesting

as subcutaneous purpuraa, occurs frequently. When the platelet concentrations are lower than $20 \times 10^9$/L, a patient's condition can be life-threatening, caused by trauma or sudden intracranial bleeding, gastrointestinal bleeding, and the like. In the method of the present invention, the thrombocytopenia in the subject can be characterized by a platelet concentration of less than $100 \times 10^9$/L, less than $50 \times 10^9$/L, or less than $20 \times 10^9$/L as indicated by direct blood platelet count. In one embodiment, a disease or condition related to insufficient or decreased expression of the THPO protein in a subject is alleviated, relieved, or cured after administering a small activating nucleic acid molecule of the present invention, a nucleic acid encoding the small activating nucleic acid molecule of the present invention, the cell comprising the small activating nucleic acid molecule of the present invention, or the nucleic acid encoding the small activating nucleic acid molecule of the present invention, or the composition comprising the small activating nucleic acid molecule of the present invention at a curative dose.

Another aspect of the present invention relates to a method for treating thrombocytopenia in a subject, which comprises administering a small activating nucleic acid molecule of the present invention, a nucleic acid encoding the small activating nucleic acid molecule of the present invention, a cell comprising the small activating nucleic acid molecule of the present invention, a nucleic acid encoding the small activating nucleic acid molecule of the present invention, or a composition comprising the small activating nucleic acid molecule of the present invention at a curative dose to the subject. In the method of the present invention, the thrombocytopenia in the subject can be characterized by a platelet concentration of less than $100 \times 10^9$/L, less than $50 \times 10^9$/L, or less than $20 \times 10^9$/L as indicated by direct blood platelet count. In a method of the present invention, the thrombocytopenia can be related to insufficient or decreased expression of the THPO protein, drug-induced thrombocytopenia, immune thrombocytopenia, thrombotic thrombocytopenic purpuraa, and the like. The thrombocytopenia as described herein can be caused due to various factors such as myelothrombocytopenia, increased peripheral platelet destruction and splenomegaly sequestration, such as tumor radiotherapy, tumor chemotherapy, bone marrow transplantation, chronic liver disease, drug-induced thrombocytopenia, immune thrombocytopenia, thrombotic thrombocytopenic purpura, and hereditary thrombocytopenia. In one embodiment, the method for treating thrombocytopenia of the present invention comprises administering a small activating nucleic acid molecule of the present invention, a nucleic acid encoding a small activating nucleic acid molecule of the present invention, a cell comprising a small activating nucleic acid molecule of the present invention or a nucleic acid encoding the small activating nucleic acid molecule of the present invention, or a composition comprising a small activating nucleic acid molecule of the present invention and/or the aforementioned the small activating nucleic acid molecule, the nucleic acid encoding the aforementioned small activating nucleic acid molecule, the cell comprising the small activating nucleic acid molecule of the present invention or the nucleic acid encoding the small activating nucleic acid molecule of the present invention, or a composition consisting of the composition comprising the small activating nucleic acid molecule of the present invention and one or more other formulations at a curative dose to the subject, wherein the other formulation comprises, such as a low-molecular-weight compound, antibody, polypeptide and protein. The subject can be a mammal, such as a mouse or human. In the aforementioned method, the thrombocytopenia can comprise drug-induced thrombocytopenia, immune thrombocytopenia, thrombotic thrombocytopenia purpura, and the like. In the aforementioned method, the subject comprises a mammal, such as a mouse or human. In one embodiment, the thrombocytopenia in a subject is alleviated, relieved, or cured after administering the small activating nucleic acid molecule of the present invention, the nucleic acid encoding the small activating nucleic acid molecule of the present invention, the cell comprising the small activating nucleic acid molecule of the present invention, or the nucleic acid encoding the small activating nucleic acid molecule of the present invention, or the composition comprising the small activating nucleic acid molecule of the present invention at a curative dose. Preferably, the alleviation, relief, or cure may be an increase or recovery of the platelet concentration to near normal physiological levels, such as in the range of $100 \times 10^9$/L to $300 \times 10^9$/L, as indicated by direct blood platelet count.

Another aspect of the present invention relates to a use of a small activating nucleic acid molecule of the present invention, a nucleic acid encoding the small activating nucleic acid molecule of the present invention, a cell comprising the small activating nucleic acid molecule of the present invention or the nucleic acid encoding the small activating nucleic acid molecule of the present invention, or the composition comprising the small activating nucleic acid molecule of the present invention in the preparation of a drug for treating a disease or condition related to thrombocytopenia and/or insufficient or decreased expression of the THPO protein. The subject can be a mammal, such as a human. In uses of present invention, thrombocytopenia is caused by various factors, including, but not limited to, myelo-thrombocytopenia, increased peripheral platelet destruction, and splenic sequestration. Factors causing a reduction in platelets comprise congenital (hereditary) and acquired thrombocytopenia. In one embodiment, thrombocytopenia caused by acquired factors include, but are not limited to, thrombocytopenia caused by aplastic anemia, myelodysplastic syndromes, leukemia, drugs, infection, tumor diseases, radiotherapy, bone marrow transplantation, and chronic liver diseases, and immune thrombocytopenia (ITP). Factors causing increased platelet destruction include, but are not limited to, thrombotic thrombocytopenia, heparin-induced thrombocytopenia, drug-induced thrombocytopenia, ITP, and thrombotic thrombocytopenia purpura. In one embodiment, the thrombocytopenia can comprise, for example, thrombocytopenia related to insufficient or decreased expression of the THPO protein. Preferably, the thrombocytopenia can comprises or be selected

from thrombocytopenia related to insufficient or decreased expression of the THPO protein, drug-induced thrombocytopenia, immune thrombocytopenia, thrombotic thrombocytopenic purpuraa, and the like. Thrombocytopenia as described herein can be caused by various factors such as tumor radiotherapy, tumor chemotherapy, bone marrow transplantation, chronic liver disease, splenomegaly sequestration, transfusion of banked blood with low platelet content, and an increase of platelet consumption due to drugs. In one embodiment, thrombocytopenia can be manifested as a platelet concentration of less than 100 $\times$ $10^9$/L, less than 50 $\times$ $10^9$/L, or less than 20 $\times$ $10^9$/L as indicated by direct blood platelet counts.

In one embodiment, a use of a small activating nucleic acid molecule described in the present invention, a nucleic acid encoding the small activating nucleic acid molecule of the present invention, a cell comprising the small activating nucleic acid molecule of the present invention or the nucleic acid encoding the small activating nucleic acid molecule of the present invention, or the composition comprising the small activating nucleic acid molecule of the present invention in the preparation of a drug for treating thrombocytopenia or a disease related to insufficient or decreased expression of the THPO protein is provided. In one embodiment, thrombocytopenia can comprise thrombocytopenia related to insufficient or decreased expression of THPO protein, and preferably, thrombocytopenia can comprise drug-induced thrombocytopenia, immune thrombocytopenia, thrombotic thrombocytopenic purpuraa, and the like. In one embodiment, thrombocytopenia can be manifested as a platelet concentration of less than 100 $\times$ $10^9$/L, less than 50 $\times$ $10^9$/L, or less than 20 $\times$ $10^9$/L as indicated by direct blood platelet count.

Another aspect of the present invention relates to a use of the small activating nucleic acid molecule described in the present invention, the nucleic acid encoding the small activating nucleic acid molecule of the present invention, the cell comprising the small activating nucleic acid molecule of the present invention or the nucleic acid encoding the small activating nucleic acid molecule of the present invention, or the composition or formulation comprising the small activating nucleic acid molecule of the present invention in the preparation of a drug or pharmaceutical composition for treating thrombocytopenia. Preferably, the thrombocytopenia can comprise or be selected from thrombocytopenia related to insufficient or decreased expression of the THPO protein or thrombocytopenia due to other various causes, such as drug-induced thrombocytopenia, immune thrombocytopenia, thrombotic thrombocytopenic purpuraa, and the like. The thrombocytopenia described herein can be caused by various factors such as tumor radiotherapy, tumor chemotherapy, bone marrow transplantation, chronic liver disease, splenomegaly sequestration, transfusion of banked blood with low platelet content and an increase of platelet consumption due to drugs. In one embodiment, thrombocytopenia can be manifested as a platelet concentration of less than 100 $\times$ $10^9$/L, less than 50 $\times$ $10^9$/L, or less than 20 $\times$ $10^9$/L as indicated by direct blood platelet count.

In one embodiment, a use of s small activating nucleic acid molecule described in the present invention, a nucleic acid encoding the small activating nucleic acid molecule of the present invention, a cell comprising the small activating nucleic acid molecule of the present invention or the nucleic acid encoding the small activating nucleic acid molecule of the present invention, or a composition or formulation comprising the small activating nucleic acid molecule of the present invention in the preparation of a drug or pharmaceutical composition for treating a disease related to insufficient or decreased expression of the THPO protein or thrombocytopenia.

**Advantages of the Present Invention**

The small activating nucleic acid molecule, such as small activating RNA (saRNA), is capable of activating/up-regulating endogenous *Thpo*/*THPO* gene expression provided by the present invention and can specifically activate *Thpo*/*THPO* gene, thereby up-regulating or restoring the expression of *Thpo*/*THPO* gene and protein with lower toxic and side effects, which can be used for treating thrombocytopenia caused by insufficient platelet production or excessive destruction due to insufficient *Thpo*/*THPO* protein expression and various causes, or in preparing a drug or formulation for treating thrombocytopenia.

BRIEF DESCRIPTION OF THE DRAWINGS

**FIG. 1 shows changes in the expression of mouse *Thpo* mRNA mediated by mouse** *Thpo* **saRNA.** BNL.CL2 cells were transfected with 322 saRNAs targeting mouse *Thpo* promoter at a final concentration of 10 nM, and *Thpo* mRNA expression was analyzed 72 h after transfection using one-step RT-qPCR. The figure shows the descending order of mRNA expression changes ($\log_2$) of *Thpo* relative to control treatment (Mock). The ordinate values represent the mean of 2 repeat treatment $\pm$ SD.

**FIG. 2 shows the hotspot region of mouse *Thpo* saRNA on the mouse *Thpo* promoter.** BNL.CL2 cells were transfected with 322 saRNAs targeting mouse *Thpo* promoter at a final concentration of 10 nM for 72 h, and *Thpo*

mRNA expression was analyzed after transfection using one-step RT-qPCR. The figure shows changes in the expression of *Thpo* relative to control treatment (Mock) ranked from -500 to -0 according to the target positions of the saRNA in the *Thpo* promoter. Black solid dots represent functional saRNAs, white open dots represent non-functional saRNAs, and dotted lines frame the hotspot regions where 3 functional saRNAs gather (HI to H3). The ordinate values represent the mean of 2 repeat treatment.

**FIG. 3 shows the screening results of two-step RT-qPCR validated mouse *Thpo* saRNA HTS.** As shown, the BNL.CL2 cells were transfected with saRNA at a final concentration of 10 nM for 72 h. After transfection, RNA was extracted with Qiagen RNeasy kit and qPCR amplification was performed with ABI 7500 rapid real-time PCR system after reverse transcription. The Tbp gene was amplified as internal control. The relative expression values of *Thpo* mRNA are shown after treatment of cells with a single saRNA. Mock, dsCon2, and DS03-432i are shown respectively as blank transfection, sequence-independent double-stranded RNA transfection, and small interference RNA control transfection. The ordinate values represent the mean of 2 repeat treatment ± SD.

**FIG. 4 shows the screening results of two-step RT-qPCR validated mouse *Thpo* saRNA HTS.** As shown, the LPC-H12 cells were transfected with saRNA at a final concentration of 10 nM for 72 h. After transfection, RNA was extracted with Qiagen RNeasy kit after transfection and qPCR amplification was performed with ABI 7500 rapid real-time PCR system after reverse transcription. The *Tbp* gene was amplified as internal control. The relative expression values of *Thpo* mRNA are shown after treatment of cells with a single saRNA. Mock, dsCon2, and DS03-432i are shown respectively as blank transfection, sequence-independent double-stranded RNA transfection, and small interference RNA control transfection. The ordinate values represent the mean of 2 repeat treatment ± SD.

**FIG. 5 shows the screening results of two-step RT-qPCR validated mouse *Thpo* saRNA HTS in a primary mouse liver cell. As shown, the** primary mouse liver cell were transfected with saRNA at a final concentration of 10 nM for 72 h. After transfection, RNA was extracted with Qiagen RNeasy kit after transfection and qPCR amplification was performed with ABI 7500 rapid real-time PCR system after reverse transcription. The *Tbp* gene was amplified as internal control. The relative expression values of *Thpo* mRNA are shown after treatment of cells with a single saRNA. Mock, dsCon2, and DS03-432i are are shown respectively as blank transfection, sequence-independent double-stranded RNA transfection, and small interference RNA control transfection. The ordinate values represent the mean of 2 repeat treatment ± SD.

**FIG. 6 shows that mouse *Thpo* saRNA increases concentration of Thpo in mice serum.** Fifteen male *Balb/c* mice were randomly divided into 3 groups with 5 mice in each group, and received PBS, LNP-entrapped *Thpo*-siRNA (DS03-432i, 5 mg/kg) and LNP-entrapped *Thpo*-saRNA (DS03-0024, 5 mg/kg) by tail vein injection. Blood samples were collected after 72 h and serum was separated. A mouse **Thpo** ELISA kit was used to determine Thpo protein levels in mice serum. PBS is a blank control and DS03-432i is a small interfering RNA control. (*** $p < 0.001$).

**FIG. 7 shows that mouse *Thpo* saRNA increases concentration of Thpo in mice serum.** Thirty two male *Balb/c* mice were randomly divided into 4 groups with 8 mice in each group, and received PBS and LNP-entrapped DS03-0024 (1 mg/kg, 3 mg/kg and 6 mg/kg) by tail vein injection. Blood samples were collected and serum was isolated 1 day prior to administration and 2 days after administration. A mouse **Thpo** ELISA kit was used to determine the Thpo protein levels in mice serum (**** $p < 0.0001$).

**FIG. 8 shows that mouse *Thpo* saRNA increases the number of platelets of mouse.** Thirty two male *Balb/c* mice were randomly divided into 4 groups with 8 mice in each group, and received PBS and LNP-entrapped DS03-0024 (1 mg/kg, 3 mg/kg and 6 mg/kg) by tail vein injection. Blood samples were collected 6 days after administration, and platelets were collected using a mouse peripheral platelet isolation kit, which utilized a labeled CD41a-FITC antibody to facilitate counting using a flow cytometer. (*$p < 0.05$).

**FIG. 9 shows that changes in the number of platelets of mouse caused by mouse *Thpo* saRNA 9 days after administration.** Thirty two male *Balb/c* mice were randomly divided into 4 groups with 8 mice in each group, and received PBS and LNP-entrapped DS03-0024 (1 mg/kg, 3 mg/kg and 6 mg/kg) by tail vein injection. Blood samples were collected 17 days after administration and platelets were collected using a mouse peripheral platelet isolation kit, which utilized a labeled CD41a-FITC antibody to facilitate counting using a flow cytometer. (* $p < 0.05$).

**FIG. 10 shows changes in the expression of human *THPO* mRNA mediated by human *THPO* saRNA.** HepG2 cells were transfected with candidate saRNAs targeting the human *THPO* promoter at a final concentration of 25 nM. Human *THPO* mRNA expression was analyzed 72 h after transfection using one-step RT-qPCR. The figure

shows the descending order of mRNA expression changes ($log_2$) of *THPO* relative to control treatment (Mock). The ordinate values represent the mean of 2 repeat treatment ± SD.

**FIG. 11 shows the hotspot region of human *THPO* saRNA on the** human ***THPO*** **promoter.** HepG2 cells were transfected with candidate saRNAs targeting the human *THPO* promoter at a final concentration of 25 nM for 72 h. Human *THPO* mRNA expression was analyzed after transfection using one-step RT-qPCR. The figure showschanges in the expression of *THPO* relative to control treatment (Mock) ranked according to the target positions of the saRNA in the *THPO* promoter. Black solid dots represent functional saRNAs, white open dots represent non-functional saRNAs, and dotted lines frame the hotspot regions where functional saRNAs gather. The ordinate values represent the mean of 2 repeat treatment ± SD.

**FIG. 12 shows the screening results of two-step RT-qPCR validated human *THPO* saRNA HTS.** HepG2 cells were transfected with saRNA at a final concentration of 25 nM for 72 h. After transfection, RNA was extracted with Qiagen RNeasy kit. After reverse transcription, qPCR amplification was performed with ABI 7500 rapid real-time PCR system. *TBP* and *HPRT1* genes were amplified as internal controls. Shown are the relative mRNA expression values *of THPO* after treatingcells with a single saRNA. Shown are Mock, dsCon2, and *siTHPO* (DS3A-365i) which are blank transfection, sequence-independent double-stranded RNA transfection, and small interference RNA control transfection, respectively. The ordinate values represent the mean of 2 repeat treatment ± SD.

**FIG. 13 shows the effect of human *THPO* saRNA on the expression of human THPO protein by ELISA method.** HepG2 cells were transfected with saRNA at a final concentration of 25 nM for 72 h. After transfection, culture medium was taken and the supernatant was centrifuged to remove dead cell debris. The level of THPO protein secreted into the culture medium was determined by ELISA kit (R & D Systems, DTP00B). Shown are the expression values of THPO protein after treating the cells with a single saRNA. Shown are Mock, dsCon2, and *siTHPO* (DS3A-365i) which are blank transfection, sequence-independent double-stranded RNA transfection, and small interference RNA control transfection, respectively. The ordinate values represent the mean of 2 repeat treatment ± SD.

## DETAILED DESCRIPTION

In the present invention, the related terms are defined as follows.

The term "complementarity" as used herein refers to the capability of forming base pairs between two oligonucleotide strands. The base pairs are generally formed through hydrogen bonds between nucleotides in the antiparallel oligonucleotide strands. The bases of the complementary oligonucleotide strands can be paired in the Watson-Crick manner (such as A to T, A to U, and C to G) or in any other manner allowing the formation of a duplex (such as Hoogsteen or reverse Hoogsteen base pairing).

Complementarity comprises complete complementarity and incomplete complementarity. "Complete complementarity" or "100% complementarity" means that each nucleotide from the first oligonucleotide strand can form a hydrogen bond with a nucleotide at a corresponding position in the second oligonucleotide strand in the double-stranded region of the double-stranded oligonucleotide molecule without "mispairing". "Incomplete complementarity" means that not all the nucleotide units of the two strands are bonded with each other by hydrogen bonds. For example, for two oligonucleotide strands each of 20 nucleotides in length in the double-stranded region, if only two base pairs in this double-stranded region can be formed through hydrogen bonds, the oligonucleotide strands have a complementarity of 10%. In the same example, if 18 base pairs in this double-stranded region can be formed through hydrogen bonds, the oligonucleotide strands have a complementarity of 90%. Substantial complementarity refers to at least about 75%, about 79%, about 80%, about 85%, about 90%, about 95%, about 99% or about 100% complementarity.

The term "oligonucleotide" as used herein refers to polymers of nucleotides, and includes, but is not limited to, single-stranded or double-stranded molecules of DNA, RNA, or DNA/RNA hybrid, oligonucleotide strands containing regularly and irregularly alternating deoxyribosyl portions and ribosyl portions, as well as modified and naturally or unnaturally existing frameworks for such oligonucleotides. The oligonucleotide for activating target gene transcription described herein is a small activating nucleic acid molecule.

The terms "oligonucleotide strand" and "oligonucleotide sequence" as used herein can be used interchangeably, referring to a generic term for short nucleotide sequences having less than 35 bases (including nucleotides in deoxyribonucleic acid (DNA) or ribonucleic acid (RNA)). In the present invention, an oligonucleotide strand may have any of 16 to 35 nucleotides in length.

As used herein, the term "first nucleic acid strand" can be a sense strand or an antisense strand. The sense strand of a small activating RNA refers to a nucleic acid strand contained in a small activating RNA duplex which has identity to the coding strand of the promoter DNA sequence of a target gene, and the antisense strand refers to a nucleic acid strand in the small activating RNA duplex which is complementary to the sense strand.

As used herein, the term "second nucleic acid strand" can also be a sense strand or an antisense strand. If the first oligonucleotide strand is a sense strand, the second oligonucleotide strand is an antisense strand; if the first oligonucleotide strand is an antisense strand, the second oligonucleotide strand is a sense strand.

The term "gene" as used herein refers to all nucleotide sequences required to encode a polypeptide chain or to transcribe a functional RNA. "Gene" can be an endogenous or fully or partially recombinant gene for a host cell (for example, because an exogenous oligonucleotide and a coding sequence for encoding a promoter are introduced into a host cell, or a heterogeneous promoter adjacent to an endogenous encoding sequence is introduced into a host cell). For example, the term "gene" comprises a nucleic acid sequence consisting of exons and introns. Protein-encoding sequences are, for example, sequences contained within exons in an open reading frame between an initiation codon and a termination codon, and as used herein, "gene" can comprise such as a gene regulatory sequence, such as a promoter, an enhancer, and all other sequences known in the art for controlling the transcription, expression or activity of another gene, no matter whether the gene comprises a coding sequence or a non-coding sequence. In one case, for example, "gene" can be used to describe a functional nucleic acid comprising a regulatory sequence such as a promoter or an enhancer. The expression of a recombinant gene can be controlled by one or more types of heterogeneous regulatory sequences.

The term "target gene" as used herein can refer to nucleic acid sequences naturally present in organisms, transgenes, viral or bacterial sequences, can be chromosomes or extrachromosomal genes, and/or can be transiently or stably transfected or incorporated into cells and/or chromatins thereof. The target gene can be a protein-encoding gene or a non-protein-encoding gene (such as a microRNA gene and a long non-coding RNA gene). The target gene generally contains a promoter sequence, and the positive regulation for the target gene can be achieved by designing a small activating nucleic acid molecule having sequence identity (also called homology) to the promoter sequence, characterized as the up-regulation of expression of the target gene. "Sequence of a target gene promoter" refers to a non-coding sequence of the target gene, and the reference of the sequence of a target gene promoter in the phrase "complementary to the sequence of a target gene promoter" of the present invention refers to a coding strand of the sequence, also known as a non-template strand, i.e., a nucleic acid sequence having the same sequence as the coding sequence of the gene. "Target" or "target sequence" refers to a sequence fragment in the sequence of a target gene promoter which is homologous or complementary with a sense oligonucleotide strand or an antisense oligonucleotide strand of a small activating nucleic acid molecule.

As used herein, the terms "sense strand" and "sense nucleic acid strand" can be used interchangeably, and the sense oligonucleotide strand of a small activating nucleic acid molecule refers to the first nucleic acid strand having identity to the coding strand of the sequence of a target gene promoter in the small activating nucleic acid molecule duplex.

As used herein, the terms "antisense strand" and "antisense nucleic acid strand" can be used interchangeably, and the antisense oligonucleotide strand of a small activating nucleic acid molecule refers to the second nucleic acid strand complementary with the sense oligonucleotide strand in the small activating nucleic acid molecule duplex.

The term "coding strand" as used herein refers to a DNA strand in the target gene which cannot be used for transcription, and the nucleotide sequence of this strand is the same as that of an RNA produced from transcription (in the RNA, T in DNA is replaced by U). The coding strand of the double-stranded DNA sequence of the target gene promoter described herein refers to a promoter sequence on the same DNA strand as the DNA coding strand of the target gene.

The term "template strand" as used herein refers to the other strand complementary with the coding strand in the double-stranded DNA of the target gene, i.e., the strand that, as a template, can be transcribed into RNA, and this strand is complementary with the transcribed RNA (A to U and G to C). In the process of transcription, RNA polymerase binds to the template strand, moves along the 3'→5' direction of the template strand, and catalyzes the synthesis of the RNA along the 5'→3' direction. The template strand of the double-stranded DNA sequence of the target gene promoter described herein refers to a promoter sequence on the same DNA strand as the DNA template strand of the target gene.

The term "promoter" as used herein refers to a sequence which plays a regulatory role for the transcription of a protein-coding or RNA-coding nucleic acid sequence by associating with them spatially. Generally, a eukaryotic gene promoter contains 100 to 5000 base pairs, although this length range is not intended to limit the term "promoter" as used herein. Although the promoter sequence is generally located at the 5' terminus of a protein-coding or RNA-coding sequence, the promoter sequence may also exist in exon and intron sequences.

The term "transcription start site" as used herein refers to a nucleotide marking the transcription start on the template strand of a gene. The transcription start site may appear on the template strand of the promoter region. A gene can have more than one transcription start site.

The term "identity" or "homology" as used herein means that one oligonucleotide strand (a sense or an antisense strand) of a small activating RNA has similarity to a coding strand or a template strand in a region of the promoter sequence of a target gene. As used herein, the "identity" or "homology" may be at least about 75%, about 79%, about 80%, about 85%, about 90%, about 95%, about 99% or about 100%.

The term "overhang" as used herein refers to non-base-paired nucleotides at the terminus (5' or 3') of an

oligonucleotide strand, which is formed by one strand extending out of the other strand in a double-stranded oligonucleotide. A single-stranded region extending out of the 3' terminus and/or 5' terminus of a duplex is referred to as an overhang.

As used herein, the terms "gene activation", "activating gene expression", "gene up-regulation" and "up-regulating gene expression" can be used interchangeably, and mean an increase in transcription, translation, expression or activity of a certain nucleic acid as determined by measuring the transcriptional level, mRNA level, protein level, enzymatic activity, methylation state, chromatin state or configuration, translation level or the activity or state in a cell or biological system of a gene. These activities or states can be determined directly or indirectly. In addition, "gene activation", "activating gene expression", "gene up-regulation" or "up-regulating gene expression" refers to an increase in activity associated with a nucleic acid sequence, regardless of the mechanism of such activation. For example, the nucleic acid sequence plays a regulatory role as a regulatory sequence, the nucleic acid sequence is transcribed into RNA and the RNA is translated into a protein, thereby increasing the expression of the protein.

As used herein, the terms "small activating RNA", "saRNA", and "small activating nucleic acid molecule" can be used interchangeably, and refer to a nucleic acid molecule that can up-regulate target gene expression and can be composed of the first nucleic acid fragment (antisense nucleic acid strand, also referred to as antisense oligonucleotide strand) containing a nucleotide sequence having sequence identity or homology with the non-coding nucleic acid sequence (e.g., a promoter and an enhancer) of a target gene and the second nucleic acid fragment (sense nucleic acid strand, also referred to as sense oligonucleotide strand) containing a nucleotide sequence complementary with the first nucleic acid fragment, wherein the first nucleic acid fragment and the second nucleic acid fragment form a duplex. The small activating nucleic acid molecule can also be composed of a synthesized or vector-expressed single-stranded RNA molecule that can form a hairpin structure by two complementary regions within the molecule, wherein the first region comprises a nucleotide sequence having sequence identity to the target sequence of a promoter of a gene, and the second region comprises a nucleotide sequence which is complementary to the first region. The length of the duplex region of the small activating nucleic acid molecule is typically about 10 to about 50, about 12 to about 48, about 14 to about 46, about 16 to about 44, about 18 to about 42, about 20 to about 40, about 22 to about 38, about 24 to about 36, about 26 to about 34, and about 28 to about 32 base pairs, and typically about 10, about 15, about 20, about 25, about 30, about 35, about 40, about 45, or about 50 base pairs. In addition, the terms "saRNA", "small activating RNA", and "small activating nucleic acid molecule" also comprise nucleic acids other than the ribonucleotide, including, but not limited to, modified nucleotides or analogues.

As used herein, the term "hotspot" refers to a gene promoter region of at least 30 bp in length. The gathering of targets of functional small activating nucleic acid molecules appears in these hotspot regions, wherein at least 30% of the small activating nucleic acid molecules targeting these hotspot regions can induce a 1.1-fold to 2.89-fold in the mRNA expression of a target gene.

As used herein, the term "synthesis" refers to a method for synthesis of an oligonucleotide, including any method allowing RNA synthesis, such as chemical synthesis, *in vitro* transcription, and/or vector-based expression.

According to the present invention, the expression of the *Thpo*/*THPO* gene is up-regulated by RNA activation, and a related disease (particularly thrombocytopenia) is treated by increasing the expression of the THPO protein. The *Thpo*/*THPO* gene in the present invention is sometimes also called a target gene.

The method for preparing the small activating nucleic acid molecule provided by the present invention comprises sequence design and sequence synthesis.

The synthesis of the sequence of the small activating nucleic acid molecule of the present invention can be performed by adopting a chemical synthesis or can be entrusted to a biotechnology company specialized in nucleic acid synthesis.

Generally speaking, the chemical synthesis comprises the following four steps: (1) synthesis of oligomeric ribonucleotides, (2) deprotection, (3) purification and isolation; and (4) desalination and annealing.

For example, the specific steps for chemically synthesizing the saRNA described herein are as follows:

(1) **Synthesis of oligomeric ribonucleotides**

Synthesis of 1 μM RNA was set in an automatic DNA/RNA synthesizer (e.g., Applied Biosystems EXPEDITE8909), and the coupling time of each cycle was also set as 10 to 15 min. With a solid phase-bonded 5'-O-p-dimethoxytriphenylmethyl-thymidine substrate as an initiator, one base was bonded to the solid phase substrate in the first cycle, and then, in the $n^{th}$ ($19 \geq n \geq 2$) cycle, one base was bonded to the base bonded in the $n-1^{th}$ cycle. This process was repeated until the synthesis of the whole nucleic acid sequence was completed.

(2) **Deprotection**

The solid phase substrate bonded with the saRNA was put into a test tube, and 1 mL of a mixed solution of

ethanol and ammonium hydroxide (volume ratio: 1:3) was added to the test tube. The test tube was then sealed and placed in an incubator, and the mixture was incubated at 25-70 °C for 2 to 30 h. The solution containing the solid phase substrate bonded with the saRNA was filtered, and the filtrate was collected. The solid phase substrate was rinsed with double distilled water twice (1 mL each time), and the filtrate was collected. The collected eluent was combined and dried under vacuum for 1 to 12 h. Then the solution was added with 1 mL of a solution of tetrabutylammonium fluoride in tetrahydrofuran (1 M), let stand at room temperature for 4 to 12 h, followed by addition of 2 mL of *n*-butanol. Precipitate was collected to give a single-stranded crude product of saRNA by high-speed centrifugation.

(3) **Purification and isolation**

The resulting crude product of saRNA was dissolved in 2 mL of triethylamine acetate solution with a concentration of 1 mol/L, and the solution was separated by a reversed-phase C18 column of high pressure liquid chromatography to give a purified single-stranded product of saRNA.

(4) **Desalination and annealing**

Salts were removed by gel filtration (size exclusion chromatography). A single sense oligomeric ribonucleic acid strand and a single antisense oligomeric ribonucleic acid strand were mixed in a 1 to 2 mL of buffer (10 mM Tris, pH = 7.5-8.0, 50 mM NaCl) at a molar ratio of 1:1. The solution was heated to 95 °C, and was then slowly cooled to room temperature to give a solution containing saRNA.

It was discovered in this study that after being introduced into a cell, the aforementioned saRNA could effectively increase *Thpo*/*THPO* mRNA and expression of THPO protein.

The present invention will be further illustrated with reference to specific examples and drawings below. It should be understood that these examples are merely intended to illustrate the present invention rather than limit the scope of the present invention. In the following examples, study methods without specific conditions were generally in accordance with conventional conditions, such as conditions described in Sambrook, et al., Molecular Cloning: Laboratory Manual (New York: Cold Spring Harbor Laboratory Press, 1989), or conditions recommended by the manufacturer.

## EXAMPLES

### Example 1: Design and Synthesis of saRNA Targeting Mouse *Thpo* Gene Promoter

To screen functional saRNAs capable of activating *Thpo* gene expression, a *Thpo* promoter sequence with a length of 500 bp was used as a template, a target with a length of 19 bp was selected from -500 bp upstream of TSS. Target sequences were filtered and the criteria for retaining target sequences were: (1) GC content between 35% and 65%, (2) with less than 5 continuous identical nucleotides, (3) with 3 or less dinucleotide repeat sequences; and (4) with 3 or less trinucleotide repeat sequences. There were 322 target sequences remaining after filtration and these entered the screening process as candidates. Corresponding double-stranded saRNAs were chemically synthesized based on these candidate sequences.

Each sense strand and antisense strand in the double-stranded saRNA used in the study had 21 nucleotides in length. The 19 nucleotides in the 5' region of the first ribonucleic acid strand (sense strand) of the double-stranded saRNA had 100% identity to the target sequence of the promoter, and the 3' terminus of the first ribonucleic acid strand contained a TT sequence. The 19 nucleotides in the 5' region of the second ribonucleic acid strand were complementary to the first ribonucleic acid strand sequence, and the 3' terminus of the second ribonucleic acid strand contained a TT sequence. The aforementioned two strands of the double-stranded saRNA were mixed at a molar ratio of 1:1, and after annealing, a double-stranded saRNA was formed.

The sequence of the mouse *Thpo* promoter region is shown as follows, which corresponds to position 1 to position 500 from 5' to 3' of SEQ ID No: 1 in the sequence listing:

- 500 taatataggc aatacctact ttgccagttg gaaaggggaa atgccaggaa
- 450 agacactcct ctggagcagg ggaatataga aggaaaagca agcaagccag
- 400 catgctgtca gaattcagca gcaggtatac tgttcaggga aaagaaattt
- 350 ggagagccag gaactgagac ttcatccatc taaaacaaga cctctctttc
- 300 ccagagagaa ccgtattctg ccagatttct cctggactaa ccctcttctc
- 250 tctctcactt aggtcccagg ttctcatgtc gccaggacat gagacagttc
- 200 ccgctataca aattctctct agagtgtgtg tgggtggagg agctgggctc
- 150 tattcatgga ccatggtcca gccatgggct ctagtcccag tagacaggaa
- 100 aagaagtcgt catttccggg ggagccttca cctgggcttg gtggctctct

- 50 gctctctgat tgggcggaag tggcctgggc aggcttgtga ccctactac

Example 2: High-throughput Screening of saRNAs Targeting Mouse *Thpo* Promoter

**Cell culture and transfection**

Mouse embryonic liver cells (BNL.CL2) (Shanghai Institutes for Biological Sciences, GNM22) were cultured in DMEM media (Gibco), containing 10% of calf serum (Sigma-Aldrich) and 1% of penicillin/streptomycin (Gibco). The cells were cultured at 37 °C under 5% $CO_2$. The BNL.CL2 cells were plated into a 96-well plate at 3500 cells/well. Following the instructions provided by the manufacturer, RNAiMax (Invitrogen, Carlsbad, CA) was used to transfect small activating RNAs at concentrations of 10 nM (unless otherwise specified), and the duration of transfection was 72 h with 2 replicate wells for each treatment.

(2) **One-step RT-qPCR**

The media were discarded after transfection and each well was washed once with 150 μL of PBS. After discarding PBS, 50 μL of cell lysis buffer was added to each well and samples were incubated at room temperature for 5 min. From each well, 1 μL of cell lysis buffer was subjected to qPCR analysis on an ABI 7500 fast real-time PCR system (Applied Biosystems) using a one-step TB Green™ PrimeScrip™ RT-PCR kit II (Takara, RR086A) and each sample was amplified in 3 replicate wells. The PCR reaction conditions are shown in Table 1.

Table 1. PCR reaction preparation

| Reagent | Volume |
|---|---|
| 2 × One-step TB Green RT-PCR buffer 4 | 2.5 μL |
| PrimeScript 1 step enzyme mixture 2 | 0.2 μL |
| Mixture of forward and reverse primers (5 μM) | 0.4 μL |
| No RNase $dH_2O$ | 1.4 μL |
| Crude lysate (RNA) | 0.5 μL |
| Sum | 5 μL |

The reaction conditions are as follows: Stage 1: reverse transcription reaction: 5 min at 42 °C, 10 s at 95 °C; Stage 2: PCR reaction: 5 s at 95 °C, 20 s at 60 °C, 45 cycles of amplification. *Sdha* and *Tbp* were used as internal control genes. The PCR primers used by *Thpo, Sdha* and *Tbp* are shown in Table 2 and *Thpo* was amplified using *Thpo* F1/R1 primer pair.

Table 2. Primer sequences for RT-qPCR analysis

| Primer | Sequence No. | Sequence (5'-3') |
|---|---|---|
| *Thpo* F1 | SEQ ID NO: 297 | TGGAGCTGACTGATTTGCTCCTG |
| *Thpo* R1 | SEQ ID NO: 298 | AACAGGGATAGACAAAGGGTCG |
| *Sdha* F | SEQ ID NO: 299 | ATGGTCACTAGGGCTGGTTTG |
| *Sdha* R | SEQ ID NO: 300 | ACGACACCCTTCTGTGATGAG |
| *Tbp* F | SEQ ID NO: 301 | CCGTGAATCTTGGCTGTAAACT |
| *Tbp* R | SEQ ID NO: 302 | TGTCCGTGGCTCTCTTATTC |

In order to calculate the expression value ($E_{rel}$) of a *Thpo* gene (target) in an saRNA-transfected sample relative to control treatment (Mock), the Ct values of the target gene and two internal control genes were inserted into Formula 1.

$$E_{rel} = 2^{(CtT_m - CtT_s)} / ((2^{(CtR1_m - CtR1_s)} * 2^{(CtR2_m - CtR2_s)})^{(1/2)})$$

**Formula 1**

wherein $CtT_m$ is the Ct value of the target gene from the control treatment (Mock) sample, CtTs is the Ct value of the

target gene from the saRNA-treated sample. CtRlm is the Ct value of the internal control gene 1 from the Mock-treated sample, CtR1s is the Ct value of the internal control gene 1 from the saRNA-treated sample, CtR2m is the Ct value of the internal control gene 2 from the control treatment sample, and CtR2s is the Ct value of the internal control gene 2 from the saRNA-treated sample.

(3) **Screening of mouse functional saRNAs**

To obtain saRNAs capable of activating *Thpo* transcription, BNL.CL2 cells were transfected by the aforementioned 322 saRNAs at a concentration of 10 nM. According to methods described herein, cells were lysed 72h later and subjected to one-step RT-qPCR analysis to obtain the relative expression value of the *Thpo* gene for each saRNA-treated sample when compared with the control treatment. As shown in Table 3, 18 (5.6%) saRNAs showed high activation, 79 (24.5%) saRNAs showed mild activation, and 142 (69.9%) saRNAs did not affect *Thpo* expression. The maximum activation was 2.89-fold, and the maximum inhibition was 0.26-fold.The saRNAs with activation activity are called activating saRNAs.

Table 3. High-throughput screening results of mouse *Thpo* saRNA

| **saRNA activity** | **$log_2$ value (fold) of changes in *Thpo* mRNA** | **Number of saRNAs** | **Percentage** |
|---|---|---|---|
| High activation | ≥ 0.58 (1.50) ~ ≤ 1.53 (2.89) | 18 | 5.6% |
| Mild activation | ≥ 0.13 (1.10) ~ < 0.58 (1.50) | 79 | 24.5% |
| No activating effect | < 0.13 (1.10) | 225 | 69.9% |
| Sum | | 322 | 100% |

Shown in **FIG. 1,** are the changes in *Thpo* expression of mouse *Thpo* saRNA in descending order. *Thpo* active saRNA sequences, active target sequences, and fold of changes in relative expression of Thpo mRNA shown in Table 4.

Table 4. Mouse active saRNA sequences, active target sequences thereof and changes in expression of mouse *Thpo* mRNA

| saRNA | Target sequence (5'-3') | Sense sequence (5'-3') of saRNA | Antisense sequence (5'-3') of saRNA | Fold of changes in relative expression of *Thpo* mRNA |
|---|---|---|---|---|
| RAG3-493 | GGCAATACCTACTTTGCCA(SEQID NO: 200) | GGCAAUACCUACUUUGCCATT (SEQ ID NO: 6) | UGGCAAAGUAGGUAUUGCCTT (SEQ ID NO: 103) | 1.17 |
| RAG3-490 | AATACCTACTTTGCCAGTT (SEQ ID NO: 201) | AAUACCUACUUUGCCAGUUTT (SEQ ID NO: 7) | AACUGGCAAAGUAGGUAUUTT (SEQ ID NO: 104) | 1.21 |
| RAG3-486 | CCTACTTTGCCAGTTGGAA(SEQID NO: 202) | CCUACUUUGCCAGUUGGAATT (SEQ ID NO: 8) | UUCCAACUGGCAAAGUAGGTT (SEQ ID NO: 105) | 1.76 |
| RAG3-484 | TACTTTGCCAGTTGGAAAG (SEQ ID NO: 203) | UACUUUGCCAGUUGGAAAGTT (SEQ ID NO: 9) | CUUUCCAACUGGCAAAGUATT (SEQ ID NO: 106) | 1.13 |
| RAG3-483 | ACTTTGCCAGTTGGAAAGG (SEQ ID NO: 204) | ACUUUGCCAGUUGGAAAGGTT (SEQ ID NO: 10) | CCUUUCCAACUGGCAAAGUTT (SEQ ID NO: 107) | 1.24 |
| RAG3-479 | TGCCAGTTGGAAAGGGGAA (SEQ ID NO: 205) | UGCCAGUUGGAAAGGGGAATT (SEQ ID NO: 11) | UUCCCCUUUCCAACUGGCATT (SEQ ID NO: 108) | 1.25 |
| RAG3-474 | GTTGGAAAGGGGAAATGCC (SEQ ID NO: 206) | GUUGGAAAGGGGAAAUGCCTT (SEQ ID NO: 12) | GGCAUUUCCCCUUUCCAACTT (SEQ ID NO: 109) | 1.22 |
| RAG3-472 | TGGAAAGGGGAAATGCCAG (SEQ ID NO: 207) | UGGAAAGGGGAAAUGCCAGTT (SEQ ID NO: 13) | CUGGCAUUUCCCCUUUCCATT (SEQ ID NO: 110) | 1.41 |
| RAG3-471 | GGAAAGGGGAAATGCCAGG (SEQ ID NO: 208) | GGAAAGGGGAAAUGCCAGGTT (SEQ ID NO: 14) | CCUGGCAUUUCCCCUUUCCTT (SEQ ID NO: 111) | 1.81 |
| RAG3-466 | GGGGAAATGCCAGGAAAGA (SEQ ID NO: 209) | GGGGAAAUGCCAGGAAAGATT (SEQ ID NO: 15) | UCUUUCCUGGCAUUUCCCCTT (SEQ ID NO: 112) | 1.64 |
| RAG3-465 | GGGAAATGCCAGGAAAGAC (SEQ ID NO: 210) | GGGAAAUGCCAGGAAAGACTT (SEQ ID NO: 16) | GUCUUUCCUGGCAUUUCCCTT (SEQ ID NO: 113) | 1.29 |
| RAG3-461 | AATGCCAGGAAAGACACTC (SEQ ID NO: 211) | AAUGCCAGGAAAGACACUCTT (SEQ ID NO: 17) | GAGUGUCUUUCCUGGCAUUTT (SEQ ID NO: 114) | 1.48 |
| RAG3-460 | ATGCCAGGAAAGACACTCC (SEQ ID NO: 212) | AUGCCAGGAAAGACACUCCTT (SEQ ID NO: 18) | GGAGUGUCUUUCCUGGCAUTT (SEQ ID NO: 115) | 1.14 |
| RAG3-459 | TGCCAGGAAAGACACTCCT (SEQ ID NO: 213) | UGCCAGGAAAGACACUCCUTT (SEQ ID NO: 19) | AGGAGUGUCUUUCCUGGCATT (SEQ ID NO: 116) | 1.10 |

(continued)

| saRNA | Target sequence (5'-3') | Sense sequence (5'-3') of saRNA | Antisense sequence (5'-3') of saRNA | Fold of changes in relative expression of *Thpo* mRNA |
|---|---|---|---|---|
| RAG3-454 | GGAAAGACACTCCTCTGGA (SEQ ID NO: 214) | GGAAAGACACUCCUCUGGATT (SEQ ID NO: 20) | UCCAGAGGAGUGUCUUUCCTT (SEQ ID NO: 117) | 1.24 |
| RAG3-453 | GAAAGACACTCCTCTGGAG (SEQ ID NO: 215) | GAAAGACACUCCUCUGGAGTT (SEQ ID NO: 21) | CUCCAGAGGAGUGUCUUUCTT (SEQ ID NO: 118) | 1.49 |
| RAG3-450 | AGACACTCCTCTGGAGCAG (SEQ ID NO: 216) | AGACACUCCUCUGGAGCAGTT (SEQ ID NO: 22) | CUGCUCCAGAGGAGUGUCUTT (SEQ ID NO: 119) | 1.24 |
| RAG3-449 | GACACTCCTCTGGAGCAGG (SEQ ID NO: 217) | GACACUCCUCUGGAGCAGGTT (SEQ ID NO: 23) | CCUGCUCCAGAGGAGUGUCTT (SEQ ID NO: 120) | 1.14 |
| RAG3-448 | ACACTCCTCTGGAGCAGGG (SEQ ID NO: 218) | ACACUCCUCUGGAGCAGGGTT (SEQ ID NO: 24) | CCCUGCUCCAGAGGAGUGUTT (SEQ ID NO: 121) | 1.54 |
| RAG3-446 | ACTCCTCTGGAGCAGGGGA (SEQ ID NO: 219) | ACUCCUCUGGAGCAGGGGATT (SEQ ID NO: 25) | UCCCCUGCUCCAGAGGAGUTT (SEQ ID NO: 122) | 1.36 |
| RAG3-441 | TCTGGAGCAGGGGAATATA (SEQ ID NO: 220) | UCUGGAGCAGGGGAAUAUATT (SEQ ID NO: 26) | UAUAUUCCCCUGCUCCAGATT (SEQ ID NO: 123) | 1.15 |
| RAG3-440 | CTGGAGCAGGGGAATATAG (SEQ ID NO: 221) | CUGGAGCAGGGGAAUAUAGTT (SEQ ID NO: 27) | CUAUAUUCCCCUGCUCCAGTT (SEQ ID NO: 124) | 1.14 |
| RAG3-439 | TGGAGCAGGGGAATATAGA (SEQ ID NO: 222) | UGGAGCAGGGGAAUAUAGATT (SEQ ID NO: 28) | UCUAUAUUCCCCUGCUCCATT (SEQ ID NO: 125) | 1.10 |
| RAG3-437 | GAGCAGGGGAATATAGAAG (SEQ ID NO: 223) | GAGCAGGGGAAUAUAGAAGTT (SEQ ID NO: 29) | CUUCUAUAUUCCCCUGCUCTT (SEQ ID NO: 126) | 1.12 |
| RAG3-436 | AGCAGGGGAATATAGAAGG (SEQ ID NO: 224) | AGCAGGGGAAUAUAGAAGGTT (SEQ ID NO: 30) | CCUUCUAUAUUCCCCUGCUTT (SEQ ID NO: 127) | 1.99 |
| RAG3-435 | GCAGGGGAATATAGAAGGA (SEQ ID NO: 225) | GCAGGGGAAUAUAGAAGGATT (SEQ ID NO: 31) | UCCUUCUAUAUUCCCCUGCTT (SEQ ID NO: 128) | 1.83 |
| RAG3-434 | CAGGGGAATATAGAAGGAA (SEQ ID NO: 226) | CAGGGGAAUAUAGAAGGAATT (SEQ ID NO: 32) | UUCCUUCUAUAUUCCCCUGTT (SEQ ID NO: 129) | 1.47 |
| RAG3-433 | AGGGGAATATAGAAGGAAA (SEQ ID NO: 227) | AGGGGAAUAUAGAAGGAAATT (SEQ ID NO: 33) | UUUCCUUCUAUAUUCCCCUTT (SEQ ID NO: 130) | 1.13 |

(continued)

| saRNA | Target sequence (5'-3') | Sense sequence (5'-3') of saRNA | Antisense sequence (5'-3') of saRNA | Fold of changes in relative expression of *Thpo* mRNA |
|---|---|---|---|---|
| RAG3-432 | GGGGAATATAGAAGGAAAA (SEQ ID NO: 228) | GGGGAAUAUAGAAGGAAAATT (SEQ ID NO: 34) | UUUUCCUUCUAUAUUCCCCTT (SEQ ID NO: 131) | 1.10 |
| RAG3-430 | GGAATATAGAAGGAAAAGC (SEQ ID NO: 229) | GGAAUAUAGAAGGAAAAGCTT (SEQ ID NO: 35) | GCUUUUCCUUCUAUAUUCCTT (SEQ ID NO: 132) | 1.20 |
| RAG3-426 | TATAGAAGGAAAAGCAAGC (SEQ ID NO: 230) | UAUAGAAGGAAAAGCAAGCTT (SEQ ID NO: 36) | GCUUGCUUUUCCUUCUAUATT (SEQ ID NO: 133) | 1.18 |
| RAG3-425 | ATAGAAGGAAAAGCAAGCA (SEQ ID NO: 231) | AUAGAAGGAAAAGCAAGCATT (SEQ ID NO: 37) | UGCUUGCUUUUCCUUCUAUTT (SEQ ID NO: 134) | 1.57 |
| RAG3-424 | TAGAAGGAAAAGCAAGCAA (SEQ ID NO: 232) | UAGAAGGAAAAGCAAGCAATT (SEQ ID NO: 38) | UUGCUUGCUUUUCCUUCUATT (SEQ ID NO: 135) | 1.79 |
| RAG3-421 | AAGGAAAAGCAAGCAAGCC (SEQ ID NO: 233) | AAGGAAAAGCAAGCAAGCCTT (SEQ ID NO: 39) | GGCUUGCUUGCUUUUCCUUTT (SEQ ID NO: 136) | 1.11 |
| RAG3-420 | AGGAAAAGCAAGCAAGCCA (SEQ ID NO: 234) | AGGAAAAGCAAGCAAGCCATT (SEQ ID NO: 40) | UGGCUUGCUUGCUUUUCCUTT (SEQ ID NO: 137) | 1.25 |
| RAG3-419 | GGAAAAGCAAGCAAGCCAG (SEQ ID NO: 235) | GGAAAAGCAAGCAAGCCAGTT (SEQ ID NO: 41) | CUGGCUUGCUUGCUUUUCCTT (SEQ ID NO: 138) | 1.51 |
| RAG3-418 | GAAAAGCAAGCAAGCCAGC (SEQ ID NO: 236) | GAAAAGCAAGCAAGCCAGCTT (SEQ ID NO: 42) | GCUGGCUUGCUUGCUUUUCTT (SEQ ID NO: 139) | 1.12 |
| RAG3-417 | AAAAGCAAGCAAGCCAGCA (SEQ ID NO: 237) | AAAAGCAAGCAAGCCAGCATT (SEQ ID NO: 43) | UGCUGGCUUGCUUGCUUUUTT (SEQ ID NO: 140) | 1.19 |
| RAG3-414 | AGCAAGCAAGCCAGCATGC (SEQ ID NO: 238) | AGCAAGCAAGCCAGCAUGCTT (SEQ ID NO: 44) | GCAUGCUGGCUUGCUUGCUTT (SEQ ID NO: 141) | 1.27 |
| RAG3-410 | AGCAAGCCAGCATGCTGTC (SEQ ID NO: 239) | AGCAAGCCAGCAUGCUGUCTT (SEQ ID NO: 45) | GACAGCAUGCUGGCUUGCUTT (SEQ ID NO: 142) | 1.39 |
| RAG3-409 | GCAAGCCAGCATGCTGTCA (SEQ ID NO: 240) | GCAAGCCAGCAUGCUGUCATT (SEQ ID NO: 46) | UGACAGCAUGCUGGCUUGCTT (SEQ ID NO: 143) | 1.38 |
| RAG3-408 | CAAGCCAGCATGCTGTCAG (SEQ ID NO: 241) | CAAGCCAGCAUGCUGUCAGTT (SEQ ID NO: 47) | CUGACAGCAUGCUGGCUUGTT (SEQ ID NO: 144) | 2.09 |

(continued)

| saRNA | Target sequence (5'-3') | Sense sequence (5'-3') of saRNA | Antisense sequence (5'-3') of saRNA | Fold of changes in relative expression of *Thpo* mRNA |
|---|---|---|---|---|
| RAG3-405 | GCCAGCATGCTGTCAGAAT (SEQ ID NO: 242) | GCCAGCAUGCUGUCAGAAUTT (SEQ ID NO: 48) | AUUCUGACAGCAUGCUGGCTT (SEQ ID NO: 145) | 1.53 |
| RAG3-404 | CCAGCATGCTGTCAGAATT (SEQ ID NO: 243) | CCAGCAUGCUGUCAGAAUUTT (SEQ ID NO: 49) | AAUUCUGACAGCAUGCUGGTT (SEQ ID NO: 146) | 1.18 |
| RAG3-397 | GCTGTCAGAATTCAGCAGC (SEQ ID NO: 244) | GCUGUCAGAAUUCAGCAGCTT (SEQ ID NO: 50) | GCUGCUGAAUUCUGACAGCTT (SEQ ID NO: 147) | 1.34 |
| RAG3-396 | CTGTCAGAATTCAGCAGCA (SEQ ID NO: 245) | CUGUCAGAAUUCAGCAGCATT (SEQ ID NO: 51) | UGCUGCUGAAUUCUGACAGTT (SEQ ID NO: 148) | 1.17 |
| RAG3-378 | AGGTATACTGTTCAGGGAA (SEQ ID NO: 246) | AGGUAUACUGUUCAGGGAATT (SEQ ID NO: 52) | UUCCCUGAACAGUAUACCUTT (SEQ ID NO: 149) | 1.36 |
| RAG3-377 | GGTATACTGTTCAGGGAAA (SEQ ID NO: 247) | GGUAUACUGUUCAGGGAAATT (SEQ ID NO: 53) | UUUCCCUGAACAGUAUACCTT (SEQ ID NO: 150) | 1.32 |
| RAG3-376 | GTATACTGTTCAGGGAAAA (SEQ ID NO: 248) | GUAUACUGUUCAGGGAAAATT (SEQ ID NO: 54) | UUUUCCCUGAACAGUAUACTT (SEQ ID NO: 151) | 1.67 |
| RAG3-375 | TATACTGTTCAGGGAAAAG (SEQ ID NO: 249) | UAUACUGUUCAGGGAAAAGTT (SEQ ID NO: 55) | CUUUUCCCUGAACAGUAUATT (SEQ ID NO: 152) | 1.11 |
| RAG3-360 | AAAGAAATTTGGAGAGCCA (SEQ ID NO: 250) | AAAGAAAUUUGGAGAGCCATT (SEQ ID NO: 56) | UGGCUCUCCAAAUUUCUUUTT (SEQ ID NO: 153) | 1.12 |
| RAG3-337 | CTGAGACTTCATCCATCTA (SEQ ID NO: 251) | CUGAGACUUCAUCCAUCUATT (SEQ ID NO: 57) | UAGAUGGAUGAAGUCUCAGTT (SEQ ID NO: 154) | 1.15 |
| RAG3-290 | CCGTATTCTGCCAGATTTC (SEQ ID NO: 252) | CCGUAUUCUGCCAGAUUUCTT (SEQ ID NO: 58) | GAAAUCUGGCAGAAUACGGTT (SEQ ID NO: 155) | 1.14 |
| RAG3-273 | TCTCCTGGACTAACCCTCT (SEQ ID NO: 253) | UCUCCUGGACUAACCCUCUTT (SEQ ID NO: 59) | AGAGGGUUAGUCCAGGAGATT (SEQ ID NO: 156) | 2.89 |
| RAG3-272 | CTCCTGGACTAACCCTCTT (SEQ ID NO: 254) | CUCCUGGACUAACCCUCUUTT (SEQ ID NO: 60) | AAGAGGGUUAGUCCAGGAGTT (SEQ ID NO: 157) | 1.89 |
| RAG3-271 | TCCTGGACTAACCCTCTTC (SEQ ID NO: 255) | UCCUGGACUAACCCUCUUCTT (SEQ ID NO: 61) | GAAGAGGGUUAGUCCAGGATT (SEQ ID NO: 158) | 1.17 |

(continued)

| saRNA | Target sequence (5'-3') | Sense sequence (5'-3') of saRNA | Antisense sequence (5'-3') of saRNA | Fold of changes in relative expression of *Thpo* mRNA |
|---|---|---|---|---|
| RAG3-244 | ACTTAGGTCCCAGGTTCTC (SEQ ID NO: 256) | ACUUAGGUCCCAGGUUCUCTT (SEQ ID NO: 62) | GAGAACCUGGGACCUAAGUTT (SEQ ID NO: 159) | 1.17 |
| RAG3-243 | CTTAGGTCCCAGGTTCTCA (SEQ ID NO: 257) | CUUAGGUCCCAGGUUCUCATT (SEQ ID NO: 63) | UGAGAACCUGGGACCUAAGTT (SEQ ID NO: 160) | 1.45 |
| RAG3-242 | TTAGGTCCCAGGTTCTCAT (SEQ ID NO: 258) | UUAGGUCCCAGGUUCUCAUTT (SEQ ID NO: 64) | AUGAGAACCUGGGACCUAATT (SEQ ID NO: 161) | 1.27 |
| RAG3-239 | GGTCCCAGGTTCTCATGTC (SEQ ID NO: 259) | GGUCCCAGGUUCUCAUGUCTT (SEQ ID NO: 65) | GACAUGAGAACCUGGGACCTT (SEQ ID NO: 162) | 1.10 |
| RAG3-229 | TCTCATGTCGCCAGGACAT (SEQ ID NO: 260) | UCUCAUGUCGCCAGGACAUTT (SEQ ID NO: 66) | AUGUCCUGGCGACAUGAGATT (SEQ ID NO: 163) | 1.12 |
| RAG3-227 | TCATGTCGCCAGGACATGA (SEQ ID NO: 261) | UCAUGUCGCCAGGACAUGATT (SEQ ID NO: 67) | UCAUGUCCUGGCGACAUGATT (SEQ ID NO: 164) | 1.39 |
| RAG3-226 | CATGTCGCCAGGACATGAG (SEQ ID NO: 262) | CAUGUCGCCAGGACAUGAGTT (SEQ ID NO: 68) | CUCAUGUCCUGGCGACAUGTT (SEQ ID NO: 165) | 1.30 |
| RAG3-219 | CCAGGACATGAGACAGTTC (SEQ ID NO: 263) | CCAGGACAUGAGACAGUUCTT (SEQ ID NO: 69) | GAACUGUCUCAUGUCCUGGTT (SEQ ID NO: 166) | 1.24 |
| RAG3-215 | GACATGAGACAGTTCCCGC (SEQ ID NO: 264) | GACAUGAGACAGUUCCCGCTT (SEQ ID NO: 70) | GCGGGAACUGUCUCAUGUCTT (SEQ ID NO: 167) | 1.32 |
| RAG3-214 | ACATGAGACAGTTCCCGCT (SEQ ID NO: 265) | ACAUGAGACAGUUCCCGCUTT (SEQ ID NO: 71) | AGCGGGAACUGUCUCAUGUTT (SEQ ID NO: 168) | 1.28 |
| RAG3-206 | CAGTTCCCGCTATACAAAT (SEQ ID NO: 266) | CAGUUCCCGCUAUACAAAUTT (SEQ ID NO: 72) | AUUUGUAUAGCGGGAACUGTT (SEQ ID NO: 169) | 1.31 |
| RAG3-205 | AGTTCCCGCTATACAAATT (SEQ ID NO: 267) | AGUUCCCGCUAUACAAAUUTT (SEQ ID NO: 73) | AAUUUGUAUAGCGGGAACUTT (SEQ ID NO: 170) | 1.14 |
| RAG3-204 | GTTCCCGCTATACAAATTC (SEQ ID NO: 268) | GUUCCCGCUAUACAAAUUCTT (SEQ ID NO: 74) | GAAUUUGUAUAGCGGGAACTT (SEQ ID NO: 171) | 1.65 |
| RAG3-203 | TTCCCGCTATACAAATTCT (SEQ ID NO: 269) | UUCCCGCUAUACAAAUUCUTT (SEQ ID NO: 75) | AGAAUUUGUAUAGCGGGAATT (SEQ ID NO: 172) | 1.42 |

(continued)

| saRNA | Target sequence (5'-3') | Sense sequence (5'-3') of saRNA | Antisense sequence (5'-3') of saRNA | Fold of changes in relative expression of *Thpo* mRNA |
|---|---|---|---|---|
| RAG3-202 | TCCCGCTATACAAATTCTC (SEQ ID NO: 270) | UCCCGCUAUACAAAUUCUCTT (SEQ ID NO: 76) | GAGAAUUUGUAUAGCGGGATT (SEQ ID NO: 173) | 1.31 |
| RAG3-164 | GAGGAGCTGGGCTCTATTC (SEQ ID NO: 271) | GAGGAGCUGGGCUCUAUUCTT (SEQ ID NO: 77) | GAAUAGAGCCCAGCUCCUCTT (SEQ ID NO: 174) | 1.13 |
| RAG3-158 | CTGGGCTCTATTCATGGAC (SEQ ID NO: 272) | CUGGGCUCUAUUCAUGGACTT (SEQ ID NO: 78) | GUCCAUGAAUAGAGCCCAGTT (SEQ ID NO: 175) | 1.17 |
| RAG3-153 | CTCTATTCATGGACCATGG (SEQ ID NO: 273) | CUCUAUUCAUGGACCAUGGTT (SEQ ID NO: 79) | CCAUGGUCCAUGAAUAGAGTT (SEQ ID NO: 176) | 1.17 |
| RAG3-152 | TCTATTCATGGACCATGGT (SEQ ID NO: 274) | UCUAUUCAUGGACCAUGGUTT (SEQ ID NO: 80) | ACCAUGGUCCAUGAAUAGATT (SEQ ID NO: 177) | 1.10 |
| RAG3-151 | CTATTCATGGACCATGGTC (SEQ ID NO: 275) | CUAUUCAUGGACCAUGGUCTT (SEQ ID NO: 81) | GACCAUGGUCCAUGAAUAGTT (SEQ ID NO: 178) | 1.44 |
| RAG3-150 | TATTCATGGACCATGGTCC (SEQ ID NO: 276) | UAUUCAUGGACCAUGGUCCTT (SEQ ID NO: 82) | GGACCAUGGUCCAUGAAUATT (SEQ ID NO: 179) | 1.24 |
| RAG3-147 | TCATGGACCATGGTCCAGC (SEQ ID NO: 277) | UCAUGGACCAUGGUCCAGCTT (SEQ ID NO: 83) | GCUGGACCAUGGUCCAUGATT (SEQ ID NO: 180) | 1.31 |
| RAG3-146 | CATGGACCATGGTCCAGCC (SEQ ID NO: 278) | CAUGGACCAUGGUCCAGCCTT (SEQ ID NO: 84) | GGCUGGACCAUGGUCCAUGTT (SEQ ID NO: 181) | 1.44 |
| RAG3-142 | GACCATGGTCCAGCCATGG (SEQ ID NO: 279) | GACCAUGGUCCAGCCAUGGTT (SEQ ID NO: 85) | CCAUGGCUGGACCAUGGUCTT (SEQ ID NO: 182) | 1.26 |
| RAG3-134 | TCCAGCCATGGGCTCTAGT (SEQ ID NO: 280) | UCCAGCCAUGGGCUCUAGUTT (SEQ ID NO: 86) | ACUAGAGCCCAUGGCUGGATT (SEQ ID NO: 183) | 1.41 |
| RAG3-133 | CCAGCCATGGGCTCTAGTC (SEQ ID NO: 281) | CCAGCCAUGGGCUCUAGUCTT (SEQ ID NO: 87) | GACUAGAGCCCAUGGCUGGTT (SEQ ID NO: 184) | 1.11 |
| RAG3-129 | CCATGGGCTCTAGTCCCAG (SEQ ID NO: 282) | CCAUGGGCUCUAGUCCCAGTT (SEQ ID NO: 88) | CUGGGACUAGAGCCCAUGGTT (SEQ ID NO: 185) | 1.30 |
| RAG3-122 | CTCTAGTCCCAGTAGACAG (SEQ ID NO: 283) | CUCUAGUCCCAGUAGACAGTT (SEQ ID NO: 89) | CUGUCUACUGGGACUAGAGTT (SEQ ID NO: 186) | 1.51 |

(continued)

| saRNA | Target sequence (5'-3') | Sense sequence (5'-3') of saRNA | Antisense sequence (5'-3') of saRNA | Fold of changes in relative expression of *Thpo* mRNA |
|---|---|---|---|---|
| RAG3-121 | TCTAGTCCCAGTAGACAGG (SEQ ID NO: 284) | UCUAGUCCCAGUAGACAGGTT (SEQ ID NO: 90) | CCUGUCUACUGGGACUAGATT (SEQ ID NO: 187) | 1.72 |
| RAG3-111 | GTAGACAGGAAAAGAAGTC (SEQ ID NO: 285) | GUAGACAGGAAAAGAAGUCTT (SEQ ID NO: 91) | GACUUCUUUUCCUGUCUACTT (SEQ ID NO: 188) | 1.26 |
| RAG3-110 | TAGACAGGAAAAGAAGTCG (SEQ ID NO: 286) | UAGACAGGAAAAGAAGUCGTT (SEQ ID NO: 92) | CGACUUCUUUUCCUGUCUATT (SEQ ID NO: 189) | 1.25 |
| RAG3-101 | AAAGAAGTCGTCATTTCCG (SEQ ID NO: 287) | AAAGAAGUCGUCAUUUCCGTT (SEQ ID NO: 93) | CGGAAAUGACGACUUCUUUTT (SEQ ID NO: 190) | 1.14 |
| RAG3-100 | AAGAAGTCGTCATTTCCGG (SEQ ID NO: 288) | AAGAAGUCGUCAUUUCCGGTT (SEQ ID NO: 94) | CCGGAAAUGACGACUUCUUTT (SEQ ID NO: 191) | 1.15 |
| RAG3-099 | AGAAGTCGTCATTTCCGGG (SEQ ID NO: 289) | AGAAGUCGUCAUUUCCGGGTT (SEQ ID NO: 95) | CCCGGAAAUGACGACUUCUTT (SEQ ID NO: 192) | 1.12 |
| RAG3-078 | AGCCTTCACCTGGGCTTGG (SEQ ID NO: 290) | AGCCUUCACCUGGGCUUGGTT (SEQ ID NO: 96) | CCAAGCCCAGGUGAAGGCUTT (SEQ ID NO: 193) | 1.10 |
| RAG3-071 | ACCTGGGCTTGGTGGCTCT (SEQ ID NO: 291) | ACCUGGGCUUGGUGGCUCUTT (SEQ ID NO: 97) | AGAGCCACCAAGCCCAGGUTT (SEQ ID NO: 194) | 1.10 |
| RAG3-047 | CTCTGATTGGGCGGAAGTG (SEQ ID NO: 292) | CUCUGAUUGGGCGGAAGUGTT (SEQ ID NO: 98) | CACUUCCGCCCAAUCAGAGTT (SEQ ID NO: 195) | 1.66 |
| RAG3-046 | TCTGATTGGGCGGAAGTGG (SEQ ID NO: 293) | UCUGAUUGGGCGGAAGUGGTT (SEQ ID NO: 99) | CCACUUCCGCCCAAUCAGATT (SEQ ID NO: 196) | 1.15 |
| RAG3-044 | TGATTGGGCGGAAGTGGCC (SEQ ID NO: 294) | UGAUUGGGCGGAAGUGGCCTT (SEQ ID NO: 100) | GGCCACUUCCGCCCAAUCATT (SEQ ID NO: 197) | 1.20 |
| RAG3-023 | GGCAGGCTTGTGACCCTAC (SEQ ID NO: 295) | GGCAGGCUUGUGACCCUACTT (SEQ ID NO: 101) | GUAGGGUCACAAGCCUGCCTT (SEQ ID NO: 198) | 1.27 |
| RAG3-022 | GCAGGCTTGTGACCCTACT (SEQ ID NO: 296) | GCAGGCUUGUGACCCUACUTT (SEQ ID NO: 102) | AGUAGGGUCACAAGCCUGCTT (SEQ ID NO: 199) | 1.11 |

It can be clearly seen from the arrangement of activities of the 322 saRNAs according to their positions in the *Thpo* promoter region that the functional saRNAs are gathered together, that is, in certain promoter regions, the activating saRNAs gathered in specific "hotspot" regions **(FIG. 2).** As shown in **FIG. 2**, 3 hotspot regions respectively appeared in region (H1) from -493 to -356, region (H2) from -273 to -183, and region (H3) from -164 to -80 of the promoter, showing a high gathering of the activating saRNAs. The analysis results indicates that the activating saRNAs were not randomly distributed on the promoter but existed in the specific hotspot regions.

The sequence of hotspot H1 (5' to 3': -493 to -356) corresponds to position 1 to position 138, from 5' to 3', of SEQ ID NO: 2 in the sequence listing:

1 ggcaatacct actttgccag ttggaaaggg gaaatgccag gaaagacact
51 cctctggagc aggggaatat agaaggaaaa gcaagcaagc cagcatgctg
101 tcagaattca gcagcaggta tactgttcag ggaaaaga

The sequence of the hotspot H2 (5' to 3': -273 to -183) corresponds to position 1 to position 91, from 5' to 3', of SEQ ID NO: 3 in the sequence listing:

1 tctcctggac taaccctctt ctctctctca cttaggtccc aggttctcat
51 gtcgccagga catgagacag ttcccgctat acaaattctc t

The sequence of the hotspot H3 (5' to 3': -164 to -80) corresponds to position 1 to position 85, from 5' to 3', of SEQ ID NO: 4 in the sequence listing:

1 gaggagctgg gctctattca tggaccatgg tccagccatg ggctctagtc
51 ccagtagaca ggaaaagaag tcgtcatttc cgggg

Example 3: SaRNA Promoting Expression of *Thpo* mRNA in a Mouse Liver Cell

**(1) Cell culture and transfection**

Cell culture was described in Example 2. Mouse embryonic liver cells (BNL.CL2) were plated into a 6-well plate, at $1\times10^5$ cells/well. According to the instructions provided by the manufacturer, RNAiMax (Invitrogen, Carlsbad, CA) was used to transfect mouse *Thpo* saRNA at a concentration of 10 nM, transfection duration was 72 h, and 2 replicate wells were used in each treatment.

**(2) Two-step RT-qPCR**

After transfection, the media were discarded, each well was added with 500 μL of cell lysis buffer, and incubation was performed at room temperature for 5 min. RNA was extracted using Qiagen RNeasy kit, reverse transcribed and subjected to qPCR analysis on ABI 7500 fast real-time PCR system (Applied Biosystems), and each sample was repeatedly amplified in 3 replicate wells. PCR reaction conditions were shown in Tables 5 and 6.

Table 5. RT reaction preparation

| Reagent (RT reaction 1) | Volume | | Reagent (RT reaction 2) | Volume |
|---|---|---|---|---|
| 5 × gDNA Eraser buffer | 2 μL | | 5 × PrimeScript buffer 2 | 4 μL |
| gDNA Eraser | 1 μl | | PrimeScript RT enzyme mixture 1 | 1 μL |
| Total amount of RNA (1 μg) + D.W | 7 μL | | RT primer mixture | 1 μL |
| Final volume | 10 μL | | No RNase $dH_2O$ | 4 μL |
| 2 min at 42°C and stored at 4°C | | | RT reaction 1 | 10 μL |
| | | | Final volume | 20 μL |
| | | | 15 min at 37°C, 5s at 85°C and stored at 4°C | |
| | | | | |
| | | | | |

Table 6. RT-qPCR reaction preparation

| Reagent | Volume |
|---|---|
| SYBR Premix Ex Taq II (2 ×) | 5 μL |
| ROX reference dye II (50 ×) | 0.2 μL |
| Mixture of forward and reverse primers (5 μM) | 0.8 μL |
| cDNA (RT products) | 4 μL |
| Sum | 10 μL |

The reaction conditions were as follows: 30s at 95 °C, 5s at 95 °C, 30s at 60 °C, 40 cycles of amplification. The amplification of the *Tbp* gene with the *Thpo* F1/R1 primer pair served as an internal control. Amplification primers are shown in a Table 2.

To calculate the expression value ($E_{rel}$) of the *Thpo* (target gene) of an saRNA-transfected sample relative to the control treatment (Mock),Ct values of the target gene and one internal control gene were inserted into Formula 2 for calculation.

$$E_{rel} = 2^{(CtTm-CtTs)} / 2^{(CtRm-CtRs)}$$

***Formula 2***

**FIG. 3** shows the relative mRNA expression values of *Thpo* after treatment of cells with different mouse saRNAs. The relative mRNA expression of mouse *Thpo* small interfering RNA (siRNA) in the DS03-432i group was reduced by 53.5% compared to the control group using the control treatment as a blank transfection control. This result indicates that this siRNA was successful as a small interfering RNA control for transfection. The following groups with different saRNAs include DS03-0007 (RAG3-424), DS03-0014 (RAG3-425), DS03-0022 (RAG3-243) and DS03-0024 (RAG3-151), and showed higher relative mRNA expression values when compared to the control group, which increased by 25.5%, 16.5%, 16% and 41%, respectively. This result indicates that randomly selected activating saRNAs are capable of promoting expression of *Thpo* mRNA in BNL.CL2 cells.

Example 4: Mouse *Thpo* saRNA Promoting Expression of *Thpo* mRNA in LPC-H12 cells

The cell culture was described in Example 2. Mouse liver cancer cells (LPC-H12) (BNBIO, BNCC101945) were plated into a 6-well plate at $2 \times 10^5$ cells/well. According to the instructions provided by the manufacturer, RNAiMax (Invitrogen, Carlsbad, CA) was used to transfect mouse Thpo saRNA at a concentration of 10 nM for a duration of 72h. Two replicate wells were used for each treatment. Two-step RT-qPCR was described in Example 3.

**FIG. 4** shows the relative mRNA expression values of *Thpo* after cells were treated with different saRNAs. The relative mRNA expression of the mouse siRNA (DS03-432i) group was reduced by 47% compared to the control group using the control treatment as a blank transfection control. This result indicates that this siRNA was successful as a small interfering RNA control transfection. The relative mRNA expression values of DS03-0007 (RAG3-424), DS03-0019 (RAG3-453), DS03-0022 (RAG3-243), DS03-0024 (RAG3-151) and DS03-0027 (RAG3-134) groups were higher than those of a control group, and the relative mRNA expression values were increased by 52%, 90%, 30.5%, 11.5% and 34%, respectively, and the activation effect of DS03-0019 was particularly obvious (90%). The results show that randomly selected activating saRNAs are capable of promoting expression of *Thpo* mRNA to varying degrees in LPC-H12 cells.

Example 5: Mouse *Thpo* saRNA Promoting Expression of *Thpo* mRNA in Primary Mouse Liver Cells

**Culture and transfection of primary mouse liver cells**

C57 female mice (6 to 8 weeks old, approximately 25 g in weight, purchased from Comparative Medicine Center of Yangzhou University) were acclimated in the mouse animal room for 3 days and primary mouse liver cells were isolated by a two-step perfusion method. The specific operation of the two-step perfusion method is described as follows: Anesthetized mice were maintained using isoflurane and sterilized with alcohol, and the abdominal cavity of the mice was opened with scissors to expose hepatic portal vein and inferior vena cava; a needle was inserted into the hepatic portal vein, and a peristaltic pump was used to infuse pre-perfusion (HBSS+HEPES+EGTA) at 37°C at a rate of 1 to 2 mL/min, and the mouse liver swelled and turned white, indicating successful perfusion; the inferior vena cava was cut,

and the perfusion rate was gradually increased to 7 to 9 mL/min, perfusion was performed for 10 to 15 min; collagenase solution for digestion was added for about 15 min, and the perfusion rate was 5 to 8 mL/min. After digestion, the liver was transferred to a petri dish and liver cells were collected, passed through a 200-mesh filter, washed 3 times with DMEM medium, and centrifuged (100 g) at 4 °C for 5 min. The cells were cultured at $2\times10^5$ cells/well (collagen-coated well plate) in DMEM-L medium containing 10% serum, the DMEM-L medium without serum was replaced after 4 to 6 h, and the cells were cultured overnight at 37 °C under 5% $CO_2$. The next day, RNAiMax (Invitrogen, Carlsbad, CA) was used to transfect mouse *Thpo* saRNA at a concentration of 10 nM for a duration of 72 h. Two replicate wells were used in each treatment. Two-step RT-qPCR was described in Example 3.

**FIG. 5** shows the relative mRNA expression values of *Thpo* after treatment of cells with different mouse saRNAs. The relative mRNA expression values of DS03-0007 (RAG3-424), DS03-0012 (RAG3-204), DS03-0014 (RAG3-425), DS03-0019 (RAG3-453), DS03-0022 (RAG3-243), DS03-0024 (RAG3-151) and DS03-0027 (RAG3-134) groups were higher than control group and all increased by 33.5%, 25.5%, 51.5%, 44.5%, 28.5%, 26% and 34.5%, respectively, compared to the control group using the control treatment as a blank transfection control. The results show that randomly selected activating saRNAs are capable of promoting expression of *Thpo* mRNA in primary mouse liver cells.

Example 6: *In vivo* Assay of *Thpo* saRNA Activation of *Thpo* Gene in Mouse

**Preparation of oligonucleotide formulation**

Based on the published method of preparing Lipid Nanoparticle (LNP) *(see,* CN104873464B, CN102625696B, US 9,394,234 B2, WO 2010/144740A1, US 8,158,601 B2, and US 8,802,644 B2), LNP was prepared with an improved method. The resulting LNP was used to entrap oligonucleotide. The specific method used is described as follows.

Stock solution preparation *of Thpo* siRNA (DS03-432i) and saRNA (RAG3-151/DS03-0024): stock solutions *of Thpo* siRNA (DS03-432i) and saRNA (RAG3-151/DS03-0024) with 5 mg/mL theoretical final concentration were prepared by dissolving siRNA (DS03-432i) and saRNA (RAG3-151/DS03-0024) with DEPC water. The appropriate amounts of siRNA (DS03-432i) and saRNA (RAG3-151/DS03-0024) were taken and diluted 200-fold with PBS to determine the concentrations of siRNA (DS03-432i) and saRNA (DS03-0024).

Table 7. RNA concentrations of siRNA (DS03-432i) and saRNA (DS03-0024)

| Sample | RNA determination concentration (ng/μL) | Dilution factor | Concentration of RNA stock solution (mg/mL) |
|---|---|---|---|
| DS03-432i | 41 | 200 | 8.2 |
| DS03-0024 | 41 | 200 | 8.2 |

Table 8: Sequences of dsCon2 andsiRNA (DS03-432i)

| RNA duplexes | Sense sequence (5'-3') | Antisense sequence (5'-3') |
|---|---|---|
| dsCon2 | ACUACUGAGUGACAGUAGATT (SEQ ID NO: 303) | UCUACUGUCACUCAGUAGUTT (SEQ ID NO: 304) |
| DS03-432i | GGACUUUAGCCUGGGAGAATT (SEQ ID NO: 305) | UUCUCCCAGGCUAAAGUCCTT (SEQ ID NO: 306) |

Preparation of D-Lin-MC3 lipid: in a patent publication method (see Lipid Formulation, Publication No.: US 2010/0324120 A1), the D-Lin-MC3 lipid was synthesized chemically as follows.

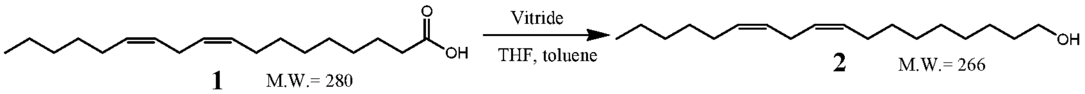


A dry 1 L glass reactor was purged with dry argon and cooled to 0 °C using an acetone-dry ice bath. 25 g of reaction 1 (linoleic acid) and 300 mL of THF were added. 60 mL of sodium bis(2-methoxyethoxy)aluminum dihydride (70% wt/vol) in toluene was slowly added dropwise to a reactor. After the completion of dropwise addition, the mixture was carried out at room temperature for 2 h. A small sample was taken and starting material reaction was monitored by

TLC. After the completion of reaction, saturated sodium sulfate solution was slowly added. After the completion of addition, 130 mL of ethyl acetate was added dropwise over 30 min, and vigorously stirred. The reaction mixture was filtered, the solid was washed with 90 mL of ethyl acetate, and the organic phases were combined and concentrated. The product was dissolved in 80 mL of ethyl acetate, washed twice with water, and dried over anhydrous sodium sulfate. The organic phases were filtered and concentrated, organic solvent was removed using a vacuum pump to give product 2 (17.49 g).

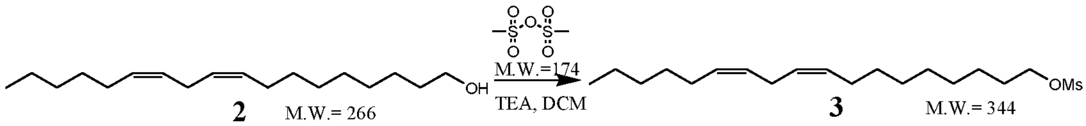


A dry 1 L glass reactor was purged with dry argon, 17 g of product 2 and 150 mL of dichloromethane were then added, 40 mL of triethylamine and 1 g of 4-dimethylaminopyridine were then added, and the mixture was cooled to -10 °C using an acetone-dry ice bath. 30 gof methanesulfonic anhydridewas dissolved in 45 mL of dichloromethane and slowly added dropwise to the reactor. After the completion of dropwise addition of the reaction mixture, the reaction was continued at 0 °C for 1 h. Starting material reaction was monitored by TLC. After the completion of reaction, 80 mL of ice water was added to the reaction mixture, the mixture was separated, and the aqueous phase was extracted with 40 mL of dichloromethane. The organic phases were combined, washed with dilute hydrochloric acid, water and saturated NaCl solution, and dried over anhydrous sodium sulfate. The organic phases were filtered and concentrated, organic solvent was removed using a vacuum pump to give product 3 (22.3 g).

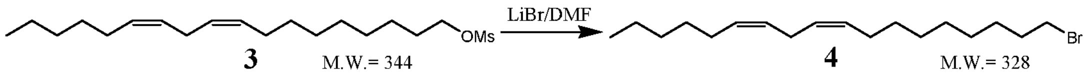


A dry 1 L glass reactor was purged with dry argon and then 100 mL of DMF and 22 g of product 3 were added and the solution was cooled to -10 °C using an acetone-dry ice bath. 8.5 g of LiBr was dissolved in 80 mL of DMF, stirred and slowly added dropwise to the reactor. After the completion of dropwise addition, the reaction mixture was heated to 45 °C and stirred for reaction for 18 to 20 h. Starting material reaction was monitored by TLC. After the completion of reaction, 300 mL of water were added and extracted with 240 mL of *n*-hexane, and the aqueous phase was further extracted with *n*-hexane. The organic phases were combined, washed with water and saturated NaCl solution and dried over anhydrous sodium sulfate. The organic phases were filtered and concentrated, organic solvent was removed using a vacuum pump to give crude product (16.76 g). The mixture was purified with 60 to 120 mesh silica gel (*n*-hexane as mobile phase) to give product 4 (13.86 g).

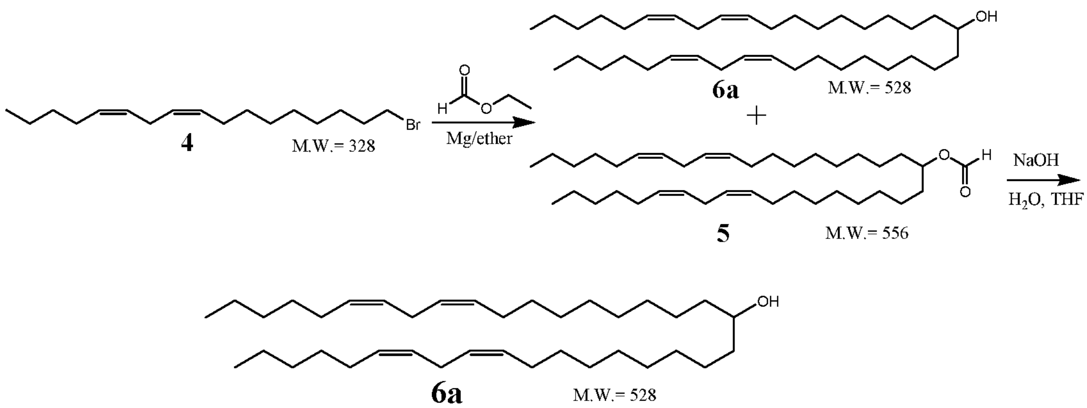


A dry 1 L three-neck flask was purged with dry argon, and a reflux device was additionally arranged. 2 g of magnesium turnings and 12 mL of anhydrous diethyl ether were added into the reactor. 13 g of product 4 were dissolved in 40 mL of anhydrous diethyl ether. Under the protection of argon, 8 mL of reation mixture was added dropwise to the reactor and 0.2 mL of dibromomethane was added further. The reactor was warmed to 40 °C in a water bath. After the reaction started, the heating was stopped and the remaining 32 mL of the reaction mixture was added dropwise to the reactor, and the mixture was allowed to keep a slight reflux. After the completion of dropwise addition, the reaction mixture was heated to maintain the reflux state (about 45 °C) and reacted for 1 h. A small sample was quenched with

water and the starting material reaction was monitored by TLC. After the completion of starting materials reaction, the reaction mixture was cooled to below 10 °C by an ice bath, and then ethyl formate solution was slowly added. After the completion of dropwise addition, the mixture was carried out at room temperature for 1 h. Then 56 mL of ice-water and 10% of sulphuric acid solution were added, the organic phases were separated and the aqueous phase was extracted with diethyl ether. The organic phases were combined, washed with saturated NaCl solution and dried over anhydrous sodium sulfate. The organic phase was filtered and concentrated, organic solvent was removed using a vacuum pump to give crude product (13 g). The crude product was dissolved in 100 mL of 85% ethanol, 7 g of NaOH solid was added, stirred at room temperature for 24 h and the starting material reaction was monitored by TLC. After the completion of reaction, the reaction mixture was extracted with diethyl ether, and the organic phases were combined and washed with saturated NaCl solution. The reaction mixture was dried over anhydrous sodium sulfate. The organic phases were filtered and concentrated. The crude product was purified with 60 to 120 mesh silica gel (4% ether/*n*-hexane) to give product 6 (10.4 g).

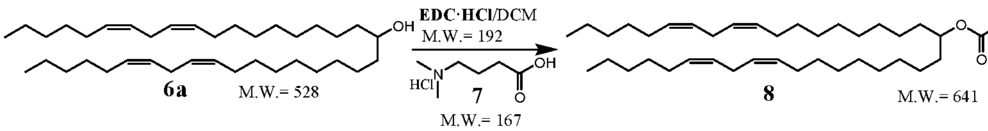


The reaction product 6 (3 g) was added into a 100 mL flask and dissolved in dichloromethane (24 mL), the reaction product 7 (4-(dimethylamino)butanoic acid hydrochloride) (1.1 g) was added, then diisopropylethylamine (1.5 mL) and 4-dimethylaminopyridine (0.09 g) were added. After stirring at room temperature for 5 min, the reaction mixture was added with EDC HCl (1.7 g) and stirred at room temperature overnight. Starting material reaction was monitored by TLC. After the completion of reaction, the reaction mixture was diluted with 20 mL of dichloromethane. Then the reaction mixture was washed with saturated $NaHCO_3$solution, water and saturated NaCl solution, dried with anhydrous $Na_2SO_4$and filtered, and the organic phases were concentrated to give about crude product (3.3 g). The reaction mixture was isolated using chromatography (serial elution with 1% to 5% methanol/dichloromethane) to give product 8 (Dlin-MC3-DMA, 2.9 g). $^1$H-NMR ($CDCl_3$, 400 MHz), δ=5.44-5.22 (m, 8H), 4.95-4.79 (m, 1H), 2.81-2.63 (m, 4H),2.36-2.21 (m, 4H), 2.12-1.92 (m, 9H), 1.84-1.67 (m, 2H), 1.48 (d, J=5.4, 4H), 1.36-1.14 (m, 39H), 0.91-0.81 (m, 6H).

Preparation of PEG-c-DMA

PEG-c-DMA was prepared by the chemical method as follows:

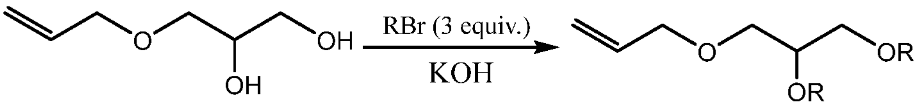


1 2

$R = C_{14}H_{29}$

*Preparation of product 2*

A dry 1 L glass reactor was purged with dry argon, a mixture of 9 g of the reaction product **1**, (3-allyloxy-1,2-propanediol), 60 g of 1-bromotetradecane and 15.5 g of potassium hydroxide was placed in 500 mL of anhydrous benzene, refluxed overnight, and removed with a Dean-Stark splitter. After the mixture was cooled to room temperature, the reaction mixture was diluted with 200mL of anhydrous benzene. The organic phases were washed with water and saturated NaCl solution, and dried over anhydrous magnesium sulfate. The organic solvent was removed by vacuum pump to give a clear yellow oil (53 g). The reaction mixture was purified with silica gel column (particle size 230 to 400 mesh, 1300 mL) and serial elution with 0 to 5% ethyl acetate/n-hexane was taken as mobile phase. Product 2 was 33 g.

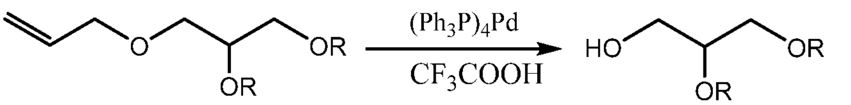


2 3

*Preparation of product 3*

A dry 1 L glass reactor was purged with dry argon and 45 mL of trifluoroacetic acid was added to give the ethanol solution of product 2 (33 g of product 2 was dissolved in 500 mL of ethanol). After 10 g of tetrakis(triphenylphosphine)palladium was added, the mixture was refluxed overnight in the absence of light. After the completion of reaction, the organic solvent was removed by vacuum pump to give a brown oil. The crude product was purified with silica gel column (particle size 230 to 400 mesh, 1500 mL) and serial elution with 0 to 1% methanol/dichloromethane was taken as mobile phase. The final product 3 (19 g) was a yellow wax.

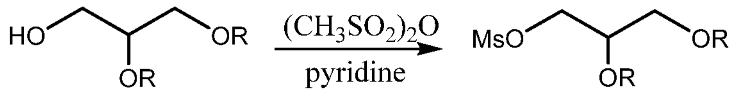


3 4

*Preparation of product 4*

A dry 1 L glass reactor was purged with dry argon and 13.8 g of methanesulfonic anhydride was dissolved in 150 mL of dichloromethane in the reactor. And 6.4 mL of pyridine was added dropwise to the reactor. 19 g of product **3** was dissolved in 150 mL of methylene chloride and added to reaction system. The reaction mixture was stirred at room temperature under the protection of argon overnight. After the completion of reaction, the product was diluted with 200 mL of dichloromethane, washed with water and saturated NaCl solution, and dried over anhydrous magnesium sulfate. The organic solvent was removed by rotary evaporation to give a crude yellow waxy product **4** (22.1 g).

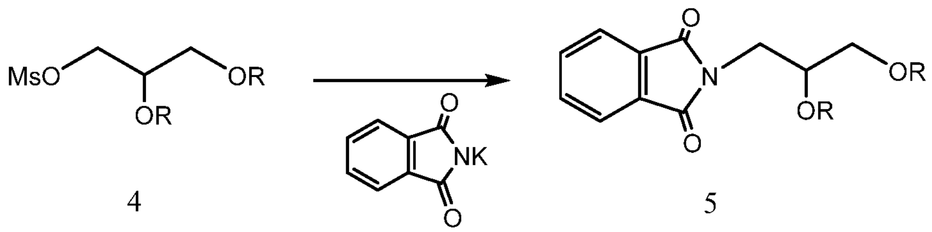

4 5

*Preparation of product 5*

A dry 1 L glass reactor was purged with dry argon and 22 g of product **4** and 19 g of potassium phthalimide were mixed, dissolved in 400 mL of DMF solution, heated to 70 °C and stirred overnight to give a yellow suspension. After the reaction solution was cooled to room temperature, and poured into 800 mL of ice-water. The aqueous phase was washed with ethyl acetate. The organic phases were combined, washed with water and saturated NaCl solution, and dried over anhydrous magnesium sulfate. The organic solvent was removed by vacuum pump. The mixture was dissolved in 500 mL of *n*-hexane, filtered and washed with 100 mL of hexane. The organic solvent was removed by rotary evaporation to give 20.7 g of a crude wax. The crude product was purified with silica gel column (particle size 230 to 400 mesh, 600 mL) and serial elution with 0 to 5% ethyl acetate/*n*-hexane was taken as mobile phase. A pure white waxy product **5** (13.4 g) was given.

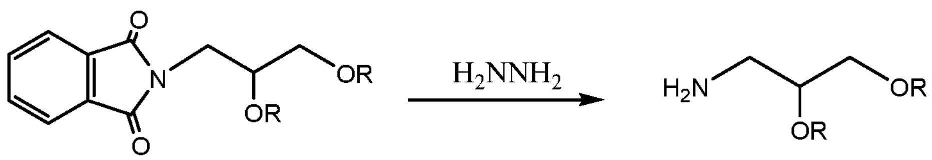


5 6

*Preparation of product 6*

13.4 g of product **5** and 15.8 mL of hydrazine hydrate was dissolved in 300 mL of ethanol solution at reflux overnight to give a white solid. The reaction mixture was cooled to room temperature and then filtered. The solid was

washed twice with ethanol (100 mL). The organic phases were combined, the organic solvent was removed by rotary evaporation and dissolved in 500 mL of chloroform and filtered. The solid was washed once with 100 mL of chloroform. The organic phases were combined, washed with water and saturated NaCl solution, and dried over anhydrous magnesium sulfate. The organic solvent was removed by vacuum pump to give a light-colored oily crude product (10.6 g). The crude product was purified with silica gel column (particle size 230 to 400 mesh, 300 mL) and serial elution with 0 to 10% methanol/chloroform was taken as mobile phase. A light-colored oily crude product (10 g) was given.

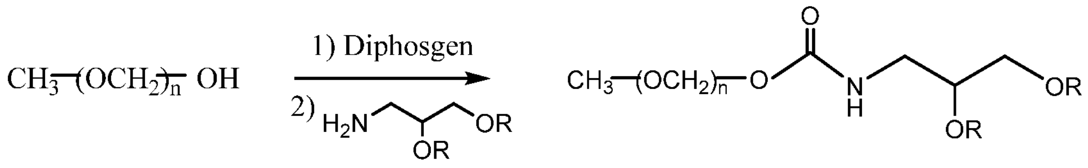


6

7

*Preparation of product 7*

29.5 g of monomethoxy polyethylene glycol 2000 was dissolved in 150 mL of anhydrous benzene, and the organic solvent was removed by rotary evaporation. The product was dissolved in 250 mL of anhydrous dichloromethane, added into a 1 L dry glass reactor and purged with dry argon. 7.3 g of trichloromethyl chloroformate was added and stirred at room temperature for 3 h. The organic solvent was removed by rotary evaporation, 150 mL of anhydrous benzene was added, and the organic solvent was removed by rotary evaporation. 10 g of product 6 was dissolved in 150 mL of dichloromethane, then added with 3.2 mL of triethylamine and added to 1 L of the reaction under the protection of argon and stirred at room temperature overnight. The reaction product was diluted with 200 mL of dichloromethane. The organic phases were washed with 1% HCl solution, then washed with water, 0.1% sodium carbonate solution, and water and saturated NaCl solution, and finally dried over anhydrous magnesium sulfate. The organic solvent was removed by vacuum pump to give a light-colored slurry crude product (46 g). The crude product was purified with silica gel column (particle size 230 to 400 mesh, 1600 mL). The serial elution with 0 to 10% methanol/chloroform was taken as mobile phase to obtain the product PEG-c-DMA and the remaining reactant which was white or light yellow wax. The waxy mixture was added to 150 mL of diethyl ether to dissolve unreacted reactant. The reaction mixture was filtered, and the solid was washed twice with diethyl ether, dried under vacuum to give product 7 (32.2 g). $^1H$ NMR ($CDCl_3$, 400MHz) δ:5.15 (1H, m, NH), 4.21 (2H, m, $CH_2$), 3.80-3.1 (8H, m), 3.60 (130H, s, $OCH_2CH_2O$), 3.34 (3H, s, $OCH_3$), 2.42 (2H, m, NCH2), 1.49 (4H, m, 2 × $CH_2$), 1.21 (44H, s, 22 × $CH_2$), 0.84 (6H, t, 2 × $CH_3$) ppm.

Preparation of lipid stock solution: D-Lin-MC3 Lipid, 1,2-distearoyl-sn-glycero-3-phosphocholine, i.e., DSPC (Avanti Polar Lipid, 850365), cholesterol (Sigma, C3045), and PEG-C-DMA were equilibrated at room temperature for about 30 min before weighing. D-Lin-MC3 lipid was formulated into 40 mg/mL of stock solution with 100% ethanol, cholesterol was formulated into 20 mg/mL of stock solution with 100% ethanol, and DSPC was formulated into 20 mg/mL stock solution with 100% ethanol. Long-acting lipid was formulated as 50 mg/mL of stock solution in 100% ethanol.

Preparation of pre-formed vesicles (PFV): D-Lin-MC3, cholesterol, DSPC, PEG-c-DMA and anhydrous ethanol were formulated into lipid ethanol solutions in a molar ratio of 40:40:10:10, and the required amount of each lipid was shown in Table 9. After all lipids were dissolved, the above lipid ethanol solution was added to the formulation buffer (50 mM of citric acid, pH = 3.0) with stirring to a final ethanol concentration of 30% (v/v). At room temperature, the Avanti lipid extruder equipped with a layer of Nuclepore Membrane was repeatedly extruded to give PFV with uniform particle size. The PFV extruded by the extruder was measured for particle size using a particle size analyzer.

Table 9. Requirements for lipid in F113 formulation

| Total volume: 10 mL | | |
|---|---|---|
| Volume of ethanol: 3 mL of ethanol with a concentration of 30% (v/v) | | |
| **Lipid** | **Concentration (mg/mL)** | **Required amount (mL)** |
| D-Lin-MC3 | 40 | 1.25 |
| DSPC | 20 | 0.462 |
| Cholesterol | 20 | 0.904 |
| PEG-c-DMA | 50 | 0.596 |
| Addition of ethanol | | 0.286 |

To prepare liposome formulations of siRNA (DS03-432i) and saRNA (RAG3-151/DS03-0024), PFV was dispensed into 15 mL Falcon tubes and pre-equilibrated for 5 min in a water bath at 35°C. Two hundred microliters (8.2 mg/mL) of siRNA (DS03-432i) and saRNA (DS03-0024) stock solutions were formulated into 0.4 mL of a solution containing siRNA, formulation buffer, ethanol solution and a solution containing saRNA, formulation buffer, ethanol solution at a ratio of RNA stock solution:formulation buffer:ethanol solution = 200 μL:100 μL:100 μL. The siRNA (DS03-432i) or the saRNA (RAG3-151/DS03-0024)/formulation buffer/ethanol solution was slowly added to the above PFV and incubated at 35 °C for 30 min. The siRNA (DS03-432i) and saRNA (RAG3-151/DS03-0024) liposome formulations were then dialyzed overnight using an MD24 dialysis bag, 1 × phosphate buffer solution (PBS), and the product recovered after dialysis was the LNP-entrapped oligonucleotide used in the experiment. The LNP-entrapped oligonucleotide was filtered and sterilized using a 0.2 μm needle head-type filter, and 20 μL of sterilized products was used to determine the encapsulation rate.

**(2) LNP-entrapped oligonucleotide injected into tail vein of mice**

Fifteen male Balb/c mice (purchased from SPF (Beijing) Biotechnology Co.,Ltd.), 6 to 8 weeks old, and weights of 25g were prepared for the experiments. After acclimatation in a mouse animal room for 3 days, the mice were randomly divided into 3 groups with 5 mice in each group based on body weight. A control group (PBS), LNP-entrapped *Thpo-siRNA* (DS03-432i, 5 mg/kg) and LNP-entrapped *Thpo-saRNA* (RAG3-151/DS03-0024, 5 mg/kg) was administered respectively by tail vein injection at a dose of 20 mL/kg. Seventy two hours after administration, mice were anesthetized with isoflurane and 200 μL of blood was collected by cardiac puncture for analysis of THPO protein concentration in serum. After blood collection was completed, the mice were sacrificed by carbon dioxide inhalation asphyxiation. The experimental mice to be euthanized were placed in a closable experimental box to release a large amount of carbon dioxide, so that the experimental mice were suffocated and died.

**(3) Mouse serum THPO protein determined using ELISA**

Forty microliters of whole blood from mice was let stand at room temperature for 30 min, then centrifuged (4 °C, 13000 rpm, 15 min) to collect serum. An ELISA kit with mouse THPO (R&D Systems, MTP00) was used to determine the content of THPO in the collected serum. The procedures were briefly described below, which were according to the instructions provided in the kit. Sufficient strips (provided by the kit) were prepared and 50 μL of RD1-23 was added into two replicate wells for each group. Fifty microliters of standard, control, and sample was added and the mixture was uniformly mixed by lightly beating the strips and then placing them at room temperature for 2h. The liquid was discarded, the mixture was washed 4 times with 400 μL of wash buffer, and 100 μL of mouse Thpo conjugate was added to each well and let to stand at room temperature for 2h. Next, the liquid was discarded, the mixture was washed 4 times with 400 μL of wash buffer, and 100 μL of substrate was added to each well and let to stand at room temperature in the absence of light for 30 minutes. After the completion of the reaction, 100 μL of stop solution was added to each well, the mixture was mixed uniformly by lightly beating the strips, and absorbance at 450 nm was determined using an enzyme labeling instrument (Infinite M200 Pro). A guarantee curve was prepared using a known standard concentration (4000 pg/mL), provided by the kit. The absorbance of the sample to be tested was subtracted from that of a blank control (0 pg/mL) and the THPO protein concentration (pg/mL) was calculated from standard curve.

**(4) Statistical analysis**

Differences between groups of continuous variables were compared using one-way analysis of variance (ANOVA) and between groups were compared using Tukey's multiple comparisons. The groups with less than 0.05 of P value were significantly different.

**FIG. 6** shows the concentration of Thpo protein in mice serum of different treatment groups. Compared with the control group (PBS), the Thpo protein content of the LNP-entrapped *Thpo-siRNA* (DS03-432i) group was reduced by 16.1%, and was statistically significant *($p < 0.001$)*. This result indicates that LNP-entrapped Thpo-siRNA (DS03-432i) can efficiently deliver *Thpo*-siRNA and significantly reduce the content of *Thpo*-siRNA protein in mice which is expected and consistent with *in vitro* cell study results. Compared with the control group, the Thpo protein content of the LNP-entrapped *Thpo*-saRNA (DS03-0024) group was reduced by 32.9%, and was statistically significant *($p < 0.001$)*. This result indicates that LNP-entrapped *Thpo*-saRNA (DS03-0024) can efficiently deliver *Thpo*-saRNA and significantly increase the content of Thpo protein in mice. N = 5; (N, number of samples); *** represents $p < 0.001$.

Example 7: Tail Vein Delivery of saRNA in Mice Increasing the Level of Serum Thpo and Number of Peripheral Blood Platelets

Thirty two male Balb/c mice (6 to 8 weeks old, 28 to 30g in weight, purchased from Comparative Medicine Center of Yangzhou University) were prepared. After acclimated in the mouse animal room for 7 days, the above mice were randomly divided into 4 groups with 8 mice in each group based on body weight. The four groups consisted of a control group (PBS) and 3 groups receiving different doses of LNP-entrapped *Thpo*-saRNA (DS03-0024, 1 mg/kg; DS03-0024, 3 mg/kg, and DS03-0024, 6 mg/kg), resepctively. The drug was administered by tail vein injection at a dose of 10 mL/kg. Forty microliters of blood was collected by tail vein 1 day before administration, and at 2 days, 4 days, 6 days and 9 days after dose administration to analyze the concentration of Thpo in the serum. To assay the number of peripheral blood platelets, the mice were anesthetized with isoflurane and 800 μL of blood was collected by cardiac puncture 17 days after the dose administration. After the blood collection was completed, the mice were sacrificed by carbon dioxide inhalation asphyxiation. The experimental mice to be euthanized were placed in a closable experimental box to release a large amount of carbon dioxide, so that the experimental mice were suffocated and died.

(2) Assay of peripheral blood platelet in mice

To assay peripheral blood platelets in mice, five microliters of whole blood and 5 μL of 0.01% heparin sodium was uniformly mixed and added to 90 μL of physiological saline (1:20 dilution). The mixture was gently mixed and added to 200 μL of the upper layer of blood platelet separation liquid (Solarbio, P1620) and centrifuged at room temperature for 15 min at 250 ×g. Thereafter, 20 μL of the uppermost blood platelet enrichment layer was collected. Eighty microliters of 1×FASC standing buffer (R&D Systems, FC001) was added to the collected supernatant and 1 μL of CD41a antibody (ThermoFisher, 11-0411-82) was added to bind platelets.The mixture was uniformly mixed and let stand at room temperature in the absence of light for 30 minutes. After completion of the reaction, the mixture was centrifuged at room temperature (800 g, 5 min), the precipitate was resuspended in 200 μL of PBS, and transferred to an enzyme-labeled assay plate determine platelet content. The preparation of the oligonucleotide formulation and methods for injecting LNP-entrapped oligonucleotides into the tail veins of mice are described in Example 6.

**FIG. 7** shows that *Thpo* saRNA increases the concentration of Thpo protein in mice serum of different treatment groups. As shown in **FIG. 7A,** the baseline concentration of Thpo protein in mice serum was determined the day before administration and there was no significant difference in the level of serum for Thpo protein in the 4 groups of mice. **FIG. 7B** shows the concentrations of Thpo protein in mice serum collected 2 days after administration. The level of Thpo protein serum in the DS03-0024-3 mg/kg group is increased compared to the control group (PBS), however not statistically significant. The concentration of Thpo protein in the DS03-0024-6 mg/kg group increased by 23.9%, with a statistical significance of $p < 0.0001$. This result indicates that LNP-entrapped *Thpo*-saRNA can quickly and efficiently deliver *Thpo*-saRNA and significantly increase the concentration of Thpo protein in mice serum.

**FIG. 8** shows that *Thpo* saRNA increased the number of platelets in mice 6 days after administration. Compared to the control group (PBS), the two groups of LNP-entrapped *Thpo*-saRNA, DS03-0024, 1 mg/kg and DS03-0024, 3 mg/kg,0 increased, though not statistically significant, especially in the number of platelets in the LNP-entrapped *Thpo*-saRNA group (DS03-0024, 3 mg/kg), which increased by 88.6%. The number of platelets in the LNP-entrapped *Thpo*-saRNA group (DS03-0024, 6 mg/kg) increased by 138.7%, with a statistical significance of $p < 0.05$. This result indicates that the LNP-entrapped Thpo-saRNA group (DS03-0024, 6 mg/kg) can increase the number of platelets 6 days after administration.

**FIG. 9** shows that *Thpo* saRNA increased the number of platelets in mice 17 days after administration. The number of platelets in mice from the two groups of LNP-entrapped *Thpo*-saRNA, DS03-0024, 1 mg/kg and DS03-0024, 3 mg/kg, respectively, determined 17 days after administration, increased significantly compared to that of the control group (PBS), increasing by 53.8% and 53.9%, respectively, wwith a statistical significance ($P < 0.05$). The number of platelets in the LNP-entrapped *Thpo*-saRNA group (DS03-0024, 6 mg/kg) tended to increase as well. This result indicates that the increases observed in platelet number caused by saRNA can persist for at least 17 days.

Example 8: Design and Synthesis of saRNA Targeting Human *THPO* Gene Promoter

To screen functional saRNAs capable of activating human *THPO* gene expression, a *THPO* promoter region sequence with a length of 1382 bp was used as a template and target sequences with a length of 19 bp were selected from -1382 bp upstream of TSS. The target sequences were then chosen using the following criteria : (1) having a GC content between 40% and 65%; (2) with less than 5 continuous identical nucleotides; (3) with 3 or less dinucleotide repeat sequences; and (4) with 3 or less trinucleotide repeat sequences. After the selection, the remaining 833 target sequences entered the screening process as candidates. Corresponding double-stranded saRNAs were chemically synthesized based on these candidate sequences.

In the study, each sense strand and antisense strand in the double-stranded saRNA had 21 nucleotides in length. The 19 nucleotides in the 5' region of the first ribonucleic acid strand (sense strand) of saRNA had 100% identity to the target sequence of the promoter, and the 3' terminus of the first ribonucleic acid strand contained a TT sequence. The 19 nucleotides in the 5' region of the second ribonucleic acid strand were complementary to the first ribonucleic acid strand sequence, and the 3' terminus of the second ribonucleic acid strand contained a TT sequence. The aforementioned two strands of the saRNA were mixed at a molar ratio of 1:1, and after annealing, a double-stranded saRNA was formed.

The sequence of human *THPO* promoter is shown as follows, which corresponds to position 1 to position -1382 from 5' to 3' of SEQ ID No: 600 in the sequence listing:

- 1382 gtccatcagc cgatctcccc ctgcctgggc ccacagcgcc ccccaaaccc
- 1332 tcaccaccct ctctcactgc ctagcctgcc tccctacctt ctctctgagg
- 1282 tcgctcctca ttcttgtgtt acccaggaca gggacctagc cagaaaccgg
- 1232 cagcattccc ccttctgtgg agtgacagta tctccctctc attgtaactt
- 1182 atcctcaggc gcattcgaca gtcccctctt gctttctcac ccccttcctt
- 1132 cacccaaggg accctctgcc tctccagccc actcccagcc tcctttctct
- 1082 tggttccctg gtcatgcctg cctccctgtc tcctgtctct ccctccaca
- 1032 cacacccact atcctcccag ctatcccagc accctccttc ctaatcttgg
- 982 gagacatctc gtctggctgg acgggaaaat tccaggatct aggccacact
- 932 tctcagcaga catgcccatc cttggggagg aggaacagga gagagcctga
- 882 ggaagttctg ggggacaggg ggatgatggg atcaaggtca ggccaggaag
- 832 cccctgagga cagagactgt ggggagactg ggactgggaa gaaagcaaag
- 782 gagctagagc cagggccaaa ggaaaagggg ggccagcagg gaggtatttg
- 732 cgggggaggt ccagcagctg tctttcctaa gacagggaca catgggcctg
- 682 gttattcctc ttgtcacatg tggaacggta ggagatggaa gacggagaca
- 632 gaacaagcaa aggagggccc tgggcacaga ggtctgtgtg tgtagccatc
- 582 taagccactg gaccccagca gacgagcacc taagctcagg cttaacccag
- 532 tgcacgtgtg cgcacataca tgtgccccgc acctgacagt ccactcaacc
- 482 cgtccaaacc ctttccccat aacaccaacc cataacagga gatttctctc
- 432 atgtgggcaa tatccgtgtt cccacttcga aagggggaat gacaagatag
- 382 gactccctag gggattacag aaagaaaagc aggaaagcaa gcatcctgtt
- 332 ggatttcagc agcaggtatg atgtccaggg aaaagaaatt tggatagcca
- 282 gggagtgaaa accccaccaa tcttaaacaa gacctctgtg cttcttcccc
- 232 agcaacacaa atgtcctgcc agattcctcc tggaaaaaaa cttctgctcc
- 182 tgtccccctc caggtcccag gttgcccatg tccaggaaaa gatggatccc
- 132 cctatccaaa tcttctccgt ggtgtgtgtg ggtggaggag tggaccctgg
- 82 tccaggcagg ggctccaggg aagagaaggc gtcacttccg ggggccttca
- 32 ccagtgtctg gtggctccct tctctgattg gg

Example 9: High-throughput Screening of saRNAs Targeting Human *THPO* Promoter

**(1) Cell culture and transfection**

A human liver cancer cell HepG2 (ATCC) was cultured in MEM media (Gibco) containing 10% of calf serum (Sigma-Aldrich) and 1% of penicillin/streptomycin (Gibco). The cells were cultured at 37 °C in 5% $CO_2$. The HepG2 cells were plated at 5000 cells/well in 96-well plates. RNAiMax (Invitrogen, Carlsbad, CA) was used to transfect small activating RNAs at a concentration of 25 nM for a durationof 72 h, with 2 replicate wells for each treatment.

**(2) One-step RT-qPCR**

After transfection, the media were discarded, and each well was washed once with 150 μL of PBS once. After washing, 50 μL of cell lysis buffer was added to each well, and incubation was performed at room temperature for 5 min. One microliter of cell lysis buffer was taken from each well and subjected to qPCR analysis on an ABI 7500 fast real-time PCR system (Applied Biosystems) using a one-step TB Green™ PrimeScrip™ RT-PCR kit II (Takara, RR086A). Each sample was repeatedly amplified in 3 replicate wells. PCR reaction conditions are shown in Table 1.

Table 10: PCR reaction preparation

| Reagent | Volume |
|---|---|
| 2 × One-step TB Green RT-PCR buffer 4 | 2.5 μL |
| PrimeScript 1 step enzyme mixture 2 | 0.2 μL |
| Mixture of forward and reverse primers (5 μM) | 0.4 μL |
| No RNase $dH_2O$ | 1.4 μL |
| Crude lysate (RNA) | 0.5 μL |
| Sum | 5 μE |

Reaction conditions were as follows: reverse transcription reaction (stage 1): 5 min at 42°C, 10s at 95°C; PCR reaction (stage 2): 5s at 95°C, 20 s at 60°C, 45 cycles of amplification. *TBP* was used as an internal control gene. PCR primers used for *THPO* and *TBP* were shown in Table 10, wherein *THPO* was amplified using the *THPO* F1/R1 primer pair.

Table 11: Primer sequences for RT-qPCR analysis

| Primer | Sequence No. | Sequence (5'-3') |
|---|---|---|
| *THPO* F | SEQ ID NO: 309 | TCGTGACTCCCATGTCCTTC |
| *THPO* R | SEQ ID NO: 310 | TGCCTTGGTCTCCTCCATCT |
| *TBP* F | SEQ ID NO: 311 | TGCTCACCCACCAACAATTTAG |
| *TBP* R | SEQ ID NO: 312 | TCTGCTCTGACTTTAGCACCTG |
| *HPRT1* F | SEQ ID NO: 313 | AAAGATGGTCAAGGTCGCAAG |
| *HPRT1* R | SEQ ID NO: 314 | TAGTCAAGGGCATATCCTACAAC |

In order to calculate the expression value ($E_{rel}$) of the *THPO* gene (target) of an saRNA-transfected sample relative to control treatment (Mock), the Ct values of the target gene and one internal control gene are substituted into Formula 2 as shown in Example 3 for calculation.

$$E_{rel} = 2^{(CtTm-CtTs)} / 2^{(CtRm-CtRs)}$$

**Formula 2**

wherein CtTm was the Ct value of the target gene from Mock sample; CtTs was the Ct value of the target gene from the saRNA-treated sample; CtRm was the Ct value of the internal control gene from Mock-treated sample; and CtRs was the Ct value of the internal control gene from the saRNA-treated sample.

**(3) Screening of functional saRNAs**

To obtain saRNAs capable of activating *THPO* transcription, HepG2 cells were transfected by the aforementioned 833 saRNAs with a transfection concentration of 25 nM. Seventy two hours later, using the same method as described above, the cells were lysed and subjected to one-step RT-qPCR analysis to obtain the relative expression value (compared to the control treatment group) of *THPO* gene in each saRNA-treated sample. As shown in Table 11, 158 (19 %) saRNAs showed high activation, 243 (29.2 %) saRNAs showed mild activation, and 432 (51.8 %) saRNAs did not affect *THPO* expression. The maximum activation was 5.15-fold; the maximum inhibition was 0.09-fold; and the saRNAs with activation activity were called activating saRNAs.

Table 12: High-throughput screening results of human *THPO* saRNA

| saRNA activity | $log_2$ value (fold) of changes in *THPO* mRNA | Number of saRNAs | Percentage |
|---|---|---|---|
| High activation | ≥ 0.58 (1.50) ~ ≤ 2.36 (5.15) | 158 | 19% |
| Mild activation | ≥ 0.13 (1.10) ~ < 0.58 (1.50) | 243 | 29.2% |
| No activating effect | < 0.13 (1.10) | 432 | 51.8% |
| Sum | | 833 | 100% |

Shown in **FIG.10** is the descending order of changes in *THPO* expression of mouse *THPO* saRNA. *THPO* active saRNA sequence and *THPO* mRNA expression were changed as shown in Table 13.

Table 13: Human active saRNA sequences and target sequences thereof and changes in expression of *THPO* mRNA

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-889 | AGCCTGAGGAAGTTCTGGG (SEQ ID NO: 606) | AGCCUGAGGAAGUUCUGGGTT (SEQ ID NO: 1048) | CCCAGAACUUCCUCAGGCUT<br>T<br>(SEQ ID NO: 1490) | 5.1 |
| RAG3A-723 | TCCAGCAGCTGTCTTTCCT (SEQ ID NO: 607) | UCCAGCAGCUGUCUUUCCUTT (SEQ ID NO: 1049) | AGGAAAGACAGCUGCUGGAT<br>T<br>(SEQ ID NO: 1491) | 4.1 |
| RAG3A-1069 | ATGCCTGCCTCCCTGTCTC (SEQ ID NO: 608) | AUGCCUGCCUCCCUGUCUCTT (SEQ ID NO: 1050) | GAGACAGGGAGGCAGGCAUT<br>T<br>(SEQ ID NO: 1492) | 4 |
| RAG3A-541 | TTAACCCAGTGCACGTGTG (SEQ ID NO: 609) | UUAACCCAGUGCACGUGUGTT (SEQ ID NO: 1051) | CACACGUGCACUGGGUUAAT<br>T<br>(SEQ ID NO: 1493) | 3.4 |
| RAG3A-480 | TCCAAACCCTTTCCCCATA (SEQ ID NO: 610) | UCCAAACCCUUUCCCCAUATT (SEQ ID NO: 1052) | UAUGGGGAAAGGGUUUGGAT<br>T<br>(SEQ ID NO: 1494) | 3.1 |
| RAG3A-980 | GACATCTCGTCTGGCTGGA (SEQ ID NO: 611) | GACAUCUCGUCUGGCUGGATT (SEQ ID NO: 1053) | UCCAGCCAGACGAGAUGUCT<br>T<br>(SEQ ID NO: 1495) | 3.1 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-462 | AACACCAACCCATAACAGG (SEQ ID NO: 612) | AACACCAACCCAUAACAGGTT (SEQ ID NO: 1054) | CCUGUUAUGGGUUGGUGUUT T (SEQ ID NO: 1496) | 3 |
| RAG3A-787 | CAAAGGAGCTAGAGCCAG G (SEQ ID NO: 613) | CAAAGGAGCUAGAGCCAGGTT (SEQ ID NO: 1055) | CCUGGCUCUAGCUCCUUUGT T (SEQ ID NO: 1497) | 2.9 |
| RAG3A-232 | AGCAACACAAATGTCCTGC (SEQ ID NO: 614) | AGCAACACAAAUGUCCUGCTT (SEQ ID NO: 1056) | GCAGGACAUUUGUGUUGCUT T (SEQ ID NO: 1498) | 2.9 |
| RAG3A-727 | GAGGTCCAGCAGCTGTCTT (SEQ ID NO: 615) | GAGGUCCAGCAGCUGUCUUTT (SEQ ID NO: 1057) | AAGACAGCUGCUGGACCUCT T (SEQ ID NO: 1499) | 2.9 |
| RAG3A-542 | CTTAACCCAGTGCACGTGT (SEQ ID NO: 616) | CUUAACCCAGUGCACGUGUTT (SEQ ID NO: 1058) | ACACGUGCACUGGGUUAAGT T (SEQ ID NO: 1500) | 2.8 |
| RAG3A-748 | AGCAGGGAGGTATTTGCGG (SEQ ID NO: 617) | AGCAGGGAGGUAUUUGCGGTT (SEQ ID NO: 1059) | CCGCAAAUACCUCCCUGCUTT (SEQ ID NO: 1501) | 2.8 |
| RAG3A-540 | TAACCCAGTGCACGTGTGC (SEQ ID NO: 618) | UAACCCAGUGCACGUGUGCTT (SEQ ID NO: 1060) | GCACACGUGCACUGGGUUAT T (SEQ ID NO: 1502) | 2.8 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-1219 | TCTGTGGAGTGACAGTATC (SEQ ID NO: 619) | UCUGUGGAGUGACAGUAUCTT (SEQ ID NO: 1061) | GAUACUGUCACUCCACAGAT T (SEQ ID NO: 1503) | 2.8 |
| RAG3A-487 | CAACCCGTCCAAACCCTTT (SEQ ID NO: 620) | CAACCCGUCCAAACCCUUUTT (SEQ ID NO: 1062) | AAAGGGUUUGGACGGGUUGT T (SEQ ID NO: 1504) | 2.7 |
| RAG3A-559 | GAGCACCTAAGCTCAGGCT (SEQ ID NO: 621) | GAGCACCUAAGCUCAGGCUTT (SEQ ID NO: 1063) | AGCCUGAGCUUAGGUGCUCT T (SEQ ID NO: 1505) | 2.7 |
| RAG3A-1308 | CCTGCCTCCCTACCTTCTC (SEQ ID NO: 622) | CCUGCCUCCCUACCUUCUCTT (SEQ ID NO: 1064) | GAGAAGGUAGGGAGGCAGGT T (SEQ ID NO: 1506) | 2.7 |
| RAG3A-24 | TGGTGGCTCCCTTCTCTGA (SEQ ID NO: 623) | UGGUGGCUCCCUUCUCUGATT (SEQ ID NO: 1065) | UCAGAGAAGGGAGCCACCAT T (SEQ ID NO: 1507) | 2.6 |
| RAG3A-1169 | TTCGACAGTCCCCTCTTGC (SEQ ID NO: 624) | UUCGACAGUCCCCUCUUGCTT (SEQ ID NO: 1066) | GCAAGAGGGGACUGUCGAAT T (SEQ ID NO: 1508) | 2.6 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-1256 | GACAGGGACCTAGCCAGA A (SEQ ID NO: 625) | GACAGGGACCUAGCCAGAATT (SEQ ID NO: 1067) | UUCUGGCUAGGUCCCUGUCT T (SEQ ID NO: 1509) | 2.6 |
| RAG3A-522 | CGCACATACATGTGCCCCG (SEQ ID NO: 626) | CGCACAUACAUGUGCCCCGTT (SEQ ID NO: 1068) | CGGGGCACAUGUAUGUGCGT T (SEQ ID NO: 1510) | 2.6 |
| RAG3A-851 | TCAAGGTCAGGCCAGGAA G (SEQ ID NO: 627) | UCAAGGUCAGGCCAGGAAGTT (SEQ ID NO: 1069) | CUUCCUGGCCUGACCUUGATT (SEQ ID NO: 1511) | 2.6 |
| RAG3A-1096 | AGCCTCCTTTCTCTTGGTT (SEQ ID NO: 628) | AGCCUCCUUUCUCUUGGUUTT (SEQ ID NO: 1070) | AACCAAGAGAAAGGAGGCUT T (SEQ ID NO: 1512) | 2.5 |
| RAG3A-227 | CACAAATGTCCTGCCAGAT (SEQ ID NO: 629) | CACAAAUGUCCUGCCAGAUTT (SEQ ID NO: 1071) | AUCUGGCAGGACAUUUGUGT T (SEQ ID NO: 1513) | 2.5 |
| RAG3A-1178 | TCAGGCGCATTCGACAGTC (SEQ ID NO: 630) | UCAGGCGCAUUCGACAGUCTT (SEQ ID NO: 1072) | GACUGUCGAAUGCGCCUGAT T (SEQ ID NO: 1514) | 2.5 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-544 | GGCTTAACCCAGTGCACGT (SEQ ID NO: 631) | GGCUUAACCCAGUGCACGUTT (SEQ ID NO: 1073) | ACGUGCACUGGGUUAAGCCT T (SEQ ID NO: 1515) | 2.5 |
| RAG3A-836 | GAAGCCCCTGAGGACAGA G (SEQ ID NO: 632) | GAAGCCCCUGAGGACAGAGTT (SEQ ID NO: 1074) | CUCUGUCCUCAGGGGCUUCT T (SEQ ID NO: 1516) | 2.4 |
| RAG3A-569 | CCCAGCAGACGAGCACCTA (SEQ ID NO: 633) | CCCAGCAGACGAGCACCUATT (SEQ ID NO: 1075) | UAGGUGCUCGUCUGCUGGGT T (SEQ ID NO: 1517) | 2.4 |
| RAG3A-1320 | CTCACTGCCTAGCCTGCCT (SEQ ID NO: 634) | CUCACUGCCUAGCCUGCCUTT (SEQ ID NO: 1076) | AGGCAGGCUAGGCAGUGAGT T (SEQ ID NO: 1518) | 2.4 |
| RAG3A-1319 | TCACTGCCTAGCCTGCCTC (SEQ ID NO: 635) | UCACUGCCUAGCCUGCCUCTT (SEQ ID NO: 1077) | GAGGCAGGCUAGGCAGUGAT T (SEQ ID NO: 1519) | 2.3 |
| RAG3A-290 | GATAGCCAGGGAGTGAAA A (SEQ ID NO: 636) | GAUAGCCAGGGAGUGAAAATT (SEQ ID NO: 1078) | UUUUCACUCCCUGGCUAUCT T (SEQ ID NO: 1520) | 2.3 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-1074 | TGGTCATGCCTGCCTCCCT (SEQ ID NO: 637) | UGGUCAUGCCUGCCUCCCUTT (SEQ ID NO: 1079) | AGGGAGGCAGGCAUGACCAT T (SEQ ID NO: 1521) | 2.2 |
| RAG3A-279 | AGTGAAAACCCCACCAATC (SEQ ID NO: 638) | AGUGAAAACCCCACCAAUCTT (SEQ ID NO: 1080) | GAUUGGUGGGGUUUUCACUT T (SEQ ID NO: 1522) | 2.2 |
| RAG3A-521 | GCACATACATGTGCCCCGC (SEQ ID NO: 639) | GCACAUACAUGUGCCCCGCTT (SEQ ID NO: 1081) | GCGGGGCACAUGUAUGUGCT T (SEQ ID NO: 1523) | 2.2 |
| RAG3A-25 | CTGGTGGCTCCCTTCTCTG (SEQ ID NO: 640) | CUGGUGGCUCCCUUCUCUGTT (SEQ ID NO: 1082) | CAGAGAAGGGAGCCACCAGT T (SEQ ID NO: 1524) | 2.1 |
| RAG3A-62 | AAGAGAAGGCGTCACTTCC (SEQ ID NO: 641) | AAGAGAAGGCGUCACUUCCTT (SEQ ID NO: 1083) | GGAAGUGACGCCUUCUCUUT T (SEQ ID NO: 1525) | 2.1 |
| RAG3A-852 | ATCAAGGTCAGGCCAGGA A (SEQ ID NO: 642) | AUCAAGGUCAGGCCAGGAATT (SEQ ID NO: 1084) | UUCCUGGCCUGACCUUGAUT T (SEQ ID NO: 1526) | 2.1 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-931 | CTCAGCAGACATGCCCATC (SEQ ID NO: 643) | CUCAGCAGACAUGCCCAUCTT (SEQ ID NO: 1085) | GAUGGGCAUGUCUGCUGAGT T (SEQ ID NO: 1527) | 2.1 |
| RAG3A-892 | GAGAGCCTGAGGAAGTTCT (SEQ ID NO: 644) | GAGAGCCUGAGGAAGUUCUTT (SEQ ID NO: 1086) | AGAACUUCCUCAGGCUCUCT T (SEQ ID NO: 1528) | 2 |
| RAG3A-235 | CCCAGCAACACAAATGTCC (SEQ ID NO: 645) | CCCAGCAACACAAAUGUCCTT (SEQ ID NO: 1087) | GGACAUUUGUGUUGCUGGGT T (SEQ ID NO: 1529) | 2 |
| RAG3A-1246 | TAGCCAGAAACCGGCAGC A (SEQ ID NO: 646) | UAGCCAGAAACCGGCAGCATT (SEQ ID NO: 1088) | UGCUGCCGGUUUCUGGCUAT T (SEQ ID NO: 1530) | 2 |
| RAG3A-19 | GCTCCCTTCTCTGATTGGG (SEQ ID NO: 647) | GCUCCCUUCUCUGAUUGGGTT (SEQ ID NO: 1089) | CCCAAUCAGAGAAGGGAGCT T (SEQ ID NO: 1531) | 2 |
| RAG3A-1322 | CTCTCACTGCCTAGCCTGC (SEQ ID NO: 648) | CUCUCACUGCCUAGCCUGCTT (SEQ ID NO: 1090) | GCAGGCUAGGCAGUGAGAGT T (SEQ ID NO: 1532) | 2 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-795 | GAAGAAAGCAAAGGAGCTA (SEQ ID NO: 649) | GAAGAAAGCAAAGGAGCUATT (SEQ ID NO: 1091) | UAGCUCCUUUGCUUUCUUCTT (SEQ ID NO: 1533) | 2 |
| RAG3A-515 | ACATGTGCCCCGCACCTGA (SEQ ID NO: 650) | ACAUGUGCCCCGCACCUGATT (SEQ ID NO: 1092) | UCAGGUGCGGGGCACAUGUTT (SEQ ID NO: 1534) | 2 |
| RAG3A-128 | TCCAAATCTTCTCCGTGGT (SEQ ID NO: 651) | UCCAAAUCUUCUCCGUGGUTT (SEQ ID NO: 1093) | ACCACGGAGAAGAUUUGGATT (SEQ ID NO: 1535) | 2 |
| RAG3A-661 | GGAACGGTAGGAGATGGAA (SEQ ID NO: 652) | GGAACGGUAGGAGAUGGAATT (SEQ ID NO: 1094) | UUCCAUCUCCUACCGUUCCTT (SEQ ID NO: 1536) | 2 |
| RAG3A-125 | AAATCTTCTCCGTGGTGTG (SEQ ID NO: 653) | AAAUCUUCUCCGUGGUGUGTT (SEQ ID NO: 1095) | CACACCACGGAGAAGAUUUTT (SEQ ID NO: 1537) | 2 |
| RAG3A-1241 | AGAAACCGGCAGCATTCCC (SEQ ID NO: 654) | AGAAACCGGCAGCAUUCCCTT (SEQ ID NO: 1096) | GGGAAUGCUGCCGGUUUCUTT (SEQ ID NO: 1538) | 2 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-463 | TAACACCAACCCATAACAG (SEQ ID NO: 655) | UAACACCAACCCAUAACAGTT (SEQ ID NO: 1097) | CUGUUAUGGGUUGGUGUUAT T (SEQ ID NO: 1539) | 2 |
| RAG3A-434 | TCATGTGGGCAATATCCGT (SEQ ID NO: 656) | UCAUGUGGGCAAUAUCCGUTT (SEQ ID NO: 1098) | ACGGAUAUUGCCCACAUGAT T (SEQ ID NO: 1540) | 1.9 |
| RAG3A-520 | CACATACATGTGCCCCGCA (SEQ ID NO: 657) | CACAUACAUGUGCCCCGCATT (SEQ ID NO: 1099) | UGCGGGGCACAUGUAUGUGT T (SEQ ID NO: 1541) | 1.9 |
| RAG3A-499 | TGACAGTCCACTCAACCCG (SEQ ID NO: 658) | UGACAGUCCACUCAACCCGTT (SEQ ID NO: 1100) | CGGGUUGAGUGGACUGUCAT T (SEQ ID NO: 1542) | 1.9 |
| RAG3A-668 | CACATGTGGAACGGTAGGA (SEQ ID NO: 659) | CACAUGUGGAACGGUAGGATT (SEQ ID NO: 1101) | UCCUACCGUUCCACAUGUGT T (SEQ ID NO: 1543) | 1.9 |
| RAG3A-591 | GTAGCCATCTAAGCCACTG (SEQ ID NO: 660) | GUAGCCAUCUAAGCCACUGTT (SEQ ID NO: 1102) | CAGUGGCUUAGAUGGCUACT T (SEQ ID NO: 1544) | 1.9 |
| RAG3A-1209 | GACAGTATCTCCCTCTCAT (SEQ ID NO: 661) | GACAGUAUCUCCCUCUCAUTT (SEQ ID NO: 1103) | AUGAGAGGGAGAUACUGUCT T (SEQ ID NO: 1545) | 1.9 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-1257 | GGACAGGGACCTAGCCAGA (SEQ ID NO: 662) | GGACAGGGACCUAGCCAGATT (SEQ ID NO: 1104) | UCUGGCUAGGUCCCUGUCCTT (SEQ ID NO: 1546) | 1.9 |
| RAG3A-1170 | ATTCGACAGTCCCCTCTTG (SEQ ID NO: 663) | AUUCGACAGUCCCCUCUUGTT (SEQ ID NO: 1105) | CAAGAGGGGACUGUCGAAUTT (SEQ ID NO: 1547) | 1.9 |
| RAG3A-39 | GCCTTCACCAGTGTCTGGT (SEQ ID NO: 664) | GCCUUCACCAGUGUCUGGUTT (SEQ ID NO: 1106) | ACCAGACACUGGUGAAGGCTT (SEQ ID NO: 1548) | 1.9 |
| RAG3A-431 | TGTGGGCAATATCCGTGTT (SEQ ID NO: 665) | UGUGGGCAAUAUCCGUGUUTT+B50 (SEQ ID NO: 1107) | AACACGGAUAUUGCCCACATT (SEQ ID NO: 1549) | 1.9 |
| RAG3A-1080 | GTTCCCTGGTCATGCCTGC (SEQ ID NO: 666) | GUUCCCUGGUCAUGCCUGCTT (SEQ ID NO: 1108) | GCAGGCAUGACCAGGGAACTT (SEQ ID NO: 1550) | 1.9 |
| RAG3A-1266 | TGTTACCCAGGACAGGGAC (SEQ ID NO: 667) | UGUUACCCAGGACAGGGACTT (SEQ ID NO: 1109) | GUCCCUGUCCUGGGUAACATT (SEQ ID NO: 1551) | 1.9 |
| RAG3A-1202 | TCTCCCTCTCATTGTAACT (SEQ ID NO: 668) | UCUCCCUCUCAUUGUAACUTT (SEQ ID NO: 1110) | AGUUACAAUGAGAGGGAGATT (SEQ ID NO: 1552) | 1.9 |
| RAG3A-979 | ACATCTCGTCTGGCTGGAC (SEQ ID NO: 669) | ACAUCUCGUCUGGCUGGACTT (SEQ ID NO: 1111) | GUCCAGCCAGACGAGAUGUTT (SEQ ID NO: 1553) | 1.9 |
| RAG3A-343 | AGCATCCTGTTGGATTTCA (SEQ ID NO: 670) | AGCAUCCUGUUGGAUUUCATT (SEQ ID NO: 1112) | UGAAAUCCAACAGGAUGCUTT (SEQ ID NO: 1554) | 1.9 |
| RAG3A-430 | GTGGGCAATATCCGTGTTC (SEQ ID NO: 671) | GUGGGCAAUAUCCGUGUUCTT (SEQ ID NO: 1113) | GAACACGGAUAUUGCCCACTT (SEQ ID NO: 1555) | 1.9 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-319 | AGGTATGATGTCCAGGGAA (SEQ ID NO: 672) | AGGUAUGAUGUCCAGGGAATT (SEQ ID NO: 1114) | UUCCCUGGACAUCAUACCUT T (SEQ ID NO: 1556) | 1.8 |
| RAG3A-612 | TGGGCACAGAGGTCTGTGT (SEQ ID NO: 673) | UGGGCACAGAGGUCUGUGUTT (SEQ ID NO: 1115) | ACACAGACCUCUGUGCCCATT (SEQ ID NO: 1557) | 1.8 |
| RAG3A-454 | CCCATAACAGGAGATTTCT (SEQ ID NO: 674) | CCCAUAACAGGAGAUUUCUTT (SEQ ID NO: 1116) | AGAAAUCUCCUGUUAUGGGT T (SEQ ID NO: 1558) | 1.8 |
| RAG3A-1312 | CTAGCCTGCCTCCCTACCT (SEQ ID NO: 675) | CUAGCCUGCCUCCCUACCUTT (SEQ ID NO: 1117) | AGGUAGGGAGGCAGGCUAGT T (SEQ ID NO: 1559) | 1.8 |
| RAG3A-342 | GCATCCTGTTGGATTTCAG (SEQ ID NO: 676) | GCAUCCUGUUGGAUUUCAGTT (SEQ ID NO: 1118) | CUGAAAUCCAACAGGAUGCT T (SEQ ID NO: 1560) | 1.8 |
| RAG3A-1118 | TCTGCCTCTCCAGCCCACT (SEQ ID NO: 677) | UCUGCCUCUCCAGCCCACUTT (SEQ ID NO: 1119) | AGUGGGCUGGAGAGGCAGAT T (SEQ ID NO: 1561) | 1.8 |
| RAG3A-970 | CTGGCTGGACGGGAAAATT (SEQ ID NO: 678) | CUGGCUGGACGGGAAAAUUTT (SEQ ID NO: 1120) | AAUUUUCCCGUCCAGCCAGT T (SEQ ID NO: 1562) | 1.8 |
| RAG3A-1133 | TCACCCAAGGGACCCTCTG (SEQ ID NO: 679) | UCACCCAAGGGACCCUCUGTT (SEQ ID NO: 1121) | CAGAGGGUCCCUUGGGUGAT T (SEQ ID NO: 1563) | 1.8 |
| RAG3A-689 | GGGCCTGGTTATTCCTCTT (SEQ ID NO: 680) | GGGCCUGGUUAUUCCUCUUTT (SEQ ID NO: 1122) | AAGAGGAAUAACCAGGCCCT T (SEQ ID NO: 1564) | 1.8 |
| RAG3A-1321 | TCTCACTGCCTAGCCTGCC (SEQ ID NO: 681) | UCUCACUGCCUAGCCUGCCTT (SEQ ID NO: 1123) | GGCAGGCUAGGCAGUGAGAT T (SEQ ID NO: 1565) | 1.8 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-662 | TGGAACGGTAGGAGATGGA (SEQ ID NO: 682) | UGGAACGGUAGGAGAUGGATT (SEQ ID NO: 1124) | UCCAUCUCCUACCGUUCCATT (SEQ ID NO: 1566) | 1.8 |
| RAG3A-817 | ACTGTGGGGAGACTGGGAC (SEQ ID NO: 683) | ACUGUGGGGAGACUGGGACTT (SEQ ID NO: 1125) | GUCCCAGUCUCCCCACAGUTT (SEQ ID NO: 1567) | 1.8 |
| RAG3A-1242 | CAGAAACCGGCAGCATTCC (SEQ ID NO: 684) | CAGAAACCGGCAGCAUUCCTT (SEQ ID NO: 1126) | GGAAUGCUGCCGGUUUCUGTT (SEQ ID NO: 1568) | 1.8 |
| RAG3A-278 | GTGAAAACCCCACCAATCT (SEQ ID NO: 685) | GUGAAAACCCCACCAAUCUTT (SEQ ID NO: 1127) | AGAUUGGUGGGGUUUUCACTT (SEQ ID NO: 1569) | 1.8 |
| RAG3A-1210 | TGACAGTATCTCCCTCTCA (SEQ ID NO: 686) | UGACAGUAUCUCCCUCUCATT (SEQ ID NO: 1128) | UGAGAGGGAGAUACUGUCATT (SEQ ID NO: 1570) | 1.8 |
| RAG3A-1084 | CTTGGTTCCCTGGTCATGC (SEQ ID NO: 687) | CUUGGUUCCCUGGUCAUGCTT (SEQ ID NO: 1129) | GCAUGACCAGGGAACCAAGTT (SEQ ID NO: 1571) | 1.8 |
| RAG3A-1166 | GACAGTCCCCTCTTGCTTT (SEQ ID NO: 688) | GACAGUCCCCUCUUGCUUUTT (SEQ ID NO: 1130) | AAAGCAAGAGGGGACUGUCTT (SEQ ID NO: 1572) | 1.8 |
| RAG3A-982 | GAGACATCTCGTCTGGCTG (SEQ ID NO: 689) | GAGACAUCUCGUCUGGCUGTT (SEQ ID NO: 1131) | CAGCCAGACGAGAUGUCUCTT (SEQ ID NO: 1573) | 1.8 |
| RAG3A-171 | AGGTCCCAGGTTGCCCATG (SEQ ID NO: 690) | AGGUCCCAGGUUGCCCAUGTT (SEQ ID NO: 1132) | CAUGGGCAACCUGGGACCUTT (SEQ ID NO: 1574) | 1.8 |
| RAG3A-256 | ACAAGACCTCTGTGCTTCT (SEQ ID NO: 691) | ACAAGACCUCUGUGCUUCUTT (SEQ ID NO: 1133) | AGAAGCACAGAGGUCUUGUTT (SEQ ID NO: 1575) | 1.7 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-563 | AGACGAGCACCTAAGCTCA (SEQ ID NO: 692) | AGACGAGCACCUAAGCUCATT (SEQ ID NO: 1134) | UGAGCUUAGGUGCUCGUCUT T (SEQ ID NO: 1576) | 1.7 |
| RAG3A-1176 | AGGCGCATTCGACAGTCCC (SEQ ID NO: 693) | AGGCGCAUUCGACAGUCCCTT (SEQ ID NO: 1135) | GGGACUGUCGAAUGCGCCUT T (SEQ ID NO: 1577) | 1.7 |
| RAG3A-707 | CCTAAGACAGGGACACATG (SEQ ID NO: 694) | CCUAAGACAGGGACACAUGTT (SEQ ID NO: 1136) | CAUGUGUCCCUGUCUUAGGT T (SEQ ID NO: 1578) | 1.7 |
| RAG3A-561 | ACGAGCACCTAAGCTCAGG (SEQ ID NO: 695) | ACGAGCACCUAAGCUCAGGTT (SEQ ID NO: 1137) | CCUGAGCUUAGGUGCUCGUT T (SEQ ID NO: 1579) | 1.7 |
| RAG3A-504 | GCACCTGACAGTCCACTCA (SEQ ID NO: 696) | GCACCUGACAGUCCACUCATT (SEQ ID NO: 1138) | UGAGUGGACUGUCAGGUGCT T (SEQ ID NO: 1580) | 1.7 |
| RAG3A-195 | AAACTTCTGCTCCTGTCCC (SEQ ID NO: 697) | AAACUUCUGCUCCUGUCCCTT (SEQ ID NO: 1139) | GGGACAGGAGCAGAAGUUUT T (SEQ ID NO: 1581) | 1.7 |
| RAG3A-850 | CAAGGTCAGGCCAGGAAG C (SEQ ID NO: 698) | CAAGGUCAGGCCAGGAAGCTT (SEQ ID NO: 1140) | GCUUCCUGGCCUGACCUUGT T (SEQ ID NO: 1582) | 1.7 |
| RAG3A-388 | AGATAGGACTCCCTAGGGG (SEQ ID NO: 699) | AGAUAGGACUCCCUAGGGGTT (SEQ ID NO: 1141) | CCCCUAGGGAGUCCUAUCUT T (SEQ ID NO: 1583) | 1.7 |
| RAG3A-1079 | TTCCCTGGTCATGCCTGCC (SEQ ID NO: 700) | UUCCCUGGUCAUGCCUGCCTT (SEQ ID NO: 1142) | GGCAGGCAUGACCAGGGAAT T (SEQ ID NO: 1584) | 1.7 |
| RAG3A-1263 | TACCCAGGACAGGGACCTA (SEQ ID NO: 701) | UACCCAGGACAGGGACCUATT (SEQ ID NO: 1143) | UAGGUCCCUGUCCUGGGUAT T (SEQ ID NO: 1585) | 1.7 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-531 | GCACGTGTGCGCACATACA (SEQ ID NO: 702) | GCACGUGUGCGCACAUACATT (SEQ ID NO: 1144) | UGUAUGUGCGCACACGUGCTT (SEQ ID NO: 1586) | 1.7 |
| RAG3A-254 | AAGACCTCTGTGCTTCTTC (SEQ ID NO: 703) | AAGACCUCUGUGCUUCUUCTT (SEQ ID NO: 1145) | GAAGAAGCACAGAGGUCUUTT (SEQ ID NO: 1587) | 1.7 |
| RAG3A-421 | ATCCGTGTTCCCACTTCGA (SEQ ID NO: 704) | AUCCGUGUUCCCACUUCGATT (SEQ ID NO: 1146) | UCGAAGUGGGAACACGGAUTT (SEQ ID NO: 1588) | 1.7 |
| RAG3A-473 | CCTTTCCCCATAACACCAA (SEQ ID NO: 705) | CCUUUCCCCAUAACACCAATT (SEQ ID NO: 1147) | UUGGUGUUAUGGGGAAAGGTT (SEQ ID NO: 1589) | 1.7 |
| RAG3A-292 | TGGATAGCCAGGGAGTGAA (SEQ ID NO: 706) | UGGAUAGCCAGGGAGUGAATT (SEQ ID NO: 1148) | UUCACUCCCUGGCUAUCCATT (SEQ ID NO: 1590) | 1.7 |
| RAG3A-283 | AGGGAGTGAAAACCCCACC (SEQ ID NO: 707) | AGGGAGUGAAAACCCCACCTT (SEQ ID NO: 1149) | GGUGGGGUUUUCACUCCCUTT (SEQ ID NO: 1591) | 1.7 |
| RAG3A-786 | AAAGGAGCTAGAGCCAGGG (SEQ ID NO: 708) | AAAGGAGCUAGAGCCAGGGTT (SEQ ID NO: 1150) | CCCUGGCUCUAGCUCCUUUTT (SEQ ID NO: 1592) | 1.7 |
| RAG3A-582 | TAAGCCACTGGACCCCAGC (SEQ ID NO: 709) | UAAGCCACUGGACCCCAGCTT (SEQ ID NO: 1151) | GCUGGGGUCCAGUGGCUUATT (SEQ ID NO: 1593) | 1.7 |
| RAG3A-933 | TTCTCAGCAGACATGCCCA (SEQ ID NO: 710) | UUCUCAGCAGACAUGCCCATT (SEQ ID NO: 1152) | UGGGCAUGUCUGCUGAGAATT (SEQ ID NO: 1594) | 1.7 |
| RAG3A-472 | CTTTCCCCATAACACCAAC (SEQ ID NO: 711) | CUUUCCCCAUAACACCAACTT (SEQ ID NO: 1153) | GUUGGUGUUAUGGGGAAAGTT (SEQ ID NO: 1595) | 1.7 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-646 | GGAAGACGGAGACAGAACA (SEQ ID NO: 712) | GGAAGACGGAGACAGAACATT (SEQ ID NO: 1154) | UGUUCUGUCUCCGUCUUCCT T (SEQ ID NO: 1596) | 1.6 |
| RAG3A-389 | AAGATAGGACTCCCTAGGG (SEQ ID NO: 713) | AAGAUAGGACUCCCUAGGGTT (SEQ ID NO: 1155) | CCCUAGGGAGUCCUAUCUUT T (SEQ ID NO: 1597) | 1.6 |
| RAG3A-971 | TCTGGCTGGACGGGAAAAT (SEQ ID NO: 714) | UCUGGCUGGACGGGAAAAUTT (SEQ ID NO: 1156) | AUUUUCCCGUCCAGCCAGATT (SEQ ID NO: 1598) | 1.6 |
| RAG3A-751 | GCCAGCAGGGAGGTATTTG (SEQ ID NO: 715) | GCCAGCAGGGAGGUAUUUGTT (SEQ ID NO: 1157) | CAAAUACCUCCCUGCUGGCT T (SEQ ID NO: 1599) | 1.6 |
| RAG3A-299 | AGAAATTTGGATAGCCAGG (SEQ ID NO: 716) | AGAAAUUUGGAUAGCCAGGTT (SEQ ID NO: 1158) | CCUGGCUAUCCAAAUUUCUT T (SEQ ID NO: 1600) | 1.6 |
| RAG3A-750 | CCAGCAGGGAGGTATTTGC (SEQ ID NO: 717) | CCAGCAGGGAGGUAUUUGCTT (SEQ ID NO: 1159) | GCAAAUACCUCCCUGCUGGT T (SEQ ID NO: 1601) | 1.6 |
| RAG3A-671 | TGTCACATGTGGAACGGTA (SEQ ID NO: 718) | UGUCACAUGUGGAACGGUATT (SEQ ID NO: 1160) | UACCGUUCCACAUGUGACAT T (SEQ ID NO: 1602) | 1.6 |
| RAG3A-749 | CAGCAGGGAGGTATTTGCG (SEQ ID NO: 719) | CAGCAGGGAGGUAUUUGCGTT (SEQ ID NO: 1161) | CGCAAAUACCUCCCUGCUGT T (SEQ ID NO: 1603) | 1.6 |
| RAG3A-547 | TCAGGCTTAACCCAGTGCA (SEQ ID NO: 720) | UCAGGCUUAACCCAGUGCATT (SEQ ID NO: 1162) | UGCACUGGGUUAAGCCUGAT T (SEQ ID NO: 1604) | 1.6 |
| RAG3A-655 | GTAGGAGATGGAAGACGGA (SEQ ID NO: 721) | GUAGGAGAUGGAAGACGGATT (SEQ ID NO: 1163) | UCCGUCUUCCAUCUCCUACTT (SEQ ID NO: 1605) | 1.6 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-170 | GGTCCCAGGTTGCCCATGT (SEQ ID NO: 722) | GGUCCCAGGUUGCCCAUGUTT (SEQ ID NO: 1164) | ACAUGGGCAACCUGGGACCT T (SEQ ID NO: 1606) | 1.6 |
| RAG3A-629 | CAAGCAAAGGAGGGCCCT G (SEQ ID NO: 723) | CAAGCAAAGGAGGGCCCUGTT (SEQ ID NO: 1165) | CAGGGCCCUCCUUUGCUUGT T (SEQ ID NO: 1607) | 1.6 |
| RAG3A-253 | AGACCTCTGTGCTTCTTCC (SEQ ID NO: 724) | AGACCUCUGUGCUUCUUCCTT (SEQ ID NO: 1166) | GGAAGAAGCACAGAGGUCUT T (SEQ ID NO: 1608) | 1.6 |
| RAG3A-695 | ACACATGGGCCTGGTTATT (SEQ ID NO: 725) | ACACAUGGGCCUGGUUAUUTT (SEQ ID NO: 1167) | AAUAACCAGGCCCAUGUGUT T (SEQ ID NO: 1609) | 1.6 |
| RAG3A-788 | GCAAAGGAGCTAGAGCCA G (SEQ ID NO: 726) | GCAAAGGAGCUAGAGCCAGTT (SEQ ID NO: 1168) | CUGGCUCUAGCUCCUUUGCT T (SEQ ID NO: 1610) | 1.6 |
| RAG3A-688 | GGCCTGGTTATTCCTCTTG (SEQ ID NO: 727) | GGCCUGGUUAUUCCUCUUGTT (SEQ ID NO: 1169) | CAAGAGGAAUAACCAGGCCT T (SEQ ID NO: 1611) | 1.6 |
| RAG3A-728 | GGAGGTCCAGCAGCTGTCT (SEQ ID NO: 728) | GGAGGUCCAGCAGCUGUCUTT (SEQ ID NO: 1170) | AGACAGCUGCUGGACCUCCT T (SEQ ID NO: 1612) | 1.6 |
| RAG3A-519 | ACATACATGTGCCCCGCAC (SEQ ID NO: 729) | ACAUACAUGUGCCCCGCACTT (SEQ ID NO: 117 1) | GUGCGGGGCACAUGUAUGUT T (SEQ ID NO: 1613) | 1.6 |
| RAG3A-272 | ACCCCACCAATCTTAAACA (SEQ ID NO: 730) | ACCCCACCAAUCUUAAACATT (SEQ ID NO: 1172) | UGUUUAAGAUUGGUGGGGUT T (SEQ ID NO: 1614) | 1.6 |
| RAG3A-318 | GGTATGATGTCCAGGGAAA (SEQ ID NO: 731) | GGUAUGAUGUCCAGGGAAATT (SEQ ID NO: 1173) | UUUCCCUGGACAUCAUACCT T (SEQ ID NO: 1615) | 1.6 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-1338 | AAACCCTCACCACCCTCTC (SEQ ID NO: 732) | AAACCCUCACCACCCUCUCTT (SEQ ID NO: 1174) | GAGAGGGUGGUGAGGGUUUT T (SEQ ID NO: 1616) | 1.6 |
| RAG3A-539 | AACCCAGTGCACGTGTGCG (SEQ ID NO: 733) | AACCCAGUGCACGUGUGCGTT (SEQ ID NO: 1175) | CGCACACGUGCACUGGGUUT T (SEQ ID NO: 1617) | 1.6 |
| RAG3A-826 | AGGACAGAGACTGTGGGGA (SEQ ID NO: 734) | AGGACAGAGACUGUGGGGATT (SEQ ID NO: 1176) | UCCCCACAGUCUCUGUCCUTT (SEQ ID NO: 1618) | 1.6 |
| RAG3A-337 | CTGTTGGATTTCAGCAGCA (SEQ ID NO: 735) | CUGUUGGAUUUCAGCAGCATT (SEQ ID NO: 1177) | UGCUGCUGAAAUCCAACAGT T (SEQ ID NO: 1619) | 1.6 |
| RAG3A-273 | AACCCCACCAATCTTAAAC (SEQ ID NO: 736) | AACCCCACCAAUCUUAAACTT (SEQ ID NO: 1178) | GUUUAAGAUUGGUGGGGUUT T (SEQ ID NO: 1620) | 1.6 |
| RAG3A-478 | CAAACCCTTTCCCCATAAC (SEQ ID NO: 737) | CAAACCCUUUCCCCAUAACTT (SEQ ID NO: 1179) | GUUAUGGGGAAAGGGUUUGT T (SEQ ID NO: 1621) | 1.6 |
| RAG3A-963 | GACGGGAAAATTCCAGGAT (SEQ ID NO: 738) | GACGGGAAAAUUCCAGGAUTT (SEQ ID NO: 1180) | AUCCUGGAAUUUUCCCGUCT T (SEQ ID NO: 1622) | 1.6 |
| RAG3A-248 | TCTGTGCTTCTTCCCCAGC (SEQ ID NO: 739) | UCUGUGCUUCUUCCCCAGCTT (SEQ ID NO: 1181) | GCUGGGGAAGAAGCACAGAT T (SEQ ID NO: 1623) | 1.6 |
| RAG3A-35 | TCACCAGTGTCTGGTGGCT (SEQ ID NO: 740) | UCACCAGUGUCUGGUGGCUTT (SEQ ID NO: 1182) | AGCCACCAGACACUGGUGAT T (SEQ ID NO: 1624) | 1.5 |
| RAG3A-613 | CTGGGCACAGAGGTCTGTG (SEQ ID NO: 741) | CUGGGCACAGAGGUCUGUGTT (SEQ ID NO: 1183) | CACAGACCUCUGUGCCCAGT T (SEQ ID NO: 1625) | 1.5 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-1141 | CCCTTCCTTCACCCAAGGG (SEQ ID NO: 742) | CCCUUCCUUCACCCAAGGGTT (SEQ ID NO: 1184) | CCCUUGGGUGAAGGAAGGGT T (SEQ ID NO: 1626) | 1.5 |
| RAG3A-477 | AAACCCTTTCCCCATAACA (SEQ ID NO: 743) | AAACCCUUUCCCCAUAACATT (SEQ ID NO: 1185) | UGUUAUGGGGAAAGGGUUUT T (SEQ ID NO: 1627) | 1.5 |
| RAG3A-634 | CAGAACAAGCAAAGGAGG G (SEQ ID NO: 744) | CAGAACAAGCAAAGGAGGGTT (SEQ ID NO: 1186) | CCCUCCUUUGCUUGUUCUGT T (SEQ ID NO: 1628) | 1.5 |
| RAG3A-1280 | GCTCCTCATTCTTGTGTTA (SEQ ID NO: 745) | GCUCCUCAUUCUUGUGUUATT (SEQ ID NO: 1187) | UAACACAAGAAUGAGGAGCT T (SEQ ID NO: 1629) | 1.5 |
| RAG3A-1062 | CCTCCCTGTCTCCTGTCTC (SEQ ID NO: 746) | CCUCCCUGUCUCCUGUCUCTT (SEQ ID NO: 1188) | GAGACAGGAGACAGGGAGGT T (SEQ ID NO: 1630) | 1.5 |
| RAG3A-778 | TAGAGCCAGGGCCAAAGG A (SEQ ID NO: 747) | UAGAGCCAGGGCCAAAGGATT (SEQ ID NO: 1189) | UCCUUUGGCCCUGGCUCUATT (SEQ ID NO: 1631) | 1.5 |
| RAG3A-939 | CCACACTTCTCAGCAGACA (SEQ ID NO: 748) | CCACACUUCUCAGCAGACATT (SEQ ID NO: 1190) | UGUCUGCUGAGAAGUGUGGT T (SEQ ID NO: 1632) | 1.5 |
| RAG3A-779 | CTAGAGCCAGGGCCAAAG G (SEQ ID NO: 749) | CUAGAGCCAGGGCCAAAGGTT (SEQ ID NO: 1191) | CCUUUGGCCCUGGCUCUAGT T (SEQ ID NO: 1633) | 1.5 |
| RAG3A-803 | GGGACTGGGAAGAAAGCA A (SEQ ID NO: 750) | GGGACUGGGAAGAAAGCAATT (SEQ ID NO: 1192) | UUGCUUUCUUCCCAGUCCCT T (SEQ ID NO: 1634) | 1.5 |
| RAG3A-1382 | GTCCATCAGCCGATCTCCC (SEQ ID NO: 751) | GUCCAUCAGCCGAUCUCCCTT (SEQ ID NO: 1193) | GGGAGAUCGGCUGAUGGACT T (SEQ ID NO: 1635) | 1.5 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-97 | AGGAGTGGACCCTGGTCCA (SEQ ID NO: 752) | AGGAGUGGACCCUGGUCCATT (SEQ ID NO: 1194) | UGGACCAGGGUCCACUCCUT T (SEQ ID NO: 1636) | 1.5 |
| RAG3A-543 | GCTTAACCCAGTGCACGTG (SEQ ID NO: 753) | GCUUAACCCAGUGCACGUGTT (SEQ ID NO: 1195) | CACGUGCACUGGGUUAAGCT T (SEQ ID NO: 1637) | 1.5 |
| RAG3A-556 | CACCTAAGCTCAGGCTTAA (SEQ ID NO: 754) | CACCUAAGCUCAGGCUUAATT (SEQ ID NO: 1196) | UUAAGCCUGAGCUUAGGUGT T (SEQ ID NO: 1638) | 1.5 |
| RAG3A-60 | GAGAAGGCGTCACTTCCGG (SEQ ID NO: 755) | GAGAAGGCGUCACUUCCGGTT (SEQ ID NO: 1197) | CCGGAAGUGACGCCUUCUCT T (SEQ ID NO: 1639) | 1.5 |
| RAG3A-647 | TGGAAGACGGAGACAGAA C (SEQ ID NO: 756) | UGGAAGACGGAGACAGAACTT (SEQ ID NO: 1198) | GUUCUGUCUCCGUCUUCCATT (SEQ ID NO: 1640) | 1.5 |
| RAG3A-243 | GCTTCTTCCCCAGCAACAC (SEQ ID NO: 757) | GCUUCUUCCCCAGCAACACTT (SEQ ID NO: 1199) | GUGUUGCUGGGGAAGAAGCT T (SEQ ID NO: 1641) | 1.5 |
| RAG3A-533 | GTGCACGTGTGCGCACATA (SEQ ID NO: 758) | GUGCACGUGUGCGCACAUATT (SEQ ID NO: 1200) | UAUGUGCGCACACGUGCACT T (SEQ ID NO: 1642) | 1.5 |
| RAG3A-628 | AAGCAAAGGAGGGCCCTG G (SEQ ID NO: 759) | AAGCAAAGGAGGGCCCUGGTT (SEQ ID NO: 1201) | CCAGGGCCCUCCUUUGCUUT T (SEQ ID NO: 1643) | 1.5 |
| RAG3A-849 | AAGGTCAGGCCAGGAAGC C (SEQ ID NO: 760) | AAGGUCAGGCCAGGAAGCCTT (SEQ ID NO: 1202) | GGCUUCCUGGCCUGACCUUT T (SEQ ID NO: 1644) | 1.5 |
| RAG3A-1307 | CTGCCTCCCTACCTTCTCT (SEQ ID NO: 761) | CUGCCUCCCUACCUUCUCUTT (SEQ ID NO: 1203) | AGAGAAGGUAGGGAGGCAGT T (SEQ ID NO: 1645) | 1.5 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-293 | TTGGATAGCCAGGGAGTGA (SEQ ID NO: 762) | UUGGAUAGCCAGGGAGUGATT (SEQ ID NO: 1204) | UCACUCCCUGGCUAUCCAATT (SEQ ID NO: 1646) | 1.5 |
| RAG3A-1285 | AGGTCGCTCCTCATTCTTG (SEQ ID NO: 763) | AGGUCGCUCCUCAUUCUUGTT (SEQ ID NO: 1205) | CAAGAAUGAGGAGCGACCUT T (SEQ ID NO: 1647) | 1.5 |
| RAG3A-1223 | CCCTTCTGTGGAGTGACAG (SEQ ID NO: 764) | CCCUUCUGUGGAGUGACAGTT (SEQ ID NO: 1206) | CUGUCACUCCACAGAAGGGT T (SEQ ID NO: 1648) | 1.5 |
| RAG3A-1177 | CAGGCGCATTCGACAGTCC (SEQ ID NO: 765) | CAGGCGCAUUCGACAGUCCTT (SEQ ID NO: 1207) | GGACUGUCGAAUGCGCCUGT T (SEQ ID NO: 1649) | 1.5 |
| RAG3A-226 | ACAAATGTCCTGCCAGATT (SEQ ID NO: 766) | ACAAAUGUCCUGCCAGAUUTT (SEQ ID NO: 1208) | AAUCUGGCAGGACAUUUGUT T (SEQ ID NO: 1650) | 1.5 |
| RAG3A-1097 | CAGCCTCCTTTCTCTTGGT (SEQ ID NO: 767) | CAGCCUCCUUUCUCUUGGUTT (SEQ ID NO: 1209) | ACCAAGAGAAAGGAGGCUGT T (SEQ ID NO: 1651) | 1.5 |
| RAG3A-1188 | TAACTTATCCTCAGGCGCA (SEQ ID NO: 768) | UAACUUAUCCUCAGGCGCATT (SEQ ID NO: 1210) | UGCGCCUGAGGAUAAGUUAT T (SEQ ID NO: 1652) | 1.5 |
| RAG3A-593 | GTGTAGCCATCTAAGCCAC (SEQ ID NO: 769) | GUGUAGCCAUCUAAGCCACTT (SEQ ID NO: 1211) | GUGGCUUAGAUGGCUACACT T (SEQ ID NO: 1653) | 1.5 |
| RAG3A-670 | GTCACATGTGGAACGGTAG (SEQ ID NO: 770) | GUCACAUGUGGAACGGUAGTT (SEQ ID NO: 1212) | CUACCGUUCCACAUGUGACT T (SEQ ID NO: 1654) | 1.5 |
| RAG3A-503 | CACCTGACAGTCCACTCAA (SEQ ID NO: 771) | CACCUGACAGUCCACUCAATT (SEQ ID NO: 1213) | UUGAGUGGACUGUCAGGUGT T (SEQ ID NO: 1655) | 1.5 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-1167 | CGACAGTCCCCTCTTGCTT (SEQ ID NO: 772) | CGACAGUCCCCUCUUGCUUTT (SEQ ID NO: 1214) | AAGCAAGAGGGGACUGUCGTT (SEQ ID NO: 1656) | 1.5 |
| RAG3A-583 | CTAAGCCACTGGACCCCAG (SEQ ID NO: 773) | CUAAGCCACUGGACCCCAGTT (SEQ ID NO: 1215) | CUGGGGUCCAGUGGCUUAGTT (SEQ ID NO: 1657) | 1.5 |
| RAG3A-706 | CTAAGACAGGGACACATGG (SEQ ID NO: 774) | CUAAGACAGGGACACAUGGTT (SEQ ID NO: 1216) | CCAUGUGUCCCUGUCUUAGTT (SEQ ID NO: 1658) | 1.5 |
| RAG3A-983 | GGAGACATCTCGTCTGGCT (SEQ ID NO: 775) | GGAGACAUCUCGUCUGGCUTT (SEQ ID NO: 1217) | AGCCAGACGAGAUGUCUCCTT (SEQ ID NO: 1659) | 1.5 |
| RAG3A-718 | CAGCTGTCTTTCCTAAGAC (SEQ ID NO: 776) | CAGCUGUCUUUCCUAAGACTT (SEQ ID NO: 1218) | GUCUUAGGAAAGACAGCUGTT (SEQ ID NO: 1660) | 1.5 |
| RAG3A-378 | CCCTAGGGGATTACAGAAA (SEQ ID NO: 777) | CCCUAGGGGAUUACAGAAATT (SEQ ID NO: 1219) | UUUCUGUAAUCCCCUAGGGTT (SEQ ID NO: 1661) | 1.5 |
| RAG3A-1095 | GCCTCCTTTCTCTTGGTTC (SEQ ID NO: 778) | GCCUCCUUUCUCUUGGUUCTT (SEQ ID NO: 1220) | GAACCAAGAGAAAGGAGGCTT (SEQ ID NO: 1662) | 1.4 |
| RAG3A-698 | GGGACACATGGGCCTGGTT (SEQ ID NO: 779) | GGGACACAUGGGCCUGGUUTT (SEQ ID NO: 1221) | AACCAGGCCCAUGUGUCCCTT (SEQ ID NO: 1663) | 1.4 |
| RAG3A-277 | TGAAAACCCCACCAATCTT (SEQ ID NO: 780) | UGAAAACCCCACCAAUCUUTT (SEQ ID NO: 1222) | AAGAUUGGUGGGGUUUUCATT (SEQ ID NO: 1664) | 1.4 |
| RAG3A-644 | AAGACGGAGACAGAACAAG (SEQ ID NO: 781) | AAGACGGAGACAGAACAAGTT (SEQ ID NO: 1223) | CUUGUUCUGUCUCCGUCUUTT (SEQ ID NO: 1665) | 1.4 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-941 | GGCCACACTTCTCAGCAGA (SEQ ID NO: 782) | GGCCACACUUCUCAGCAGATT (SEQ ID NO: 1224) | UCUGCUGAGAAGUGUGGCCT T (SEQ ID NO: 1666) | 1.4 |
| RAG3A-794 | AAGAAAGCAAAGGAGCTA G (SEQ ID NO: 783) | AAGAAAGCAAAGGAGCUAGTT (SEQ ID NO: 1225) | CUAGCUCCUUUGCUUUCUUT T (SEQ ID NO: 1667) | 1.4 |
| RAG3A-890 | GAGCCTGAGGAAGTTCTGG (SEQ ID NO: 784) | GAGCCUGAGGAAGUUCUGGTT (SEQ ID NO: 1226) | CCAGAACUUCCUCAGGCUCT T (SEQ ID NO: 1668) | 1.4 |
| RAG3A-1310 | AGCCTGCCTCCCTACCTTC (SEQ ID NO: 785) | AGCCUGCCUCCCUACCUUCTT (SEQ ID NO: 1227) | GAAGGUAGGGAGGCAGGCUT T (SEQ ID NO: 1669) | 1.4 |
| RAG3A-27 | GTCTGGTGGCTCCCTTCTC (SEQ ID NO: 786) | GUCUGGUGGCUCCCUUCUCTT (SEQ ID NO: 1228) | GAGAAGGGAGCCACCAGACT T (SEQ ID NO: 1670) | 1.4 |
| RAG3A-38 | CCTTCACCAGTGTCTGGTG (SEQ ID NO: 787) | CCUUCACCAGUGUCUGGUGTT (SEQ ID NO: 1229) | CACCAGACACUGGUGAAGGT T (SEQ ID NO: 1671) | 1.4 |
| RAG3A-532 | TGCACGTGTGCGCACATAC (SEQ ID NO: 788) | UGCACGUGUGCGCACAUACTT (SEQ ID NO: 1230) | GUAUGUGCGCACACGUGCAT T (SEQ ID NO: 1672) | 1.4 |
| RAG3A-1023 | TATCCTCCCAGCTATCCCA (SEQ ID NO: 789) | UAUCCUCCCAGCUAUCCCATT (SEQ ID NO: 1231) | UGGGAUAGCUGGGAGGAUAT T (SEQ ID NO: 1673) | 1.4 |
| RAG3A-797 | GGGAAGAAAGCAAAGGAG C (SEQ ID NO: 790) | GGGAAGAAAGCAAAGGAGCTT (SEQ ID NO: 1232) | GCUCCUUUGCUUUCUUCCCT T (SEQ ID NO: 1674) | 1.4 |
| RAG3A-399 | GGGGAATGACAAGATAGGA (SEQ ID NO: 791) | GGGGAAUGACAAGAUAGGATT (SEQ ID NO: 1233) | UCCUAUCUUGUCAUUCCCCT T (SEQ ID NO: 1675) | 1.4 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-945 | TCTAGGCCACACTTCTCAG (SEQ ID NO: 792) | UCUAGGCCACACUUCUCAGTT (SEQ ID NO: 1234) | CUGAGAAGUGUGGCCUAGATT (SEQ ID NO: 1676) | 1.4 |
| RAG3A-344 | AAGCATCCTGTTGGATTTC (SEQ ID NO: 793) | AAGCAUCCUGUUGGAUUUCTT (SEQ ID NO: 1235) | GAAAUCCAACAGGAUGCUUT T (SEQ ID NO: 1677) | 1.4 |
| RAG3A-1255 | ACAGGGACCTAGCCAGAA A (SEQ ID NO: 794) | ACAGGGACCUAGCCAGAAATT (SEQ ID NO: 1236) | UUUCUGGCUAGGUCCCUGUT T (SEQ ID NO: 1678) | 1.4 |
| RAG3A-524 | TGCGCACATACATGTGCCC (SEQ ID NO: 795) | UGCGCACAUACAUGUGCCCTT (SEQ ID NO: 1237) | GGGCACAUGUAUGUGCGCAT T (SEQ ID NO: 1679) | 1.4 |
| RAG3A-244 | TGCTTCTTCCCCAGCAACA (SEQ ID NO: 796) | UGCUUCUUCCCCAGCAACATT (SEQ ID NO: 1238) | UGUUGCUGGGGAAGAAGCAT T (SEQ ID NO: 1680) | 1.4 |
| RAG3A-340 | ATCCTGTTGGATTTCAGCA (SEQ ID NO: 797) | AUCCUGUUGGAUUUCAGCATT (SEQ ID NO: 1239) | UGCUGAAAUCCAACAGGAUT T (SEQ ID NO: 1681) | 1.4 |
| RAG3A-687 | GCCTGGTTATTCCTCTTGT (SEQ ID NO: 798) | GCCUGGUUAUUCCUCUUGUTT (SEQ ID NO: 1240) | ACAAGAGGAAUAACCAGGCT T (SEQ ID NO: 1682) | 1.4 |
| RAG3A-568 | CCAGCAGACGAGCACCTAA (SEQ ID NO: 799) | CCAGCAGACGAGCACCUAATT (SEQ ID NO: 1241) | UUAGGUGCUCGUCUGCUGGT T (SEQ ID NO: 1683) | 1.4 |
| RAG3A-804 | TGGGACTGGGAAGAAAGC A (SEQ ID NO: 800) | UGGGACUGGGAAGAAAGCATT (SEQ ID NO: 1242) | UGCUUUCUUCCCAGUCCCATT (SEQ ID NO: 1684) | 1.4 |
| RAG3A-496 | CAGTCCACTCAACCCGTCC (SEQ ID NO: 801) | CAGUCCACUCAACCCGUCCTT (SEQ ID NO: 1243) | GGACGGGUUGAGUGGACUGT T (SEQ ID NO: 1685) | 1.4 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-810 | GGAGACTGGGACTGGGAAG (SEQ ID NO: 802) | GGAGACUGGGACUGGGAAGTT (SEQ ID NO: 1244) | CUUCCCAGUCCCAGUCUCCTT (SEQ ID NO: 1686) | 1.4 |
| RAG3A-356 | AAGCAGGAAAGCAAGCATC (SEQ ID NO: 803) | AAGCAGGAAAGCAAGCAUCTT (SEQ ID NO: 1245) | GAUGCUUGCUUUCCUGCUUTT (SEQ ID NO: 1687) | 1.4 |
| RAG3A-1248 | CCTAGCCAGAAACCGGCAG (SEQ ID NO: 804) | CCUAGCCAGAAACCGGCAGTT (SEQ ID NO: 1246) | CUGCCGGUUUCUGGCUAGGTT (SEQ ID NO: 1688) | 1.4 |
| RAG3A-1015 | CAGCTATCCCAGCACCCTC (SEQ ID NO: 805) | CAGCUAUCCCAGCACCCUCTT (SEQ ID NO: 1247) | GAGGGUGCUGGGAUAGCUGTT (SEQ ID NO: 1689) | 1.4 |
| RAG3A-645 | GAAGACGGAGACAGAACAA (SEQ ID NO: 806) | GAAGACGGAGACAGAACAATT (SEQ ID NO: 1248) | UUGUUCUGUCUCCGUCUUCTT (SEQ ID NO: 1690) | 1.4 |
| RAG3A-517 | ATACATGTGCCCCGCACCT (SEQ ID NO: 807) | AUACAUGUGCCCCGCACCUTT (SEQ ID NO: 1249) | AGGUGCGGGGCACAUGUAUTT (SEQ ID NO: 1691) | 1.4 |
| RAG3A-194 | AACTTCTGCTCCTGTCCCC (SEQ ID NO: 808) | AACUUCUGCUCCUGUCCCCTT (SEQ ID NO: 1250) | GGGGACAGGAGCAGAAGUUTT (SEQ ID NO: 1692) | 1.4 |
| RAG3A-927 | GCAGACATGCCCATCCTTG (SEQ ID NO: 809) | GCAGACAUGCCCAUCCUUGTT (SEQ ID NO: 1251) | CAAGGAUGGGCAUGUCUGCTT (SEQ ID NO: 1693) | 1.4 |
| RAG3A-271 | CCCCACCAATCTTAAACAA (SEQ ID NO: 810) | CCCCACCAAUCUUAAACAATT (SEQ ID NO: 1252) | UUGUUUAAGAUUGGUGGGGTT (SEQ ID NO: 1694) | 1.4 |
| RAG3A-398 | GGGAATGACAAGATAGGAC (SEQ ID NO: 811) | GGGAAUGACAAGAUAGGACTT (SEQ ID NO: 1253) | GUCCUAUCUUGUCAUUCCCTT (SEQ ID NO: 1695) | 1.4 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-230 | CAACACAAATGTCCTGCCA (SEQ ID NO: 812) | CAACACAAAUGUCCUGCCATT (SEQ ID NO: 1254) | UGGCAGGACAUUUGUGUUGT T (SEQ ID NO: 1696) | 1.4 |
| RAG3A-1104 | CCACTCCCAGCCTCCTTTC (SEQ ID NO: 813) | CCACUCCCAGCCUCCUUUCTT (SEQ ID NO: 1255) | GAAAGGAGGCUGGGAGUGGT T (SEQ ID NO: 1697) | 1.4 |
| RAG3A-1254 | CAGGGACCTAGCCAGAAA C (SEQ ID NO: 814) | CAGGGACCUAGCCAGAAACTT (SEQ ID NO: 1256) | GUUUCUGGCUAGGUCCCUGT T (SEQ ID NO: 1698) | 1.4 |
| RAG3A-288 | TAGCCAGGGAGTGAAAAC C (SEQ ID NO: 815) | UAGCCAGGGAGUGAAAACCTT (SEQ ID NO: 1257) | GGUUUUCACUCCCUGGCUAT T (SEQ ID NO: 1699) | 1.4 |
| RAG3A-837 | GGAAGCCCCTGAGGACAG A (SEQ ID NO: 816) | GGAAGCCCCUGAGGACAGATT (SEQ ID NO: 1258) | UCUGUCCUCAGGGGCUUCCT T (SEQ ID NO: 1700) | 1.4 |
| RAG3A-935 | ACTTCTCAGCAGACATGCC (SEQ ID NO: 817) | ACUUCUCAGCAGACAUGCCTT (SEQ ID NO: 1259) | GGCAUGUCUGCUGAGAAGUT T (SEQ ID NO: 1701) | 1.4 |
| RAG3A-476 | AACCCTTTCCCCATAACAC (SEQ ID NO: 818) | AACCCUUUCCCCAUAACACTT (SEQ ID NO: 1260) | GUGUUAUGGGGAAAGGGUUT T (SEQ ID NO: 1702) | 1.4 |
| RAG3A-589 | AGCCATCTAAGCCACTGGA (SEQ ID NO: 819) | AGCCAUCUAAGCCACUGGATT (SEQ ID NO: 1261) | UCCAGUGGCUUAGAUGGCUT T (SEQ ID NO: 1703) | 1.4 |
| RAG3A-1207 | CAGTATCTCCCTCTCATTG (SEQ ID NO: 820) | CAGUAUCUCCCUCUCAUUGTT (SEQ ID NO: 1262) | CAAUGAGAGGGAGAUACUGT T (SEQ ID NO: 1704) | 1.4 |
| RAG3A-1284 | GGTCGCTCCTCATTCTTGT (SEQ ID NO: 821) | GGUCGCUCCUCAUUCUUGUTT (SEQ ID NO: 1263) | ACAAGAAUGAGGAGCGACCT T (SEQ ID NO: 1705) | 1.4 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-956 | AAATTCCAGGATCTAGGCC (SEQ ID NO: 822) | AAAUUCCAGGAUCUAGGCCTT (SEQ ID NO: 1264) | GGCCUAGAUCCUGGAAUUUT T (SEQ ID NO: 1706) | 1.4 |
| RAG3A-557 | GCACCTAAGCTCAGGCTTA (SEQ ID NO: 823) | GCACCUAAGCUCAGGCUUATT (SEQ ID NO: 1265) | UAAGCCUGAGCUUAGGUGCT T (SEQ ID NO: 1707) | 1.4 |
| RAG3A-500 | CTGACAGTCCACTCAACCC (SEQ ID NO: 824) | CUGACAGUCCACUCAACCCTT (SEQ ID NO: 1266) | GGGUUGAGUGGACUGUCAGT T (SEQ ID NO: 1708) | 1.4 |
| RAG3A-1208 | ACAGTATCTCCCTCTCATT (SEQ ID NO: 825) | ACAGUAUCUCCCUCUCAUUTT (SEQ ID NO: 1267) | AAUGAGAGGGAGAUACUGUT T (SEQ ID NO: 1709) | 1.4 |
| RAG3A-686 | CCTGGTTATTCCTCTTGTC (SEQ ID NO: 826) | CCUGGUUAUUCCUCUUGUCTT (SEQ ID NO: 1268) | GACAAGAGGAAUAACCAGGT T (SEQ ID NO: 1710) | 1.4 |
| RAG3A-1168 | TCGACAGTCCCCTCTTGCT (SEQ ID NO: 827) | UCGACAGUCCCCUCUUGCUTT (SEQ ID NO: 1269) | AGCAAGAGGGGACUGUCGAT T (SEQ ID NO: 1711) | 1.4 |
| RAG3A-218 | CCTGCCAGATTCCTCCTGG (SEQ ID NO: 828) | CCUGCCAGAUUCCUCCUGGTT (SEQ ID NO: 1270) | CCAGGAGGAAUCUGGCAGGT T (SEQ ID NO: 1712) | 1.4 |
| RAG3A-835 | AAGCCCCTGAGGACAGAG A (SEQ ID NO: 829) | AAGCCCCUGAGGACAGAGATT (SEQ ID NO: 1271) | UCUCUGUCCUCAGGGGCUUT T (SEQ ID NO: 1713) | 1.4 |
| RAG3A-291 | GGATAGCCAGGGAGTGAAA (SEQ ID NO: 830) | GGAUAGCCAGGGAGUGAAATT (SEQ ID NO: 1272) | UUUCACUCCCUGGCUAUCCT T (SEQ ID NO: 1714) | 1.3 |
| RAG3A-1222 | CCTTCTGTGGAGTGACAGT (SEQ ID NO: 831) | CCUUCUGUGGAGUGACAGUTT (SEQ ID NO: 1273) | ACUGUCACUCCACAGAAGGT T (SEQ ID NO: 1715) | 1.3 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-276 | GAAAACCCCACCAATCTTA (SEQ ID NO: 832) | GAAAACCCCACCAAUCUUATT (SEQ ID NO: 1274) | UAAGAUUGGUGGGGUUUUCT T (SEQ ID NO: 1716) | 1.3 |
| RAG3A-156 | CATGTCCAGGAAAAGATGG (SEQ ID NO: 833) | CAUGUCCAGGAAAAGAUGGTT (SEQ ID NO: 1275) | CCAUCUUUUCCUGGACAUGT T (SEQ ID NO: 1717) | 1.3 |
| RAG3A-937 | ACACTTCTCAGCAGACATG (SEQ ID NO: 834) | ACACUUCUCAGCAGACAUGTT (SEQ ID NO: 1276) | CAUGUCUGCUGAGAAGUGUT T (SEQ ID NO: 1718) | 1.3 |
| RAG3A-1262 | ACCCAGGACAGGGACCTA G (SEQ ID NO: 835) | ACCCAGGACAGGGACCUAGTT (SEQ ID NO: 1277) | CUAGGUCCCUGUCCUGGGUT T (SEQ ID NO: 1719) | 1.3 |
| RAG3A-1139 | CTTCCTTCACCCAAGGGAC (SEQ ID NO: 836) | CUUCCUUCACCCAAGGGACTT (SEQ ID NO: 1278) | GUCCCUUGGGUGAAGGAAGT T (SEQ ID NO: 1720) | 1.3 |
| RAG3A-535 | CAGTGCACGTGTGCGCACA (SEQ ID NO: 837) | CAGUGCACGUGUGCGCACATT (SEQ ID NO: 1279) | UGUGCGCACACGUGCACUGT T (SEQ ID NO: 1721) | 1.3 |
| RAG3A-719 | GCAGCTGTCTTTCCTAAGA (SEQ ID NO: 838) | GCAGCUGUCUUUCCUAAGATT (SEQ ID NO: 1280) | UCUUAGGAAAGACAGCUGCT T (SEQ ID NO: 1722) | 1.3 |
| RAG3A-726 | AGGTCCAGCAGCTGTCTTT (SEQ ID NO: 839) | AGGUCCAGCAGCUGUCUUUTT (SEQ ID NO: 1281) | AAAGACAGCUGCUGGACCUT T (SEQ ID NO: 1723) | 1.3 |
| RAG3A-384 | AGGACTCCCTAGGGGATTA (SEQ ID NO: 840) | AGGACUCCCUAGGGGAUUATT (SEQ ID NO: 1282) | UAAUCCCCUAGGGAGUCCUT T (SEQ ID NO: 1724) | 1.3 |
| RAG3A-834 | AGCCCCTGAGGACAGAGA C (SEQ ID NO: 841) | AGCCCCUGAGGACAGAGACTT (SEQ ID NO: 1283) | GUCUCUGUCCUCAGGGGCUT T (SEQ ID NO: 1725) | 1.3 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-562 | GACGAGCACCTAAGCTCAG (SEQ ID NO: 842) | GACGAGCACCUAAGCUCAGTT (SEQ ID NO: 1284) | CUGAGCUUAGGUGCUCGUCT T (SEQ ID NO: 1726) | 1.3 |
| RAG3A-940 | GCCACACTTCTCAGCAGAC (SEQ ID NO: 843) | GCCACACUUCUCAGCAGACTT (SEQ ID NO: 1285) | GUCUGCUGAGAAGUGUGGCT T (SEQ ID NO: 1727) | 1.3 |
| RAG3A-632 | GAACAAGCAAAGGAGGGC C (SEQ ID NO: 844) | GAACAAGCAAAGGAGGGCCTT (SEQ ID NO: 1286) | GGCCCUCCUUUGCUUGUUCT T (SEQ ID NO: 1728) | 1.3 |
| RAG3A-746 | CAGGGAGGTATTTGCGGGG (SEQ ID NO: 845) | CAGGGAGGUAUUUGCGGGGTT (SEQ ID NO: 1287) | CCCCGCAAAUACCUCCCUGTT (SEQ ID NO: 1729) | 1.3 |
| RAG3A-1309 | GCCTGCCTCCCTACCTTCT (SEQ ID NO: 846) | GCCUGCCUCCCUACCUUCUTT (SEQ ID NO: 1288) | AGAAGGUAGGGAGGCAGGCT T (SEQ ID NO: 1730) | 1.3 |
| RAG3A-780 | GCTAGAGCCAGGGCCAAA G (SEQ ID NO: 847) | GCUAGAGCCAGGGCCAAAGTT (SEQ ID NO: 1289) | CUUUGGCCCUGGCUCUAGCT T (SEQ ID NO: 1731) | 1.3 |
| RAG3A-693 | ACATGGGCCTGGTTATTCC (SEQ ID NO: 848) | ACAUGGGCCUGGUUAUUCCTT (SEQ ID NO: 1290) | GGAAUAACCAGGCCCAUGUT T (SEQ ID NO: 1732) | 1.3 |
| RAG3A-636 | GACAGAACAAGCAAAGGA G (SEQ ID NO: 849) | GACAGAACAAGCAAAGGAGTT (SEQ ID NO: 1291) | CUCCUUUGCUUGUUCUGUCT T (SEQ ID NO: 1733) | 1.3 |
| RAG3A-258 | AAACAAGACCTCTGTGCTT (SEQ ID NO: 850) | AAACAAGACCUCUGUGCUUTT (SEQ ID NO: 1292) | AAGCACAGAGGUCUUGUUUT T (SEQ ID NO: 1734) | 1.3 |
| RAG3A-1022 | ATCCTCCCAGCTATCCCAG (SEQ ID NO: 851) | AUCCUCCCAGCUAUCCCAGTT (SEQ ID NO: 1293) | CUGGGAUAGCUGGGAGGAUT T (SEQ ID NO: 1735) | 1.3 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-381 | ACTCCCTAGGGGATTACAG (SEQ ID NO: 852) | ACUCCCUAGGGGAUUACAGTT (SEQ ID NO: 1294) | CUGUAAUCCCCUAGGGAGUT T (SEQ ID NO: 1736) | 1.3 |
| RAG3A-379 | TCCCTAGGGGATTACAGAA (SEQ ID NO: 853) | UCCCUAGGGGAUUACAGAATT (SEQ ID NO: 1295) | UUCUGUAAUCCCCUAGGGAT T (SEQ ID NO: 1737) | 1.3 |
| RAG3A-663 | GTGGAACGGTAGGAGATGG (SEQ ID NO: 854) | GUGGAACGGUAGGAGAUGGTT (SEQ ID NO: 1296) | CCAUCUCCUACCGUUCCACTT (SEQ ID NO: 1738) | 1.3 |
| RAG3A-1099 | CCCAGCCTCCTTTCTCTTG (SEQ ID NO: 855) | CCCAGCCUCCUUUCUCUUGTT (SEQ ID NO: 1297) | CAAGAGAAAGGAGGCUGGGT T (SEQ ID NO: 1739) | 1.3 |
| RAG3A-855 | GGGATCAAGGTCAGGCCA G (SEQ ID NO: 856) | GGGAUCAAGGUCAGGCCAGTT (SEQ ID NO: 1298) | CUGGCCUGACCUUGAUCCCT T (SEQ ID NO: 1740) | 1.3 |
| RAG3A-1279 | CTCCTCATTCTTGTGTTAC (SEQ ID NO: 857) | CUCCUCAUUCUUGUGUUACTT (SEQ ID NO: 1299) | GUAACACAAGAAUGAGGAGT T (SEQ ID NO: 1741) | 1.3 |
| RAG3A-34 | CACCAGTGTCTGGTGGCTC (SEQ ID NO: 858) | CACCAGUGUCUGGUGGCUCTT (SEQ ID NO: 1300) | GAGCCACCAGACACUGGUGT T (SEQ ID NO: 1742) | 1.3 |
| RAG3A-633 | AGAACAAGCAAAGGAGGG C (SEQ ID NO: 859) | AGAACAAGCAAAGGAGGGCTT (SEQ ID NO: 1301) | GCCCUCCUUUGCUUGUUCUT T (SEQ ID NO: 1743) | 1.3 |
| RAG3A-453 | CCATAACAGGAGATTTCTC (SEQ ID NO: 860) | CCAUAACAGGAGAUUUCUCTT (SEQ ID NO: 1302) | GAGAAAUCUCCUGUUAUGGT T (SEQ ID NO: 1744) | 1.3 |
| RAG3A-246 | TGTGCTTCTTCCCCAGCAA (SEQ ID NO: 861) | UGUGCUUCUUCCCCAGCAATT (SEQ ID NO: 1303) | UUGCUGGGGAAGAAGCACAT T (SEQ ID NO: 1745) | 1.3 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-592 | TGTAGCCATCTAAGCCACT (SEQ ID NO: 862) | UGUAGCCAUCUAAGCCACUTT (SEQ ID NO: 1304) | AGUGGCUUAGAUGGCUACAT T (SEQ ID NO: 1746) | 1.3 |
| RAG3A-270 | CCCACCAATCTTAAACAAG (SEQ ID NO: 863) | CCCACCAAUCUUAAACAAGTT (SEQ ID NO: 1305) | CUUGUUUAAGAUUGGUGGGT T (SEQ ID NO: 1747) | 1.3 |
| RAG3A-630 | ACAAGCAAAGGAGGGCCC T (SEQ ID NO: 864) | ACAAGCAAAGGAGGGCCCUTT (SEQ ID NO: 1306) | AGGGCCCUCCUUUGCUUGUT T (SEQ ID NO: 1748) | 1.3 |
| RAG3A-685 | CTGGTTATTCCTCTTGTCA (SEQ ID NO: 865) | CUGGUUAUUCCUCUUGUCATT (SEQ ID NO: 1307) | UGACAAGAGGAAUAACCAGT T (SEQ ID NO: 1749) | 1.3 |
| RAG3A-1103 | CACTCCCAGCCTCCTTTCT (SEQ ID NO: 866) | CACUCCCAGCCUCCUUUCUTT (SEQ ID NO: 1308) | AGAAAGGAGGCUGGGAGUGT T (SEQ ID NO: 1750) | 1.3 |
| RAG3A-1065 | CTGCCTCCCTGTCTCCTGT (SEQ ID NO: 867) | CUGCCUCCCUGUCUCCUGUTT (SEQ ID NO: 1309) | ACAGGAGACAGGGAGGCAGT T (SEQ ID NO: 1751) | 1.3 |
| RAG3A-523 | GCGCACATACATGTGCCCC (SEQ ID NO: 868) | GCGCACAUACAUGUGCCCCTT (SEQ ID NO: 1310) | GGGGCACAUGUAUGUGCGCT T (SEQ ID NO: 1752) | 1.3 |
| RAG3A-590 | TAGCCATCTAAGCCACTGG (SEQ ID NO: 869) | UAGCCAUCUAAGCCACUGGTT (SEQ ID NO: 1311) | CCAGUGGCUUAGAUGGCUAT T (SEQ ID NO: 1753) | 1.3 |
| RAG3A-505 | CGCACCTGACAGTCCACTC (SEQ ID NO: 870) | CGCACCUGACAGUCCACUCTT (SEQ ID NO: 1312) | GAGUGGACUGUCAGGUGCGT T (SEQ ID NO: 1754) | 1.3 |
| RAG3A-1247 | CTAGCCAGAAACCGGCAGC (SEQ ID NO: 871) | CUAGCCAGAAACCGGCAGCTT (SEQ ID NO: 1313) | GCUGCCGGUUUCUGGCUAGT T (SEQ ID NO: 1755) | 1.3 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-1281 | CGCTCCTCATTCTTGTGTT (SEQ ID NO: 872) | CGCUCCUCAUUCUUGUGUUTT (SEQ ID NO: 1314) | AACACAAGAAUGAGGAGCGTT (SEQ ID NO: 1756) | 1.3 |
| RAG3A-747 | GCAGGGAGGTATTTGCGGG (SEQ ID NO: 873) | GCAGGGAGGUAUUUGCGGGTT (SEQ ID NO: 1315) | CCCGCAAAUACCUCCCUGCTT (SEQ ID NO: 1757) | 1.3 |
| RAG3A-1220 | TTCTGTGGAGTGACAGTAT (SEQ ID NO: 874) | UUCUGUGGAGUGACAGUAUTT (SEQ ID NO: 1316) | AUACUGUCACUCCACAGAATT (SEQ ID NO: 1758) | 1.3 |
| RAG3A-1268 | TGTGTTACCCAGGACAGGG (SEQ ID NO: 875) | UGUGUUACCCAGGACAGGGTT (SEQ ID NO: 1317) | CCCUGUCCUGGGUAACACATT (SEQ ID NO: 1759) | 1.3 |
| RAG3A-782 | GAGCTAGAGCCAGGGCCAA (SEQ ID NO: 876) | GAGCUAGAGCCAGGGCCAATT (SEQ ID NO: 1318) | UUGGCCCUGGCUCUAGCUCTT (SEQ ID NO: 1760) | 1.3 |
| RAG3A-107 | GTGTGGGTGGAGGAGTGGA (SEQ ID NO: 877) | GUGUGGGUGGAGGAGUGGATT (SEQ ID NO: 1319) | UCCACUCCUCCACCCACACTT (SEQ ID NO: 1761) | 1.3 |
| RAG3A-923 | ACATGCCCATCCTTGGGGA (SEQ ID NO: 878) | ACAUGCCCAUCCUUGGGGATT (SEQ ID NO: 1320) | UCCCCAAGGAUGGGCAUGUTT (SEQ ID NO: 1762) | 1.3 |
| RAG3A-349 | AAAGCAAGCATCCTGTTGG (SEQ ID NO: 879) | AAAGCAAGCAUCCUGUUGGTT (SEQ ID NO: 1321) | CCAACAGGAUGCUUGCUUUTT (SEQ ID NO: 1763) | 1.3 |
| RAG3A-1085 | TCTTGGTTCCCTGGTCATG (SEQ ID NO: 880) | UCUUGGUUCCCUGGUCAUGTT (SEQ ID NO: 1322) | CAUGACCAGGGAACCAAGATT (SEQ ID NO: 1764) | 1.3 |
| RAG3A-432 | ATGTGGGCAATATCCGTGT (SEQ ID NO: 881) | AUGUGGGCAAUAUCCGUGUTT (SEQ ID NO: 1323) | ACACGGAUAUUGCCCACAUTT (SEQ ID NO: 1765) | 1.3 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-129 | ATCCAAATCTTCTCCGTGG (SEQ ID NO: 882) | AUCCAAAUCUUCUCCGUGGTT (SEQ ID NO: 1324) | CCACGGAGAAGAUUUGGAUT T (SEQ ID NO: 1766) | 1.3 |
| RAG3A-830 | CCTGAGGACAGAGACTGT G (SEQ ID NO: 883) | CCUGAGGACAGAGACUGUGTT (SEQ ID NO: 1325) | CACAGUCUCUGUCCUCAGGT T (SEQ ID NO: 1767) | 1.3 |
| RAG3A-74 | GGGGCTCCAGGGAAGAGA A (SEQ ID NO: 884) | GGGGCUCCAGGGAAGAGAATT (SEQ ID NO: 1326) | UUCUCUUCCCUGGAGCCCCTT (SEQ ID NO: 1768) | 1.3 |
| RAG3A-856 | TGGGATCAAGGTCAGGCCA (SEQ ID NO: 885) | UGGGAUCAAGGUCAGGCCATT (SEQ ID NO: 1327) | UGGCCUGACCUUGAUCCCATT (SEQ ID NO: 1769) | 1.3 |
| RAG3A-435 | CTCATGTGGGCAATATCCG (SEQ ID NO: 886) | CUCAUGUGGGCAAUAUCCGTT (SEQ ID NO: 1328) | CGGAUAUUGCCCACAUGAGT T (SEQ ID NO: 1770) | 1.3 |
| RAG3A-564 | CAGACGAGCACCTAAGCTC (SEQ ID NO: 887) | CAGACGAGCACCUAAGCUCTT (SEQ ID NO: 1329) | GAGCUUAGGUGCUCGUCUGT T (SEQ ID NO: 1771) | 1.3 |
| RAG3A-1251 | GGACCTAGCCAGAAACCG G (SEQ ID NO: 888) | GGACCUAGCCAGAAACCGGTT (SEQ ID NO: 1330) | CCGGUUUCUGGCUAGGUCCT T (SEQ ID NO: 1772) | 1.3 |
| RAG3A-1291 | TCTCTGAGGTCGCTCCTCA (SEQ ID NO: 889) | UCUCUGAGGUCGCUCCUCATT (SEQ ID NO: 1331) | UGAGGAGCGACCUCAGAGAT T (SEQ ID NO: 1773) | 1.3 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-781 | AGCTAGAGCCAGGGCCAA A (SEQ ID NO: 890) | AGCUAGAGCCAGGGCCAAATT (SEQ ID NO: 1332) | UUUGGCCCUGGCUCUAGCUT T (SEQ ID NO: 1774) | 1.3 |
| RAG3A-261 | CTTAAACAAGACCTCTGTG (SEQ ID NO: 891) | CUUAAACAAGACCUCUGUGTT (SEQ ID NO: 1333) | CACAGAGGUCUUGUUUAAGT T (SEQ ID NO: 1775) | 1.3 |
| RAG3A-1098 | CCAGCCTCCTTTCTCTTGG (SEQ ID NO: 892) | CCAGCCUCCUUUCUCUUGGTT (SEQ ID NO: 1334) | CCAAGAGAAAGGAGGCUGGT T (SEQ ID NO: 1776) | 1.3 |
| RAG3A-565 | GCAGACGAGCACCTAAGCT (SEQ ID NO: 893) | GCAGACGAGCACCUAAGCUTT (SEQ ID NO: 1335) | AGCUUAGGUGCUCGUCUGCT T (SEQ ID NO: 1777) | 1.2 |
| RAG3A-930 | TCAGCAGACATGCCCATCC (SEQ ID NO: 894) | UCAGCAGACAUGCCCAUCCTT (SEQ ID NO: 1336) | GGAUGGGCAUGUCUGCUGAT T (SEQ ID NO: 1778) | 1.2 |
| RAG3A-422 | TATCCGTGTTCCCACTTCG (SEQ ID NO: 895) | UAUCCGUGUUCCCACUUCGTT (SEQ ID NO: 1337) | CGAAGUGGGAACACGGAUAT T (SEQ ID NO: 1779) | 1.2 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-516 | TACATGTGCCCCGCACCTG (SEQ ID NO: 896) | UACAUGUGCCCCGCACCUGTT (SEQ ID NO: 1338) | CAGGUGCGGGGCACAUGUAT T (SEQ ID NO: 1780) | 1.2 |
| RAG3A-1190 | TGTAACTTATCCTCAGGCG (SEQ ID NO: 897) | UGUAACUUAUCCUCAGGCGTT (SEQ ID NO: 1339) | CGCCUGAGGAUAAGUUACAT T (SEQ ID NO: 1781) | 1.2 |
| RAG3A-360 | AGAAAAGCAGGAAAGCAA G (SEQ ID NO: 898) | AGAAAAGCAGGAAAGCAAGTT (SEQ ID NO: 1340) | CUUGCUUUCCUGCUUUUCUT T (SEQ ID NO: 1782) | 1.2 |
| RAG3A-126 | CAAATCTTCTCCGTGGTGT (SEQ ID NO: 899) | CAAAUCUUCUCCGUGGUGUTT (SEQ ID NO: 1341) | ACACCACGGAGAAGAUUUGT T (SEQ ID NO: 1783) | 1.2 |
| RAG3A-938 | CACACTTCTCAGCAGACAT (SEQ ID NO: 900) | CACACUUCUCAGCAGACAUTT (SEQ ID NO: 1342) | AUGUCUGCUGAGAAGUGUGT T (SEQ ID NO: 1784) | 1.2 |
| RAG3A-853 | GATCAAGGTCAGGCCAGG A (SEQ ID NO: 901) | GAUCAAGGUCAGGCCAGGATT (SEQ ID NO: 1343) | UCCUGGCCUGACCUUGAUCT T (SEQ ID NO: 1785) | 1.2 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-716 | GCTGTCTTTCCTAAGACAG (SEQ ID NO: 902) | GCUGUCUUUCCUAAGACAGTT (SEQ ID NO: 1344) | CUGUCUUAGGAAAGACAGCT T (SEQ ID NO: 1786) | 1.2 |
| RAG3A-1027 | CCACTATCCTCCCAGCTAT (SEQ ID NO: 903) | CCACUAUCCUCCCAGCUAUTT (SEQ ID NO: 1345) | AUAGCUGGGAGGAUAGUGGT T (SEQ ID NO: 1787) | 1.2 |
| RAG3A-640 | CGGAGACAGAACAAGCAA A (SEQ ID NO: 904) | CGGAGACAGAACAAGCAAATT (SEQ ID NO: 1346) | UUUGCUUGUUCUGUCUCCGT T (SEQ ID NO: 1788) | 1.2 |
| RAG3A-284 | CAGGGAGTGAAAACCCCA C (SEQ ID NO: 905) | CAGGGAGUGAAAACCCCACTT (SEQ ID NO: 1347) | GUGGGGUUUUCACUCCCUGT T (SEQ ID NO: 1789) | 1.2 |
| RAG3A-705 | TAAGACAGGGACACATGGG (SEQ ID NO: 906) | UAAGACAGGGACACAUGGGTT (SEQ ID NO: 1348) | CCCAUGUGUCCCUGUCUUATT (SEQ ID NO: 1790) | 1.2 |
| RAG3A-1315 | TGCCTAGCCTGCCTCCCTA (SEQ ID NO: 907) | UGCCUAGCCUGCCUCCCUATT (SEQ ID NO: 1349) | UAGGGAGGCAGGCUAGGCAT T (SEQ ID NO: 1791) | 1.2 |
| RAG3A-1381 | TCCATCAGCCGATCTCCCC (SEQ ID NO: 908) | UCCAUCAGCCGAUCUCCCCTT (SEQ ID NO: 1350) | GGGGAGAUCGGCUGAUGGAT T (SEQ ID NO: 1792) | 1.2 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-558 | AGCACCTAAGCTCAGGCTT (SEQ ID NO: 909) | AGCACCUAAGCUCAGGCUUTT (SEQ ID NO: 1351) | AAGCCUGAGCUUAGGUGCUT T (SEQ ID NO: 1793) | 1.2 |
| RAG3A-498 | GACAGTCCACTCAACCCGT (SEQ ID NO: 910) | GACAGUCCACUCAACCCGUTT (SEQ ID NO: 1352) | ACGGGUUGAGUGGACUGUCT T (SEQ ID NO: 1794) | 1.2 |
| RAG3A-581 | AAGCCACTGGACCCCAGC A (SEQ ID NO: 911) | AAGCCACUGGACCCCAGCATT (SEQ ID NO: 1353) | UGCUGGGGUCCAGUGGCUUT T (SEQ ID NO: 1795) | 1.2 |
| RAG3A-1135 | CTTCACCCAAGGGACCCTC (SEQ ID NO: 912) | CUUCACCCAAGGGACCCUCTT (SEQ ID NO: 1354) | GAGGGUCCCUUGGGUGAAGT T (SEQ ID NO: 1796) | 1.2 |
| RAG3A-1070 | CATGCCTGCCTCCCTGTCT (SEQ ID NO: 913) | CAUGCCUGCCUCCCUGUCUTT (SEQ ID NO: 1355) | AGACAGGGAGGCAGGCAUGT T (SEQ ID NO: 1797) | 1.2 |
| RAG3A-1181 | TCCTCAGGCGCATTCGACA (SEQ ID NO: 914) | UCCUCAGGCGCAUUCGACATT (SEQ ID NO: 1356) | UGUCGAAUGCGCCUGAGGAT T (SEQ ID NO: 1798) | 1.2 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-1206 | AGTATCTCCCTCTCATTGT (SEQ ID NO: 915) | AGUAUCUCCCUCUCAUUGUTT (SEQ ID NO: 1357) | ACAAUGAGAGGGAGAUACUT T (SEQ ID NO: 1799) | 1.2 |
| RAG3A-461 | ACACCAACCCATAACAGGA (SEQ ID NO: 916) | ACACCAACCCAUAACAGGATT (SEQ ID NO: 1358) | UCCUGUUAUGGGUUGGUGUT T (SEQ ID NO: 1800) | 1.2 |
| RAG3A-242 | CTTCTTCCCCAGCAACACA (SEQ ID NO: 917) | CUUCUUCCCCAGCAACACATT (SEQ ID NO: 1359) | UGUGUUGCUGGGGAAGAAGT T (SEQ ID NO: 1801) | 1.2 |
| RAG3A-1127 | AAGGGACCCTCTGCCTCTC (SEQ ID NO: 918) | AAGGGACCCUCUGCCUCUCTT (SEQ ID NO: 1360) | GAGAGGCAGAGGGUCCCUUT T (SEQ ID NO: 1802) | 1.2 |
| RAG3A-419 | CCGTGTTCCCACTTCGAAA (SEQ ID NO: 919) | CCGUGUUCCCACUUCGAAATT (SEQ ID NO: 1361) | UUUCGAAGUGGGAACACGGT T (SEQ ID NO: 1803) | 1.2 |
| RAG3A-790 | AAGCAAAGGAGCTAGAGC C (SEQ ID NO: 920) | AAGCAAAGGAGCUAGAGCCTT (SEQ ID NO: 1362) | GGCUCUAGCUCCUUUGCUUT T (SEQ ID NO: 1804) | 1.2 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-635 | ACAGAACAAGCAAAGGAGG (SEQ ID NO: 921) | ACAGAACAAGCAAAGGAGGTT (SEQ ID NO: 1363) | CCUCCUUUGCUUGUUCUGUTT (SEQ ID NO: 1805) | 1.2 |
| RAG3A-673 | CTTGTCACATGTGGAACGG (SEQ ID NO: 922) | CUUGUCACAUGUGGAACGGTT (SEQ ID NO: 1364) | CCGUUCCACAUGUGACAAGTT (SEQ ID NO: 1806) | 1.2 |
| RAG3A-1001 | CCCTCCTTCCTAATCTTGG (SEQ ID NO: 923) | CCCUCCUUCCUAAUCUUGGTT (SEQ ID NO: 1365) | CCAAGAUUAGGAAGGAGGGTT (SEQ ID NO: 1807) | 1.2 |
| RAG3A-1005 | AGCACCCTCCTTCCTAATC (SEQ ID NO: 924) | AGCACCCUCCUUCCUAAUCTT (SEQ ID NO: 1366) | GAUUAGGAAGGAGGGUGCUTT (SEQ ID NO: 1808) | 1.2 |
| RAG3A-1064 | TGCCTCCCTGTCTCCTGTC (SEQ ID NO: 925) | UGCCUCCCUGUCUCCUGUCTT (SEQ ID NO: 1367) | GACAGGAGACAGGGAGGCATT (SEQ ID NO: 1809) | 1.2 |
| RAG3A-801 | GACTGGGAAGAAAGCAAAG (SEQ ID NO: 926) | GACUGGGAAGAAAGCAAAGTT (SEQ ID NO: 1368) | CUUUGCUUUCUUCCCAGUCTT (SEQ ID NO: 1810) | 1.2 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-486 | AACCCGTCCAAACCCTTTC (SEQ ID NO: 927) | AACCCGUCCAAACCCUUUCTT (SEQ ID NO: 1369) | GAAAGGGUUUGGACGGGUUT T (SEQ ID NO: 1811) | 1.2 |
| RAG3A-555 | ACCTAAGCTCAGGCTTAAC (SEQ ID NO: 928) | ACCUAAGCUCAGGCUUAACTT (SEQ ID NO: 1370) | GUUAAGCCUGAGCUUAGGUT T (SEQ ID NO: 1812) | 1.2 |
| RAG3A-854 | GGATCAAGGTCAGGCCAG G (SEQ ID NO: 929) | GGAUCAAGGUCAGGCCAGGTT (SEQ ID NO: 1371) | CCUGGCCUGACCUUGAUCCT T (SEQ ID NO: 1813) | 1.2 |
| RAG3A-1267 | GTGTTACCCAGGACAGGGA (SEQ ID NO: 930) | GUGUUACCCAGGACAGGGATT (SEQ ID NO: 1372) | UCCCUGUCCUGGGUAACACT T (SEQ ID NO: 1814) | 1.2 |
| RAG3A-1264 | TTACCCAGGACAGGGACCT (SEQ ID NO: 931) | UUACCCAGGACAGGGACCUTT (SEQ ID NO: 1373) | AGGUCCCUGUCCUGGGUAAT T (SEQ ID NO: 1815) | 1.2 |
| RAG3A-513 | ATGTGCCCCGCACCTGACA (SEQ ID NO: 932) | AUGUGCCCCGCACCUGACATT (SEQ ID NO: 1374) | UGUCAGGUGCGGGGCACAUT T (SEQ ID NO: 1816) | 1.2 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-1286 | GAGGTCGCTCCTCATTCTT (SEQ ID NO: 933) | GAGGUCGCUCCUCAUUCUUTT (SEQ ID NO: 1375) | AAGAAUGAGGAGCGACCUCT T (SEQ ID NO: 1817) | 1.2 |
| RAG3A-653 | AGGAGATGGAAGACGGAG A (SEQ ID NO: 934) | AGGAGAUGGAAGACGGAGATT (SEQ ID NO: 1376) | UCUCCGUCUUCCAUCUCCUTT (SEQ ID NO: 1818) | 1.2 |
| RAG3A-1014 | AGCTATCCCAGCACCCTCC (SEQ ID NO: 935) | AGCUAUCCCAGCACCCUCCTT (SEQ ID NO: 1377) | GGAGGGUGCUGGGAUAGCUT T (SEQ ID NO: 1819) | 1.2 |
| RAG3A-61 | AGAGAAGGCGTCACTTCCG (SEQ ID NO: 936) | AGAGAAGGCGUCACUUCCGTT (SEQ ID NO: 1378) | CGGAAGUGACGCCUUCUCUT T (SEQ ID NO: 1820) | 1.2 |
| RAG3A-666 | CATGTGGAACGGTAGGAGA (SEQ ID NO: 937) | CAUGUGGAACGGUAGGAGATT (SEQ ID NO: 1379) | UCUCCUACCGUUCCACAUGT T (SEQ ID NO: 1821) | 1.2 |
| RAG3A-348 | AAGCAAGCATCCTGTTGGA (SEQ ID NO: 938) | AAGCAAGCAUCCUGUUGGATT (SEQ ID NO: 1380) | UCCAACAGGAUGCUUGCUUT T (SEQ ID NO: 1822) | 1.2 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-545 | AGGCTTAACCCAGTGCACG (SEQ ID NO: 939) | AGGCUUAACCCAGUGCACGTT (SEQ ID NO: 1381) | CGUGCACUGGGUUAAGCCUTT (SEQ ID NO: 1823) | 1.2 |
| RAG3A-584 | TCTAAGCCACTGGACCCCA (SEQ ID NO: 940) | UCUAAGCCACUGGACCCCATT (SEQ ID NO: 1382) | UGGGGUCCAGUGGCUUAGATT (SEQ ID NO: 1824) | 1.2 |
| RAG3A-1162 | GTCCCCTCTTGCTTTCTCA (SEQ ID NO: 941) | GUCCCCUCUUGCUUUCUCATT (SEQ ID NO: 1383) | UGAGAAAGCAAGAGGGGACTT (SEQ ID NO: 1825) | 1.2 |
| RAG3A-308 | CCAGGGAAAAGAAATTTGG (SEQ ID NO: 942) | CCAGGGAAAAGAAAUUUGGTT (SEQ ID NO: 1384) | CCAAAUUUCUUUUCCCUGGTT (SEQ ID NO: 1826) | 1.2 |
| RAG3A-158 | CCCATGTCCAGGAAAAGAT (SEQ ID NO: 943) | CCCAUGUCCAGGAAAAGAUTT (SEQ ID NO: 1385) | AUCUUUUCCUGGACAUGGGTT (SEQ ID NO: 1827) | 1.2 |
| RAG3A-161 | TTGCCCATGTCCAGGAAAA (SEQ ID NO: 944) | UUGCCCAUGUCCAGGAAAATT (SEQ ID NO: 1386) | UUUUCCUGGACAUGGGCAATT (SEQ ID NO: 1828) | 1.2 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-1253 | AGGGACCTAGCCAGAAAC C (SEQ ID NO: 945) | AGGGACCUAGCCAGAAACCTT (SEQ ID NO: 1387) | GGUUUCUGGCUAGGUCCCUT T (SEQ ID NO: 1829) | 1.2 |
| RAG3A-926 | CAGACATGCCCATCCTTGG (SEQ ID NO: 946) | CAGACAUGCCCAUCCUUGGTT (SEQ ID NO: 1388) | CCAAGGAUGGGCAUGUCUGT T (SEQ ID NO: 1830) | 1.2 |
| RAG3A-1191 | TTGTAACTTATCCTCAGGC (SEQ ID NO: 947) | UUGUAACUUAUCCUCAGGCTT (SEQ ID NO: 1389) | GCCUGAGGAUAAGUUACAAT T (SEQ ID NO: 1831) | 1.2 |
| RAG3A-697 | GGACACATGGGCCTGGTTA (SEQ ID NO: 948) | GGACACAUGGGCCUGGUUATT (SEQ ID NO: 1390) | UAACCAGGCCCAUGUGUCCT T (SEQ ID NO: 1832) | 1.2 |
| RAG3A-1224 | CCCCTTCTGTGGAGTGACA (SEQ ID NO: 949) | CCCCUUCUGUGGAGUGACATT (SEQ ID NO: 1391) | UGUCACUCCACAGAAGGGGT T (SEQ ID NO: 1833) | 1.2 |
| RAG3A-964 | GGACGGGAAAATTCCAGG A (SEQ ID NO: 950) | GGACGGGAAAAUUCCAGGATT (SEQ ID NO: 1392) | UCCUGGAAUUUUCCCGUCCT T (SEQ ID NO: 1834) | 1.2 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-791 | AAAGCAAAGGAGCTAGAGC (SEQ ID NO: 951) | AAAGCAAAGGAGCUAGAGCTT (SEQ ID NO: 1393) | GCUCUAGCUCCUUUGCUUUT T (SEQ ID NO: 1835) | 1.2 |
| RAG3A-526 | TGTGCGCACATACATGTGC (SEQ ID NO: 952) | UGUGCGCACAUACAUGUGCTT (SEQ ID NO: 1394) | GCACAUGUAUGUGCGCACAT T (SEQ ID NO: 1836) | 1.2 |
| RAG3A-972 | GTCTGGCTGGACGGGAAAA (SEQ ID NO: 953) | GUCUGGCUGGACGGGAAAATT (SEQ ID NO: 1395) | UUUUCCCGUCCAGCCAGACT T (SEQ ID NO: 1837) | 1.2 |
| RAG3A-289 | ATAGCCAGGGAGTGAAAAC (SEQ ID NO: 954) | AUAGCCAGGGAGUGAAAACTT (SEQ ID NO: 1396) | GUUUUCACUCCCUGGCUAUT T (SEQ ID NO: 1838) | 1.2 |
| RAG3A-255 | CAAGACCTCTGTGCTTCTT (SEQ ID NO: 955) | CAAGACCUCUGUGCUUCUUTT (SEQ ID NO: 1397) | AAGAAGCACAGAGGUCUUGT T (SEQ ID NO: 1839) | 1.2 |
| RAG3A-1000 | CCTCCTTCCTAATCTTGGG (SEQ ID NO: 956) | CCUCCUUCCUAAUCUUGGGTT (SEQ ID NO: 1398) | CCCAAGAUUAGGAAGGAGGT T (SEQ ID NO: 1840) | 1.2 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-694 | CACATGGGCCTGGTTATTC (SEQ ID NO: 957) | CACAUGGGCCUGGUUAUUCTT (SEQ ID NO: 1399) | GAAUAACCAGGCCCAUGUGT T (SEQ ID NO: 1841) | 1.2 |
| RAG3A-549 | GCTCAGGCTTAACCCAGTG (SEQ ID NO: 958) | GCUCAGGCUUAACCCAGUGTT (SEQ ID NO: 1400) | CACUGGGUUAAGCCUGAGCT T (SEQ ID NO: 1842) | 1.2 |
| RAG3A-1240 | GAAACCGGCAGCATTCCCC (SEQ ID NO: 959) | GAAACCGGCAGCAUUCCCCTT (SEQ ID NO: 1401) | GGGGAAUGCUGCCGGUUUCT T (SEQ ID NO: 1843) | 1.2 |
| RAG3A-978 | CATCTCGTCTGGCTGGACG (SEQ ID NO: 960) | CAUCUCGUCUGGCUGGACGTT (SEQ ID NO: 1402) | CGUCCAGCCAGACGAGAUGT T (SEQ ID NO: 1844) | 1.2 |
| RAG3A-1290 | CTCTGAGGTCGCTCCTCAT (SEQ ID NO: 961) | CUCUGAGGUCGCUCCUCAUTT (SEQ ID NO: 1403) | AUGAGGAGCGACCUCAGAGT T (SEQ ID NO: 1845) | 1.2 |
| RAG3A-1006 | CAGCACCCTCCTTCCTAAT (SEQ ID NO: 962) | CAGCACCCUCCUUCCUAAUTT (SEQ ID NO: 1404) | AUUAGGAAGGAGGGUGCUGT T (SEQ ID NO: 1846) | 1.2 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-1311 | TAGCCTGCCTCCCTACCTT (SEQ ID NO: 963) | UAGCCUGCCUCCCUACCUUTT (SEQ ID NO: 1405) | AAGGUAGGGAGGCAGGCUAT T (SEQ ID NO: 1847) | 1.2 |
| RAG3A-42 | GGGGCCTTCACCAGTGTCT (SEQ ID NO: 964) | GGGGCCUUCACCAGUGUCUTT (SEQ ID NO: 1406) | AGACACUGGUGAAGGCCCCT T (SEQ ID NO: 1848) | 1.2 |
| RAG3A-21 | TGGCTCCCTTCTCTGATTG (SEQ ID NO: 965) | UGGCUCCCUUCUCUGAUUGTT (SEQ ID NO: 1407) | CAAUCAGAGAAGGGAGCCAT T (SEQ ID NO: 1849) | 1.2 |
| RAG3A-59 | AGAAGGCGTCACTTCCGGG (SEQ ID NO: 966) | AGAAGGCGUCACUUCCGGGTT (SEQ ID NO: 1408) | CCCGGAAGUGACGCCUUCUT T (SEQ ID NO: 1850) | 1.2 |
| RAG3A-1187 | AACTTATCCTCAGGCGCAT (SEQ ID NO: 967) | AACUUAUCCUCAGGCGCAUTT (SEQ ID NO: 1409) | AUGCGCCUGAGGAUAAGUUT T (SEQ ID NO: 1851) | 1.2 |
| RAG3A-576 | ACTGGACCCCAGCAGACG A (SEQ ID NO: 968) | ACUGGACCCCAGCAGACGATT (SEQ ID NO: 1410) | UCGUCUGCUGGGGUCCAGUT T (SEQ ID NO: 1852) | 1.1 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-639 | GGAGACAGAACAAGCAAAG (SEQ ID NO: 969) | GGAGACAGAACAAGCAAAGTT (SEQ ID NO: 1411) | CUUUGCUUGUUCUGUCUCCTT (SEQ ID NO: 1853) | 1.1 |
| RAG3A-815 | TGTGGGGAGACTGGGACTG (SEQ ID NO: 970) | UGUGGGGAGACUGGGACUGTT (SEQ ID NO: 1412) | CAGUCCCAGUCUCCCCACATT (SEQ ID NO: 1854) | 1.1 |
| RAG3A-957 | AAAATTCCAGGATCTAGGC (SEQ ID NO: 971) | AAAAUUCCAGGAUCUAGGCTT (SEQ ID NO: 1413) | GCCUAGAUCCUGGAAUUUUTT (SEQ ID NO: 1855) | 1.1 |
| RAG3A-1217 | TGTGGAGTGACAGTATCTC (SEQ ID NO: 972) | UGUGGAGUGACAGUAUCUCTT (SEQ ID NO: 1414) | GAGAUACUGUCACUCCACATT (SEQ ID NO: 1856) | 1.1 |
| RAG3A-1165 | ACAGTCCCCTCTTGCTTTC (SEQ ID NO: 973) | ACAGUCCCCUCUUGCUUUCTT (SEQ ID NO: 1415) | GAAAGCAAGAGGGGACUGUTT (SEQ ID NO: 1857) | 1.1 |
| RAG3A-1134 | TTCACCCAAGGGACCCTCT (SEQ ID NO: 974) | UUCACCCAAGGGACCCUCUTT (SEQ ID NO: 1416) | AGAGGGUCCCUUGGGUGAATT (SEQ ID NO: 1858) | 1.1 |
| RAG3A-667 | ACATGTGGAACGGTAGGAG (SEQ ID NO: 975) | ACAUGUGGAACGGUAGGAGTT (SEQ ID NO: 1417) | CUCCUACCGUUCCACAUGUTT (SEQ ID NO: 1859) | 1.1 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-1339 | CAAACCCTCACCACCCTCT (SEQ ID NO: 976) | CAAACCCUCACCACCCUCUTT (SEQ ID NO: 1418) | AGAGGGUGGUGAGGGUUUGT T (SEQ ID NO: 1860) | 1.1 |
| RAG3A-676 | CCTCTTGTCACATGTGGAA (SEQ ID NO: 977) | CCUCUUGUCACAUGUGGAATT (SEQ ID NO: 1419) | UUCCACAUGUGACAAGAGGT T (SEQ ID NO: 1861) | 1.1 |
| RAG3A-153 | GTCCAGGAAAAGATGGATC (SEQ ID NO: 978) | GUCCAGGAAAAGAUGGAUCTT (SEQ ID NO: 1420) | GAUCCAUCUUUUCCUGGACT T (SEQ ID NO: 1862) | 1.1 |
| RAG3A-320 | CAGGTATGATGTCCAGGGA (SEQ ID NO: 979) | CAGGUAUGAUGUCCAGGGATT (SEQ ID NO: 1421) | UCCCUGGACAUCAUACCUGT T (SEQ ID NO: 1863) | 1.1 |
| RAG3A-1010 | ATCCCAGCACCCTCCTTCC (SEQ ID NO: 980) | AUCCCAGCACCCUCCUUCCTT (SEQ ID NO: 1422) | GGAAGGAGGGUGCUGGGAUT T (SEQ ID NO: 1864) | 1.1 |
| RAG3A-999 | CTCCTTCCTAATCTTGGGA (SEQ ID NO: 981) | CUCCUUCCUAAUCUUGGGATT (SEQ ID NO: 1423) | UCCCAAGAUUAGGAAGGAGT T (SEQ ID NO: 1865) | 1.1 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-567 | CAGCAGACGAGCACCTAAG (SEQ ID NO: 982) | CAGCAGACGAGCACCUAAGTT (SEQ ID NO: 1424) | CUUAGGUGCUCGUCUGCUGTT (SEQ ID NO: 1866) | 1.1 |
| RAG3A-862 | GGATGATGGGATCAAGGTC (SEQ ID NO: 983) | GGAUGAUGGGAUCAAGGUCTT (SEQ ID NO: 1425) | GACCUUGAUCCCAUCAUCCTT (SEQ ID NO: 1867) | 1.1 |
| RAG3A-460 | CACCAACCCATAACAGGAG (SEQ ID NO: 984) | CACCAACCCAUAACAGGAGTT (SEQ ID NO: 1426) | CUCCUGUUAUGGGUUGGUGTT (SEQ ID NO: 1868) | 1.1 |
| RAG3A-921 | ATGCCCATCCTTGGGGAGG (SEQ ID NO: 985) | AUGCCCAUCCUUGGGGAGGTT (SEQ ID NO: 1427) | CCUCCCCAAGGAUGGGCAUTT (SEQ ID NO: 1869) | 1.1 |
| RAG3A-704 | AAGACAGGGACACATGGGC (SEQ ID NO: 986) | AAGACAGGGACACAUGGGCTT (SEQ ID NO: 1428) | GCCCAUGUGUCCCUGUCUUTT (SEQ ID NO: 1870) | 1.1 |
| RAG3A-233 | CAGCAACACAAATGTCCTG (SEQ ID NO: 987) | CAGCAACACAAAUGUCCUGTT (SEQ ID NO: 1429) | CAGGACAUUUGUGUUGCUGTT (SEQ ID NO: 1871) | 1.1 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-1203 | ATCTCCCTCTCATTGTAAC (SEQ ID NO: 988) | AUCUCCCUCUCAUUGUAACTT (SEQ ID NO: 1430) | GUUACAAUGAGAGGGAGAUT T (SEQ ID NO: 1872) | 1.1 |
| RAG3A-247 | CTGTGCTTCTTCCCCAGCA (SEQ ID NO: 989) | CUGUGCUUCUUCCCCAGCATT (SEQ ID NO: 1431) | UGCUGGGGAAGAAGCACAGT T (SEQ ID NO: 1873) | 1.1 |
| RAG3A-721 | CAGCAGCTGTCTTTCCTAA (SEQ ID NO: 990) | CAGCAGCUGUCUUUCCUAATT (SEQ ID NO: 1432) | UUAGGAAAGACAGCUGCUGT T (SEQ ID NO: 1874) | 1.1 |
| RAG3A-391 | ACAAGATAGGACTCCCTAG (SEQ ID NO: 991) | ACAAGAUAGGACUCCCUAGTT (SEQ ID NO: 1433) | CUAGGGAGUCCUAUCUUGUT T (SEQ ID NO: 1875) | 1.1 |
| RAG3A-159 | GCCCATGTCCAGGAAAAGA (SEQ ID NO: 992) | GCCCAUGUCCAGGAAAAGATT (SEQ ID NO: 1434) | UCUUUUCCUGGACAUGGGCT T (SEQ ID NO: 1876) | 1.1 |
| RAG3A-437 | CTCTCATGTGGGCAATATC (SEQ ID NO: 993) | CUCUCAUGUGGGCAAUAUCTT (SEQ ID NO: 1435) | GAUAUUGCCCACAUGAGAGT T (SEQ ID NO: 1877) | 1.1 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-29 | GTGTCTGGTGGCTCCCTTC (SEQ ID NO: 994) | GUGUCUGGUGGCUCCCUUCTT (SEQ ID NO: 1436) | GAAGGGAGCCACCAGACACT T (SEQ ID NO: 1878) | 1.1 |
| RAG3A-63 | GAAGAGAAGGCGTCACTT C (SEQ ID NO: 995) | GAAGAGAAGGCGUCACUUCTT (SEQ ID NO: 1437) | GAAGUGACGCCUUCUCUUCT T (SEQ ID NO: 1879) | 1.1 |
| RAG3A-996 | CTTCCTAATCTTGGGAGAC (SEQ ID NO: 996) | CUUCCUAAUCUUGGGAGACTT (SEQ ID NO: 1438) | GUCUCCCAAGAUUAGGAAGT T (SEQ ID NO: 1880) | 1.1 |
| RAG3A-1083 | TTGGTTCCCTGGTCATGCC (SEQ ID NO: 997) | UUGGUUCCCUGGUCAUGCCTT (SEQ ID NO: 1439) | GGCAUGACCAGGGAACCAAT T (SEQ ID NO: 1881) | 1.1 |
| RAG3A-1128 | CAAGGGACCCTCTGCCTCT (SEQ ID NO: 998) | CAAGGGACCCUCUGCCUCUTT (SEQ ID NO: 1440) | AGAGGCAGAGGGUCCCUUGT T (SEQ ID NO: 1882) | 1.1 |
| RAG3A-1094 | CCTCCTTTCTCTTGGTTCC (SEQ ID NO: 999) | CCUCCUUUCUCUUGGUUCCTT (SEQ ID NO: 1441) | GGAACCAAGAGAAAGGAGGT T (SEQ ID NO: 1883) | 1.1 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-771 | AGGGCCAAAGGAAAAGGGG (SEQ ID NO: 1000) | AGGGCCAAAGGAAAAGGGGTT (SEQ ID NO: 1442) | CCCCUUUUCCUUUGGCCCUTT (SEQ ID NO: 1884) | 1.1 |
| RAG3A-287 | AGCCAGGGAGTGAAAACCC (SEQ ID NO: 1001) | AGCCAGGGAGUGAAAACCCTT (SEQ ID NO: 1443) | GGGUUUUCACUCCCUGGCUTT (SEQ ID NO: 1885) | 1.1 |
| RAG3A-981 | AGACATCTCGTCTGGCTGG (SEQ ID NO: 1002) | AGACAUCUCGUCUGGCUGGTT (SEQ ID NO: 1444) | CCAGCCAGACGAGAUGUCUTT (SEQ ID NO: 1886) | 1.1 |
| RAG3A-23 | GGTGGCTCCCTTCTCTGAT (SEQ ID NO: 1003) | GGUGGCUCCCUUCUCUGAUTT (SEQ ID NO: 1445) | AUCAGAGAAGGGAGCCACCTT (SEQ ID NO: 1887) | 1.1 |
| RAG3A-1072 | GTCATGCCTGCCTCCCTGT (SEQ ID NO: 1004) | GUCAUGCCUGCCUCCCUGUTT (SEQ ID NO: 1446) | ACAGGGAGGCAGGCAUGACTT (SEQ ID NO: 1888) | 1.1 |
| RAG3A-415 | GTTCCCACTTCGAAAGGGG (SEQ ID NO: 1005) | GUUCCCACUUCGAAAGGGGTT (SEQ ID NO: 1447) | CCCCUUUCGAAGUGGGAACTT (SEQ ID NO: 1889) | 1.1 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-1287 | TGAGGTCGCTCCTCATTCT (SEQ ID NO: 1006) | UGAGGUCGCUCCUCAUUCUTT (SEQ ID NO: 1448) | AGAAUGAGGAGCGACCUCAT T (SEQ ID NO: 1890) | 1.1 |
| RAG3A-1201 | CTCCCTCTCATTGTAACTT (SEQ ID NO: 1007) | CUCCCUCUCAUUGUAACUUTT (SEQ ID NO: 1449) | AAGUUACAAUGAGAGGGAGT T (SEQ ID NO: 1891) | 1.1 |
| RAG3A-772 | CAGGGCCAAAGGAAAAGG G (SEQ ID NO: 1008) | CAGGGCCAAAGGAAAAGGGTT (SEQ ID NO: 1450) | CCCUUUUCCUUUGGCCCUGT T (SEQ ID NO: 1892) | 1.1 |
| RAG3A-1249 | ACCTAGCCAGAAACCGGCA (SEQ ID NO: 1009) | ACCUAGCCAGAAACCGGCATT (SEQ ID NO: 1451) | UGCCGGUUUCUGGCUAGGUT T (SEQ ID NO: 1893) | 1.1 |
| RAG3A-546 | CAGGCTTAACCCAGTGCAC (SEQ ID NO: 1010) | CAGGCUUAACCCAGUGCACTT (SEQ ID NO: 1452) | GUGCACUGGGUUAAGCCUGT T (SEQ ID NO: 1894) | 1.1 |
| RAG3A-160 | TGCCCATGTCCAGGAAAAG (SEQ ID NO: 1011) | UGCCCAUGUCCAGGAAAAGTT (SEQ ID NO: 1453) | CUUUUCCUGGACAUGGGCAT T (SEQ ID NO: 1895) | 1.1 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-64 | GGAAGAGAAGGCGTCACT<br>T<br>(SEQ ID NO: 1012) | GGAAGAGAAGGCGUCACUUTT (SEQ ID NO: 1454) | AAGUGACGCCUUCUCUUCCT<br>T<br>(SEQ ID NO: 1896) | 1.1 |
| RAG3A-1002 | ACCCTCCTTCCTAATCTTG (SEQ ID NO: 1013) | ACCCUCCUUCCUAAUCUUGTT (SEQ ID NO: 1455) | CAAGAUUAGGAAGGAGGGUT<br>T<br>(SEQ ID NO: 1897) | 1.1 |
| RAG3A-1288 | CTGAGGTCGCTCCTCATTC (SEQ ID NO: 1014) | CUGAGGUCGCUCCUCAUUCTT (SEQ ID NO: 1456) | GAAUGAGGAGCGACCUCAGT<br>T<br>(SEQ ID NO: 1898) | 1.1 |
| RAG3A-891 | AGAGCCTGAGGAAGTTCTG (SEQ ID NO: 1015) | AGAGCCUGAGGAAGUUCUGTT (SEQ ID NO: 1457) | CAGAACUUCCUCAGGCUCUT<br>T<br>(SEQ ID NO: 1899) | 1.1 |
| RAG3A-231 | GCAACACAAATGTCCTGCC (SEQ ID NO: 1016) | GCAACACAAAUGUCCUGCCTT (SEQ ID NO: 1458) | GGCAGGACAUUUGUGUUGCT<br>T<br>(SEQ ID NO: 1900) | 1.1 |
| RAG3A-669 | TCACATGTGGAACGGTAGG (SEQ ID NO: 1017) | UCACAUGUGGAACGGUAGGTT (SEQ ID NO: 1459) | CCUACCGUUCCACAUGUGATT<br>(SEQ ID NO: 1901) | 1.1 |
| RAG3A-1009 | TCCCAGCACCCTCCTTCCT (SEQ ID NO: 1018) | UCCCAGCACCCUCCUUCCUTT (SEQ ID NO: 1460) | AGGAAGGAGGGUGCUGGGAT<br>T<br>(SEQ ID NO: 1902) | 1.1 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-656 | GGTAGGAGATGGAAGACG G (SEQ ID NO: 1019) | GGUAGGAGAUGGAAGACGGTT (SEQ ID NO: 1461) | CCGUCUUCCAUCUCCUACCTT (SEQ ID NO: 1903) | 1.1 |
| RAG3A-229 | AACACAAATGTCCTGCCAG (SEQ ID NO: 1020) | AACACAAAUGUCCUGCCAGTT (SEQ ID NO: 1462) | CUGGCAGGACAUUUGUGUUT T (SEQ ID NO: 1904) | 1.1 |
| RAG3A-484 | CCCGTCCAAACCCTTTCCC (SEQ ID NO: 1021) | CCCGUCCAAACCCUUUCCCTT (SEQ ID NO: 1463) | GGGAAAGGGUUUGGACGGGT T (SEQ ID NO: 1905) | 1.1 |
| RAG3A-799 | CTGGGAAGAAAGCAAAGG A (SEQ ID NO: 1022) | CUGGGAAGAAAGCAAAGGATT (SEQ ID NO: 1464) | UCCUUUGCUUUCUUCCCAGT T (SEQ ID NO: 1906) | 1.1 |
| RAG3A-296 | AATTTGGATAGCCAGGGAG (SEQ ID NO: 1023) | AAUUUGGAUAGCCAGGGAGTT (SEQ ID NO: 1465) | CUCCCUGGCUAUCCAAAUUT T (SEQ ID NO: 1907) | 1.1 |
| RAG3A-1183 | TATCCTCAGGCGCATTCGA (SEQ ID NO: 1024) | UAUCCUCAGGCGCAUUCGATT (SEQ ID NO: 1466) | UCGAAUGCGCCUGAGGAUAT T (SEQ ID NO: 1908) | 1.1 |
| RAG3A-380 | CTCCCTAGGGGATTACAGA (SEQ ID NO: 1025) | CUCCCUAGGGGAUUACAGATT (SEQ ID NO: 1467) | UCUGUAAUCCCCUAGGGAGT T (SEQ ID NO: 1909) | 1.1 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-157 | CCATGTCCAGGAAAAGATG (SEQ ID NO: 1026) | CCAUGUCCAGGAAAAGAUGTT (SEQ ID NO: 1468) | CAUCUUUUCCUGGACAUGGT T (SEQ ID NO: 1910) | 1.1 |
| RAG3A-1185 | CTTATCCTCAGGCGCATTC (SEQ ID NO: 1027) | CUUAUCCUCAGGCGCAUUCTT (SEQ ID NO: 1469) | GAAUGCGCCUGAGGAUAAGT T (SEQ ID NO: 1911) | 1.1 |
| RAG3A-1244 | GCCAGAAACCGGCAGCATT (SEQ ID NO: 1028) | GCCAGAAACCGGCAGCAUUTT (SEQ ID NO: 1470) | AAUGCUGCCGGUUUCUGGCT T (SEQ ID NO: 1912) | 1.1 |
| RAG3A-588 | GCCATCTAAGCCACTGGAC (SEQ ID NO: 1029) | GCCAUCUAAGCCACUGGACTT (SEQ ID NO: 1471) | GUCCAGUGGCUUAGAUGGCT T (SEQ ID NO: 1913) | 1.1 |
| RAG3A-436 | TCTCATGTGGGCAATATCC (SEQ ID NO: 1030) | UCUCAUGUGGGCAAUAUCCTT (SEQ ID NO: 1472) | GGAUAUUGCCCACAUGAGAT T (SEQ ID NO: 1914) | 1.1 |
| RAG3A-1029 | ACCCACTATCCTCCCAGCT (SEQ ID NO: 1031) | ACCCACUAUCCUCCCAGCUTT (SEQ ID NO: 1473) | AGCUGGGAGGAUAGUGGGUT T (SEQ ID NO: 1915) | 1.1 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-28 | TGTCTGGTGGCTCCCTTCT (SEQ ID NO: 1032) | UGUCUGGUGGCUCCCUUCUTT (SEQ ID NO: 1474) | AGAAGGGAGCCACCAGACAT T (SEQ ID NO: 1916) | 1.1 |
| RAG3A-234 | CCAGCAACACAAATGTCCT (SEQ ID NO: 1033) | CCAGCAACACAAAUGUCCUTT (SEQ ID NO: 1475) | AGGACAUUUGUGUUGCUGGT T (SEQ ID NO: 1917) | 1.1 |
| RAG3A-249 | CTCTGTGCTTCTTCCCCAG (SEQ ID NO: 1034) | CUCUGUGCUUCUUCCCCAGTT (SEQ ID NO: 1476) | CUGGGGAAGAAGCACAGAGT T (SEQ ID NO: 1918) | 1.1 |
| RAG3A-775 | AGCCAGGGCCAAAGGAAA A (SEQ ID NO: 1035) | AGCCAGGGCCAAAGGAAAATT (SEQ ID NO: 1477) | UUUUCCUUUGGCCCUGGCUT T (SEQ ID NO: 1919) | 1.1 |
| RAG3A-951 | CCAGGATCTAGGCCACACT (SEQ ID NO: 1036) | CCAGGAUCUAGGCCACACUTT (SEQ ID NO: 1478) | AGUGUGGCCUAGAUCCUGGT T (SEQ ID NO: 1920) | 1.1 |
| RAG3A-861 | GATGATGGGATCAAGGTCA (SEQ ID NO: 1037) | GAUGAUGGGAUCAAGGUCATT (SEQ ID NO: 1479) | UGACCUUGAUCCCAUCAUCT T (SEQ ID NO: 1921) | 1.1 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-259 | TAAACAAGACCTCTGTGCT (SEQ ID NO: 1038) | UAAACAAGACCUCUGUGCUTT (SEQ ID NO: 1480) | AGCACAGAGGUCUUGUUUAT T (SEQ ID NO: 1922) | 1.1 |
| RAG3A-394 | ATGACAAGATAGGACTCCC (SEQ ID NO: 1039) | AUGACAAGAUAGGACUCCCTT (SEQ ID NO: 1481) | GGGAGUCCUAUCUUGUCAUT T (SEQ ID NO: 1923) | 1.1 |
| RAG3A-534 | AGTGCACGTGTGCGCACAT (SEQ ID NO: 1040) | AGUGCACGUGUGCGCACAUTT (SEQ ID NO: 1482) | AUGUGCGCACACGUGCACUT T (SEQ ID NO: 1924) | 1.1 |
| RAG3A-316 | TATGATGTCCAGGGAAAAG (SEQ ID NO: 1041) | UAUGAUGUCCAGGGAAAAGTT (SEQ ID NO: 1483) | CUUUUCCCUGGACAUCAUAT T (SEQ ID NO: 1925) | 1.1 |
| RAG3A-560 | CGAGCACCTAAGCTCAGGC (SEQ ID NO: 1042) | CGAGCACCUAAGCUCAGGCTT (SEQ ID NO: 1484) | GCCUGAGCUUAGGUGCUCGT T (SEQ ID NO: 1926) | 1.1 |
| RAG3A-1105 | CCCACTCCCAGCCTCCTTT (SEQ ID NO: 1043) | CCCACUCCCAGCCUCCUUUTT (SEQ ID NO: 1485) | AAAGGAGGCUGGGAGUGGGT T (SEQ ID NO: 1927) | 1.1 |

(continued)

| saRNA | Target sequence (5'-3') | Sense sequence (5'-3') of saRNA | Antisense sequence (5'-3') of saRNA | Fold of changes in relative expression of *THPO* mRNA |
|---|---|---|---|---|
| RAG3A-709 | TTCCTAAGACAGGGACACA (SEQ ID NO: 1044) | UUCCUAAGACAGGGACACATT (SEQ ID NO: 1486) | UGUGUCCCUGUCUUAGGAAT T (SEQ ID NO: 1928) | 1.1 |
| RAG3A-948 | GGATCTAGGCCACACTTCT (SEQ ID NO: 1045) | GGAUCUAGGCCACACUUCUTT (SEQ ID NO: 1487) | AGAAGUGUGGCCUAGAUCCT T (SEQ ID NO: 1929) | 1.1 |
| RAG3A-998 | TCCTTCCTAATCTTGGGAG (SEQ ID NO: 1046) | UCCUUCCUAAUCUUGGGAGTT (SEQ ID NO: 1488) | CUCCCAAGAUUAGGAAGGAT T (SEQ ID NO: 1930) | 1.1 |
| RAG3A-387 | GATAGGACTCCCTAGGGGA (SEQ ID NO: 1047) | GAUAGGACUCCCUAGGGGATT (SEQ ID NO: 1489) | UCCCCUAGGGAGUCCUAUCT T (SEQ ID NO: 1931) | 1.1 |

It can be clearly seen from the arrangement of activities of the 833 saRNAs according to their positions within the *THPO* promoter region that the functional saRNAs are gathered together, that is, in certain promoter regions, the activating saRNAs gathered in specific "hotspot" regions **(FIG. 11).** As shown in **FIG. 11,** 5 hotspot regions appeared in region (H1) from -1339 to -1044, region (H2) from -1027 to -903, region (H3) from -861 to -754, region (H4) from -728 to -611, and region (H5) from -593 to -1 of the promoter, respectively, showing high gathering of the activating saRNAs. These results indicate that the activating saRNAs were not randomly distributed within the promoter, but existed in the specific hotspot regions.

The sequence of the hotspot H1 (5' to 3': -1339 to -1044) corresponds to position 1 to position 296, from 5' to 3', of SEQ ID NO: 601 in the sequence listing:

1 caaaccctca ccaccctctc tcactgccta gcctgcctcc ctaccttctc
51 tctgaggtcg ctcctcattc ttgtgttacc caggacaggg acctagccag
101 aaaccggcag cattccccct tctgtggagt gacagtatct ccctctcatt
151 gtaacttatc ctcaggcgca ttcgacagtc ccctcttgct ttctcacccc
201 cttccttcac ccaagggacc ctctgcctct ccagcccact cccagcctcc
251 tttctcttgg ttccctggtc atgcctgcct ccctgtctcc tgtctc

The sequence of the hotspot H2 (5' to 3': -1027 to -903) corresponds to position 1 to position 125, from 5' to 3', of SEQ ID NO: 602 in the sequence listing:

1 ccactatcct cccagctatc ccagcaccct ccttcctaat cttgggagac
51 atctcgtctg gctggacggg aaaattccag gatctaggcc acacttctca
101 gcagacatgc ccatccttgg ggagg

The sequence of the hotspot H3 (5' to 3': -861 to -754) corresponds to position 1 to position 108, from 5' to 3', of SEQ ID NO: 603 in the sequence listing:

1 gatgatggga tcaaggtcag gccaggaagc ccctgaggac agagactgtg
51 gggagactgg gactgggaag aaagcaaagg agctagagcc agggccaaag
101 gaaaaggg

The sequence of the hotspot H4 (5' to 3': -728 to -611) corresponds to position 1 to position 118, from 5' to 3', of SEQ ID NO: 604 in the sequence listing:

1 ggaggtccag cagctgtctt tcctaagaca gggacacatg ggcctggtta
51 ttcctcttgt cacatgtgga acggtaggag atggaagacg gagacagaac
101 aagcaaagga gggccctg

The sequence of the hotspot H5 (5' to 3': -593 to -1) corresponds to position 1 to position 593, from 5' to 3', of SEQ ID NO: 605 in the sequence listing:

1 gtgtagccat ctaagccact ggaccccagc agacgagcac ctaagctcag
51 gcttaaccca gtgcacgtgt gcgcacatac atgtgccccg cacctgacag
101 tccactcaac ccgtccaaac cctttcccca taacaccaac ccataacagg
151 agatttctct catgtgggca atatccgtgt tcccacttcg aaagggggaa
201 tgacaagata ggactcccta ggggattaca gaaagaaaag caggaaagca
251 agcatcctgt tggatttcag cagcaggtat gatgtccagg gaaaagaaat
301 ttggatagcc agggagtgaa aaccccacca atcttaaaca agacctctgt
351 gcttcttccc cagcaacaca aatgtcctgc cagattcctc ctggaaaaaa
401 acttctgctc ctgtcccccct ccaggtccca ggttgcccat gtccaggaaa
451 agatggatcc ccctatccaa atcttctccg tggtgtgtgt gggtggagga
501 gtggaccctg gtccaggcag gggctccagg gaagagaagg cgtcacttcc
551 gggggccttc accagtgtct ggtggctccc ttctctgatt ggg

Example 10: Human *THPO* saRNA Promoting the Expression of *THPO* mRNA in Human Liver Cancer Cells (HepG2)

**(1) Cell culture and transfection**

Cell culture was described in Example 9. HepG2 cells (purchased from Shanghai Institutes for Biological Sciences, TCHu119) were plated into a 6-well plate at $2\times10^5$ cells/well. Following the instructions provided by the manufacturer, RNAiMax (Invitrogen, Carlsbad, CA) was used to transfect human *THPO* saRNA at a concentration of 25 nM (unless otherwise specified), and transfection duration was 72 h, Each treatment was performed in 2 replicate wells.

**(2) Two-step RT-qPCR**

Media were discarded after transfection and 500 μL of cell lysis buffer was added to each well and incubated at room temperature for 5 min. RNA was extracted using Qiagen RNeasy kit, reverse transcribed, and subjected to qPCR analysis on a ABI 7500 fast real-time PCR system (Applied Biosystems). Each sample was amplified repeatedly in 3 replicate wells. RT-PCR and qPCR reaction systems are shown in Example 5, Tables 5-6 and the amplification primers are shown in Table 11. *TBP* and *HPRT1* served as internal controls, and *THPO* was amplified using a *THPO* F1/R1 primer pair.

The reaction conditions were as follows: 30s at 95°C, 5 s at 95°C, 30s at 60°C, with 40 cycles of amplification.

To calculate the expression value ($E_{rel}$) of the *THPO* gene (target) of an saRNA-transfected sample relative to control treatment (Mock), the Ct values of the target gene and two internal control genes were substituted into Formula 2 in Example 3 for calculation.

$$E_{rel} = 2^{(CtTm-CtTs)} / 2^{(CtRm-CtRs)}$$

***Formula 2***

**FIG. 12** shows the relative mRNA expression values of *THPO* after treatment of cells with different saRNAs. The relative mRNA expression of siRNA group (RAG3A-365i) is reduced by 85% compared to the control group which uses a blank transfection control (Mock), indicating that siRNA was successful as a small interfering RNA control transfection. The relative mRNA expression values of RAG3A-1219, RAG3A-1096, RAG3A-1308, RAG3A-1069 and RAG3A-851 groups were all higher than those of the control group, and the relative mRNA expression values increased 1.8, 1.8, 1.6, 1.7 and 1.9, respectively. The relative mRNA expression values of RAG3A-541, RAG3A-1319, RAG3A-62, RAG3A-889 and RAG3A-787 groups were all higher than those of the control group, and increased 2.5, 2.1, 2.9, 2.0 and 2.0, respectively. The activation effect of RAG3A-62 was evident, and the relative mRNA expression value was increased by nearly 3 times. This indicates that randomly selected activating saRNAs could not only be transfected successfully into HepG2 cells, but also can significantly increased expression of*THPO* mRNA.

Example 11: Human *THPO* saRNA Promoting the Expression of THPO Protein Level in Human Liver Cancer Cells (HepG2)

Cell culture methods were described in Example 9 and cell transfection methods were described in Example 10. Assay of THPO protein level in HepG2 cell culture media using ELISA Kit

1) Human THPO protein standard was dissolved using deionized water or distilled water to give a stock solution of 20,000 pg/mL protein standard. One hundred microliters of THPO protein standard stock solution was added to 900 μL of calibration diluent RD6-11 (dilution 1:5) (for cell culture supernatant samples) or calibration diluent RD6-11 (for serum/plasma samples) to give 2000 pg/mL of standard solution, of which 7 serial dilutions were performed with the appropriate calibration diluent as the zero standard (0 pg/mL).

2) Cell supernatants were sampled and centrifuged to remove dead cells and debris.

3) Proper amount of micro-well plate belts from micro-well plate frame were taken out.

4) 50 μL of assay diluent RD1-1 was added to each well.

5) 200 μL of standard, control, or treatment samples were added to each well. The membrane was covered and the samples were incubated at room temperature for 3 h.

6) The liquid was removed from each well and washed 4 times with 400 μL of wash buffer.

7) 200 μL of human THPO antibody was added to each well and incubated for 1h at room temperature.

8) Step 5 was repeated.

9) 200 μL of substrate solution (Color reagents A and B mixed in equal volumes and used up within 15 min) was added to each well and incubated for 30 min at room temperature in the absence of light.

10) 50 μL of stop solution was added to each well and mixed gently.

11) Absorbance at 450 nM was determined using a multi-functional microplate reader (within 30 min).

FIG. 13 shows the expression value of relative protein level of THPO in cell culture media after treatment of cells with different saRNAs. The relative protein expression of siRNA group (DS3A-365i) group is reduced by 81% compared to the control group which uses a blank transfection control, indicating that siRNA was successful as a small interfering RNA control transfection. The relative protein level expression of THPO of RAG3A-723, RAG3A-892, RAG3A-62, RAG3A-1178, RAG3A-889, RAG3A-563, RAG3A-487 and RAG3A-787 groups were higher than those of the control group, and the relative protein level expression of THPO was increased by 1.46, 1.26, 1.44, 1.31, 1.29, 1.32, 1.42 and 1.40, respectively. This indicates that randomly selected activating saRNAs are capable of promoting the expression of THPO protein to levels of varying degrees in HepG2 cells.

Based on the above results, a plurality of saRNAs capable of remarkably activating the expression of human and mouse *THPO*/*Thpo* genes were found through high-throughput screening of saRNAs targeting human and mouse *THPO*/*Thpo* gene promoters. It has been found by applicants from multiple studies that both mouse active saRNAs targeting the *Thpo* gene and human active saRNAs targeting the *THPO* gene could be efficiently delivered into cells by the liposome system used in the present invention and can significantly up-regulate the expression of the *Thpo*/*THPO* gene and protein in cells at the mRNA and protein level. In particular, the active saRNA of the *Thpo* gene of mouse significantly increases the expression of *Thpo* mRNA and protein in the mouse and simultaneously up-regulates or increases the number of platelets significantly. Therefore, the mouse *Thpo* active saRNA and the human *THPO* active saRNA of the present invention can be used for treating a disease or condition caused by a decrease or insufficient Thpo/THPO protein expression or a disease or condition caused by various factors such as thrombocytopenia, or in the preparation of a drug for treating the aforementioned disease or condition.

References

Bartley, T. D., J. Bogenberger, P. Hunt, Y. S. Li, H. S. Lu, F. Martin, M. S. Chang, B. Samal, J. L. Nichol, S. Swift, and et al. 1994. 'Identification and cloning of a megakaryocyte growth and development factor that is a ligand for the cytokine receptor Mpl', Cell, 77: 1117-24.

Basser, R. 2002. 'The impact of thrombopoietin on clinical practice', Curr Pharm Des, 8: 369-77. de Sauvage, F. J., P. E. Hass, S. D. Spencer, B. E. Malloy, A. L. Gurney, S. A. Spencer, W. C. Darbonne, W. J. Henzel, S. C. Wong, W. J. Kuang, and et al. 1994. 'Stimulation of megakaryocytopoiesis and thrombopoiesis by the c-Mpl ligand', Nature, 369: 533-8.

Hassan, M. N., and E. K. Waller. 2015. 'Treating chemotherapy-induced thrombocytopenia: is it time for oncologists to use thrombopoietin agonists?', Oncology (Williston Park), 29: 295-6.

Kaushansky, K. 2006. 'Lineage-specific hematopoietic growth factors', N Engl J Med, 354: 2034-45.

Kuter, D. J. 2009. 'Thrombopoietin and thrombopoietin mimetics in the treatment of thrombocytopenia', Annu Rev Med, 60: 193-206.

Laki, K. 1972. 'Our ancient heritage in blood clotting and some of its consequences', Ann N Y Acad Sci, 202: 297-307.

Machlus, K. R., J. N. Thon, and J. E. Italiano, Jr. 2014. 'Interpreting the developmental dance of the megakaryocyte: a review of the cellular and molecular processes mediating platelet formation', Br J Haematol, 165: 227-36.

Mignotte, V., I. Vigon, E. Boucher de Crevecoeur, P. H. Romeo, V. Lemarchandel, and S. Chretien. 1994. 'Structure and transcription of the human c-mpl gene (MPL)', Genomics, 20: 5-12.

Provan, D., R. Stasi, A. C. Newland, V. S. Blanchette, P. Bolton-Maggs, J. B. Bussel, B. H. Chong, D. B. Cines, T. B. Gernsheimer, B. Godeau, J. Grainger, I. Greer, B. J. Hunt, P. A. Imbach, G. Lyons, R. McMillan, F. Rodeghiero, M. A. Sanz, M. Tarantino, S. Watson, J. Young, and D. J. Kuter. 2010. 'International consensus report on the investigation and management of primary immune thrombocytopenia', Blood, 115: 168-86.

Seo, A., M. Ben-Harosh, M. Sirin, J. Stein, O. Dgany, J. Kaplelushnik, M. Hoenig, U. Pannicke, M. Lorenz, K.

Schwarz, C. Stockklausner, T. Walsh, S. Gulsuner, M. K. Lee, A. Sendamarai, M. Sanchez-Bonilla, M. C. King, H. Cario, A. E. Kulozik, K. M. Debatin, A. Schulz, H. Tamary, and A. Shimamura. 2017. 'Bone marrow failure unresponsive to bone marrow transplant is caused by mutations in thrombopoietin', Blood, 130: 875-80.

Solberg, L. A., Jr. 2005. 'Biologic aspects of thrombopoietins and the development of therapeutic agents', Curr Hematol Rep, 4: 423-8.

## Claims

1. A method for treating thrombocytopenia and/or a disease and condition related to insufficient or decreased expression of THPO protein in a subject, comprising administering to the subject a curative dose of a small activating nucleic acid molecule targeting a promoter of *THPO,* a nucleic acid molecule encoding the small activating nucleic acid molecule, or a composition or formulation comprising the small activating nucleic acid molecule.

2. The method of claim 1, wherein the thrombocytopenia comprises thrombocytopenia caused by various causes, such as congenital (hereditary) or acquired thrombocytopenia, preferably thrombocytopenia caused by myelo-thrombocytopenia, increased peripheral platelet destruction, or splenic sequestration.

3. The method of claim 1, wherein the thrombocytopenia includes, but is not limited to, thrombocytopenia related to insufficient or decreased expression of THPO protein, thrombocytopenia caused by various causes such as immune thrombocytopenia, drug-induced thrombocytopenia, thrombotic thrombocytopenia purpura, or hereditary thrombocytopenia.

4. The method of any one of claims 1 to 3, wherein the individual is a mammal.

5. The method of claim 4, wherein the individual is a human.

6. The method of any one of claims 1 to 3, wherein the small activating nucleic acid molecule comprises a first nucleic acid strand and a second nucleic acid strand, the first nucleic acid strand having at least 75% (e.g., at least about 79%, about 80%, about 85%, about 90%, about 95%, about 99% or about 100%) homology or complementarity to continuous 16 to 35 nucleotides in length of any one of SEQ ID NO: 601 to SEQ ID NO: 605, and the first nucleic acid strand and the second nucleic acid strand complementarily form a double-stranded nucleic acid structure capable of activating the expression of *THPO* gene in a cell.

7. The method of claim 6, wherein the first nucleic acid strand of the small activating nucleic acid molecule comprises or is selected from any nucleotide sequence selected from SEQ ID NOs: 1048 to SEQ ID NOs: 1489, and the second nucleic acid strand comprises or is selected from any nucleotide sequence selected from SEQ ID NOs: 1490 to SEQ ID NOs: 1931.

8. The method of claim 6, wherein the first nucleic acid strand and the second nucleic acid strand are present on two different nucleic acid strands.

9. The method of claim 6, wherein the first nucleic acid strand and the second nucleic acid strand are present on the same nucleic acid strand, preferably, the small activating nucleic acid molecule is a hairpin single-stranded nucleic acid molecule, wherein the first nucleic acid strand and the second nucleic acid strand comprise complementary regions forming a double-stranded nucleic acid structure.

10. The method of claim 1, wherein at least one strand of the small activating nucleic acid molecule has an overhang of 0 to 6 nucleotides in length at 3' terminus.

11. The method of claim 10, wherein both strands of the small activating nucleic acid molecule have an overhang of 0 to 6 nucleotides in length at the 3' terminus, preferably an overhang of 2 or 3 nucleotides in length.

12. The method of claim 6 or 7, wherein the first nucleic acid strand and the second nucleic acid strand independently have 16 to 35 nucleotides in length.

13. The method of any of claims 1 to 12, wherein the small activating nucleic acid molecule comprises at least one modification, preferably, the modification is a chemical modification.

**14.** The method of claim 13, wherein the chemical modification comprises or is selected from one or more modifications selected from the group consisting of:

(1) modification of a phosphodiester bond connecting nucleotides in the nucleotide sequence of the small activating nucleic acid molecule;
(2) modification of 2'-OH of a ribose in the nucleotide sequence of the small activating nucleic acid molecule;
(3) modification of a base in the nucleotide sequence of the small activating nucleic acid molecule; and
(4) at least one nucleotide in the nucleotide sequence of the small activating nucleic acid molecule being a locked nucleic acid.

**15.** The method of claim 13, wherein the chemical modification comprises one or more rmodifications selected from the group consisting of: 2'-fluoro modification, 2'-oxymethyl modification, 2'-oxyethylidene methoxy modification, 2,4'-dinitrophenol modification, locked nucleic acid (LNA), 2'-amino modification, 2'-deoxy modification, 5'-bromouracil modification, 5'-iodouracil modification, N-methyluracil modification, 2,6-diaminopurine modification, phosphorothioate modification, and boranophosphate modification.

**16.** The method of claim 3, wherein the acquired thrombocytopenia includes, but is not limited to, thrombocytopenia caused by aplastic anemia, myelodysplastic syndrome, leukemia, drugs, infection, tumor diseases, radiotherapy, bone marrow transplantation and chronic liver diseases, and immune thrombocytopenia, wherein the factors causing increased peripheral platelet destruction comprise thrombotic thrombocytopenia, heparin-induced thrombocytopenia, drug-induced thrombocytopenia, ITP, and thrombotic thrombocytopenia purpura.

**17.** A method for activating/up-regulating the expression of *THPO* gene in a subject or a cell, comprising administering a small activating nucleic acid molecule of the present invention, a nucleic acid coding the small activating nucleic acid molecule of the present invention, or a composition or formulation comprising the small activating nucleic acid molecule of the present invention to the subject or the cell.

**18.** A nucleic acid coding the small activating nucleic acid molecule of any one of claims 1 to 9.

**19.** A cell comprising the small activating nucleic acid molecule of any one of claims 1 to 9 or a nucleic acid encoding the small activating nucleic acid molecule of any one of claims 1 to 9.

**20.** A composition comprising the small activating nucleic acid molecule of any one of claims 1 to 9.

**21.** A formulation comprising the small activating nucleic acid molecule of any one of claims 1 to 9, wherein the formulation comprises a liposome, preferably an LNP-entrapped small activating nucleic acid molecule.

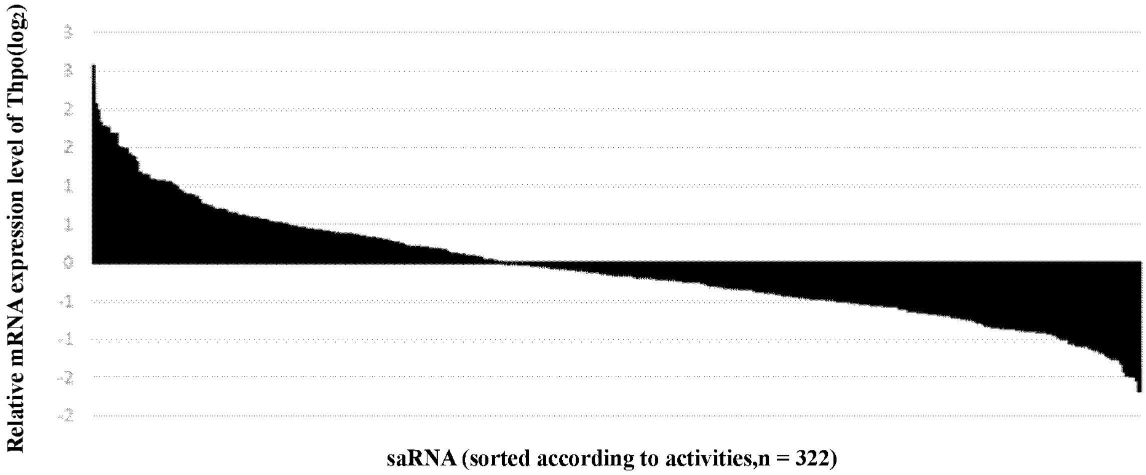


FIG. 1

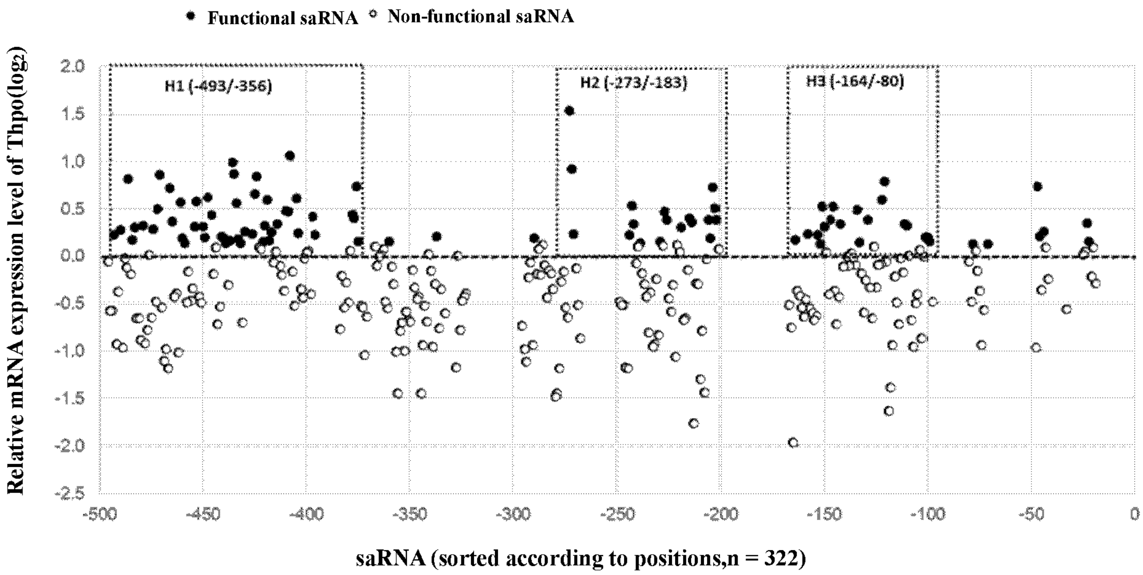


FIG. 2

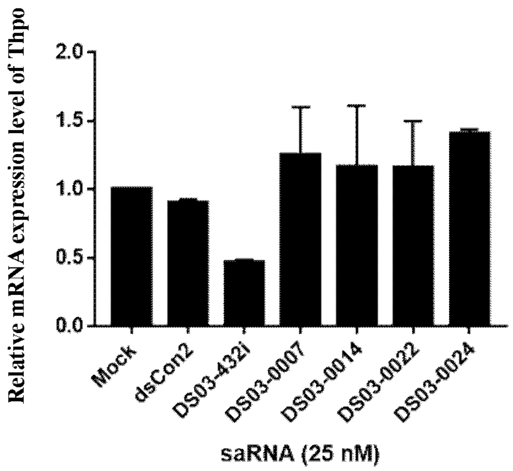


FIG. 3

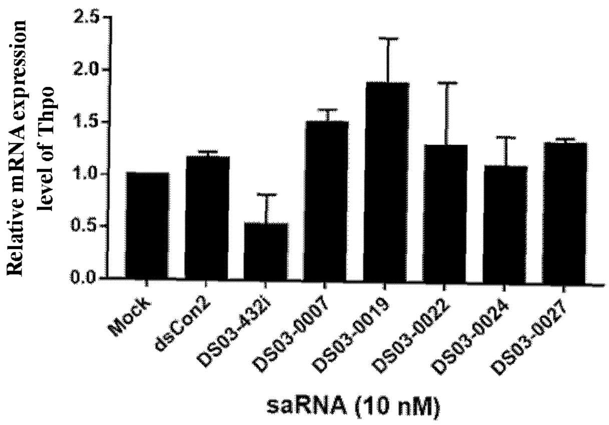


FIG. 4

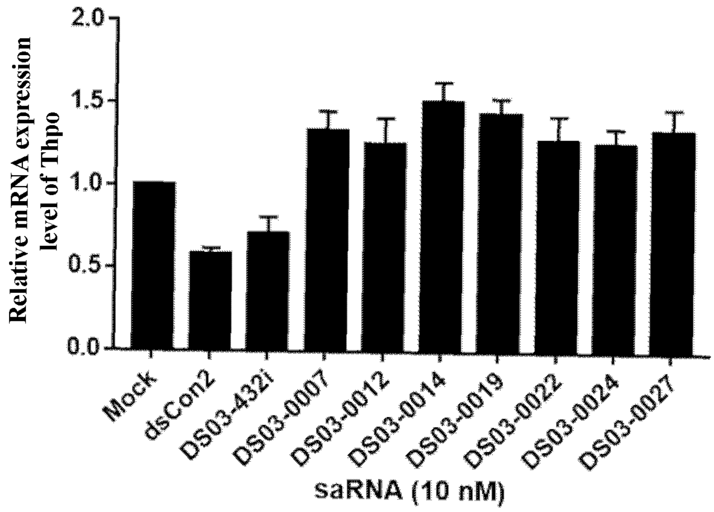


FIG. 5

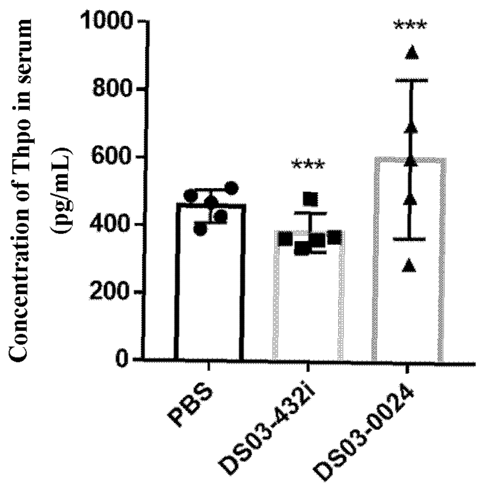


FIG. 6

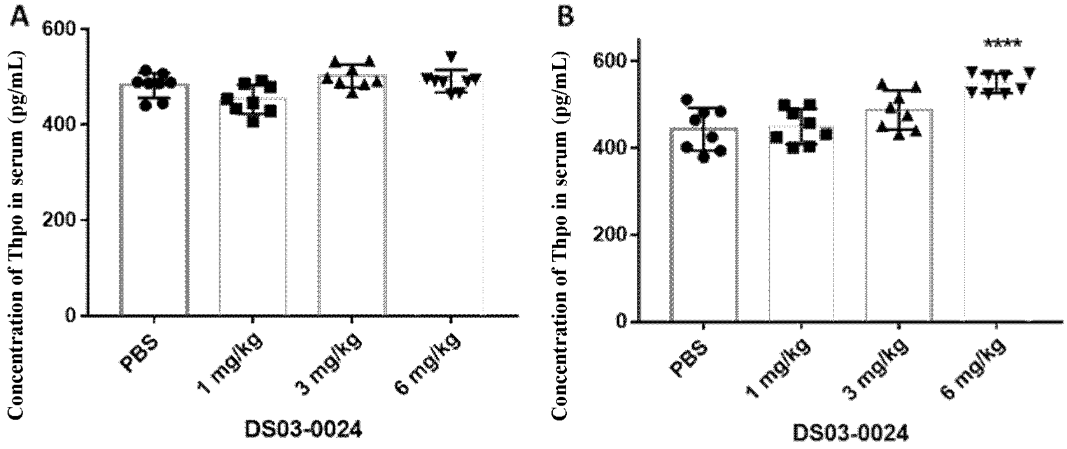


FIG. 7

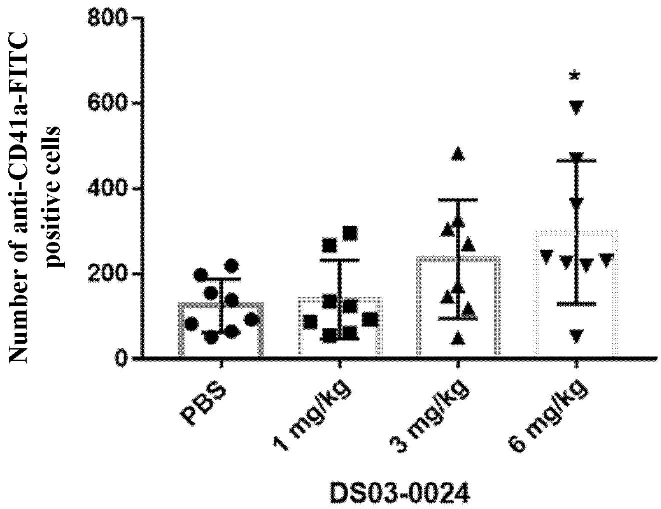


FIG. 8

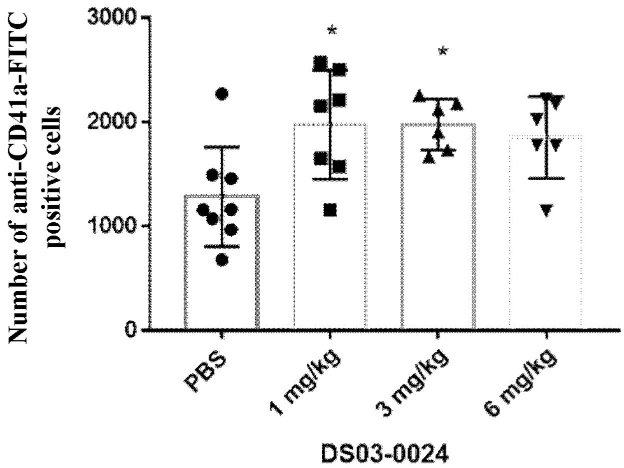


FIG. 9

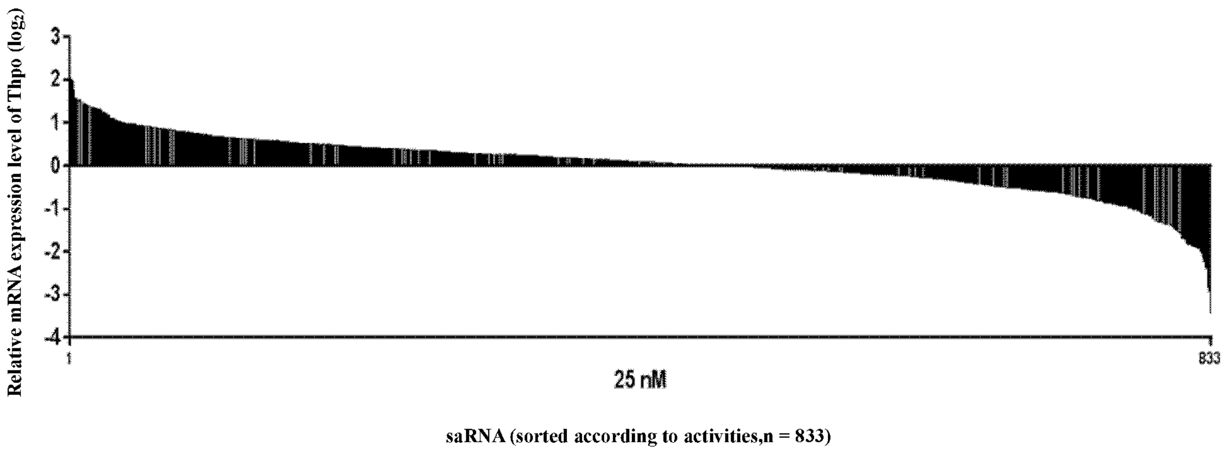


FIG. 10

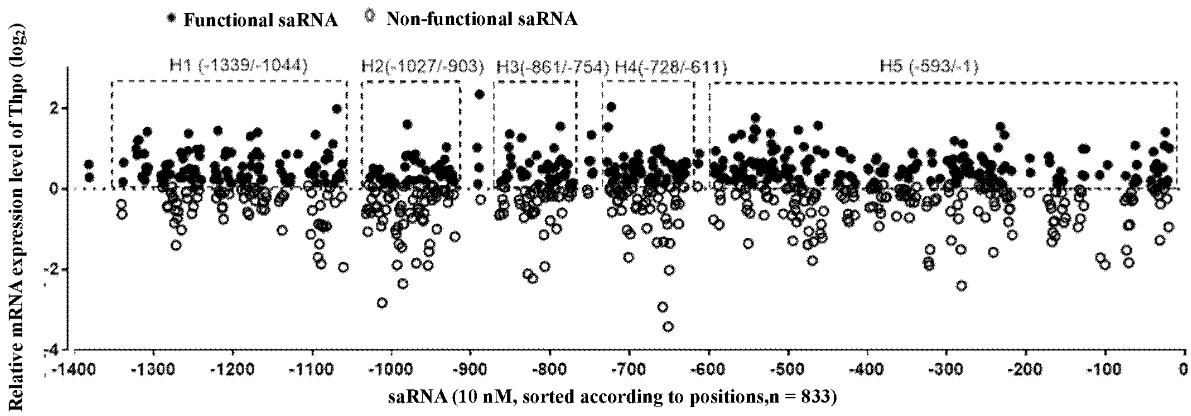


FIG. 11

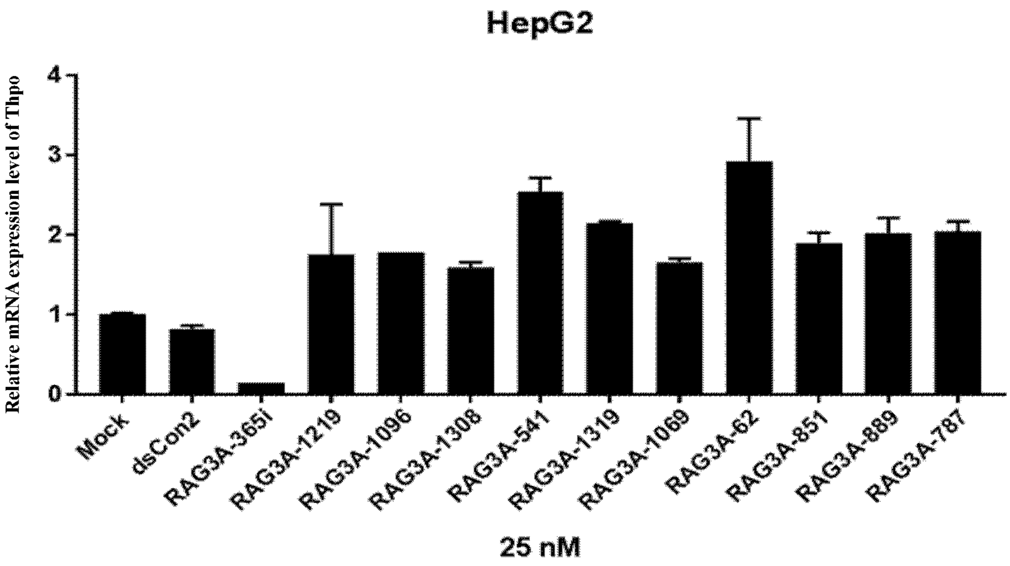


FIG. 12

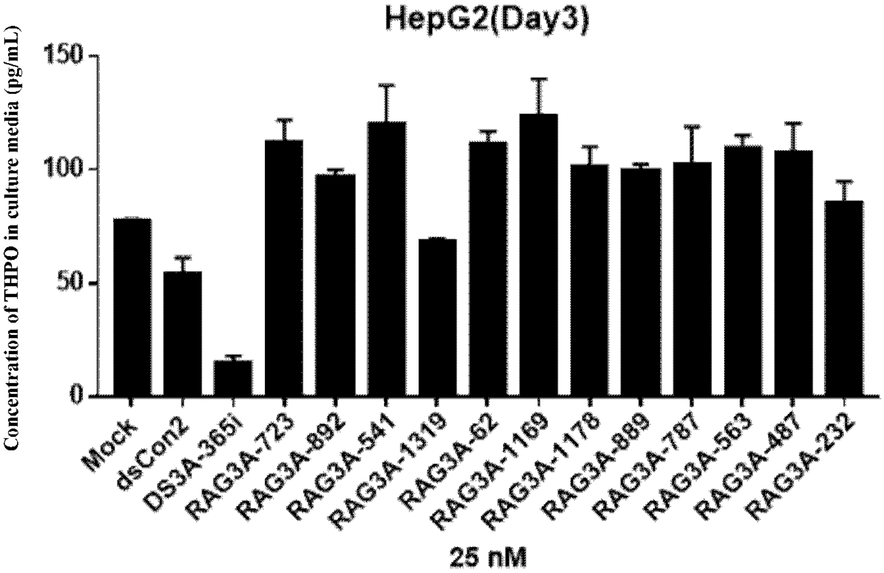
HepG2(Day3)
Concentration of THPO in culture media (pg/mL)
150
100
50
0
Mock
dsCon2
DS3A-365i
RAG3A-723
RAG3A-892
RAG3A-541
RAG3A-1319
RAG3A-62
RAG3A-1169
RAG3A-1178
RAG3A-889
RAG3A-787
RAG3A-563
RAG3A-487
RAG3A-232
25 nM

FIG. 13

**INTERNATIONAL SEARCH REPORT**

International application No.
**PCT/CN2020/116227**

**A. CLASSIFICATION OF SUBJECT MATTER**

C12N 15/113(2010.01)i； C12N 15/85(2006.01)i； A61P 7/04(2006.01)i

According to International Patent Classification (IPC) or to both national classification and IPC

**B. FIELDS SEARCHED**

Minimum documentation searched (classification system followed by classification symbols)

C12N; A61P

Documentation searched other than minimum documentation to the extent that such documents are included in the fields searched

Electronic data base consulted during the international search (name of data base and, where practicable, search terms used)

数据库: CNABS, CNKI, DWPI, SIPOABS, CNTXT, WOTXT, USTXT, EPTXT, ISI Web of Knowledge, PUBMED, 百度学术, EMBL+GenBank+DDBJ, 中国专利生物序列检索系统; 关键词: 促血小板生成素, Thrombopoietin, THPO, TPO, 血小板, thrombocyte, 激活, 上调, 巨核细胞生长和发育因子, megakaryocyte growth and development factor, MGDF, C-MPL配体, 小激活核酸分子, 小激活RNA, 活化RNA, saRNA , RNAa, 高通量, 筛选, 对说明书中人THPO启动子序列的检索

**C. DOCUMENTS CONSIDERED TO BE RELEVANT**

| Category* | Citation of document, with indication, where appropriate, of the relevant passages | Relevant to claim No. |
|---|---|---|
| Y | CN 103842508 A (MINA THERAPEUTICS LTD.) 04 June 2014 (2014-06-04)<br>see claims 1-13 | 18-21 |
| Y | CN 106032532 A (PEKING UNION MEDICAL COLLEGE HOSPITAL, CHINESE ACADEMY OF MEDICAL SCIENCES) 19 October 2016 (2016-10-19)<br>see claims 1-3 | 18-21 |
| Y | CN 106480028 A (SHANGHAI SEVENTH PEOPLE'S HOSPITAL) 08 March 2017 (2017-03-08)<br>see description, paragraphs [0008] to [0020] | 18-21 |
| A | CN 1141061 A (GENENTECH INC.) 22 January 1997 (1997-01-22)<br>see entire document | 18-21 |
| Y | Dordelmann C et al. "accession number: NG_012136.1"<br>*GenBank*, 04 August 2019 (2019-08-04),<br>see sequence list, and related information | 18-21 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 December 2020** | **24 February 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/116227**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **1-17**
because they relate to subject matter not required to be searched by this Authority, namely:

[1] Relating to a method for treatment of the living human or animal body, and thus belonging to subject matter which does not require a search under PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2020/116227**

| Patent document cited in search report | Publication date (day/month/year) | Patent family member(s) | Publication date (day/month/year) |
|---|---|---|---|
| CN 103842508 A | 04 June 2014 | CN 103842508 B | 24 February 2016 |
| | | JP 2019115345 A | 18 July 2019 |
| | | US 10704044 B2 | 07 July 2020 |
| | | US 2018201932 A1 | 19 July 2018 |
| | | US 9284553 B2 | 15 March 2016 |
| | | US 9944930 B2 | 17 April 2018 |
| | | EP 3456828 A1 | 20 March 2019 |
| | | US 9745579 B2 | 29 August 2017 |
| | | EP 2723862 B1 | 19 September 2018 |
| | | US 2014228424 A1 | 14 August 2014 |
| | | JP 2017035083 A | 16 February 2017 |
| | | JP 5993002 B2 | 14 September 2016 |
| | | JP 2014519838 A | 21 August 2014 |
| | | KR 20140039307 A | 01 April 2014 |
| | | JP 6742459 B2 | 19 August 2020 |
| | | KR 20190125504 A | 06 November 2019 |
| | | EP 2723862 A1 | 30 April 2014 |
| | | US 2016145618 A1 | 26 May 2016 |
| | | WO 2012175958 A1 | 27 December 2012 |
| | | JP 6483645 B2 | 13 March 2019 |
| | | KR 102039315 B1 | 04 November 2019 |
| | | US 2017321215 A1 | 09 November 2017 |
| CN 106032532 A | 19 October 2016 | None | |
| CN 106480028 A | 08 March 2017 | CN 106480028 B | 05 April 2019 |
| CN 1141061 A | 22 January 1997 | CA 2178482 A1 | 13 July 1995 |
| | | JP H10113186 A | 06 May 1998 |
| | | DK 149194 A | 04 July 1995 |
| | | IT TO950002 D0 | 02 January 1995 |
| | | NL 9500010 A | 01 August 1995 |
| | | GB 2285446 B | 28 July 1999 |
| | | LU 88573 A1 | 01 June 1995 |
| | | AU 704266 B2 | 15 April 1999 |
| | | GB 2285446 A | 12 July 1995 |
| | | IE 72517 B1 | 23 April 1997 |
| | | SG 47030 A1 | 20 March 1998 |
| | | CZ 192296 A3 | 14 May 1997 |
| | | NO 962783 L | 03 September 1996 |
| | | PT 101627 B | 29 November 1996 |
| | | SK 282265 B6 | 03 December 2001 |
| | | NO 962783 A | 03 September 1996 |
| | | CN 1134539 C | 14 January 2004 |
| | | IT TO950002 A1 | 03 July 1995 |
| | | PT 101627 A | 30 November 1995 |
| | | ES 2114786 B1 | 01 January 1999 |
| | | FR 2714670 A1 | 07 July 1995 |
| | | RO 117110 B1 | 30 October 2001 |
| | | NZ 278726 A | 26 June 1998 |
| | | IE 950002 A1 | 12 July 1995 |
| | | NO 962783 D0 | 02 July 1996 |
| | | FI 121573 B | 14 January 2011 |

Form PCT/ISA/210 (patent family annex) (January 2015)

**INTERNATIONAL SEARCH REPORT**
**Information on patent family members**

International application No.
**PCT/CN2020/116227**

| Patent document cited in search report | Publication date (day/month/year) | Patent family member(s) | | | Publication date (day/month/year) |
|---|---|---|---|---|---|
| | | CZ | 9601922 | A3 | 14 May 1997 |
| | | CA | 2178482 | C | 03 January 2012 |
| | | EP | 0738323 | A1 | 23 October 1996 |
| | | NO | 326903 | B1 | 16 March 2009 |
| | | BG | 63639 | B1 | 31 July 2002 |
| | | IT | 1283770 | B1 | 30 April 1998 |
| | | WO | 9518858 | A1 | 13 July 1995 |
| | | HR | P941020 | A2 | 30 April 1997 |
| | | LV | 11632 | B | 20 April 1997 |
| | | IL | 112184 | A | 25 July 2004 |
| | | BG | 100693 | A | 30 January 1998 |
| | | GB | 9425831 | D0 | 22 February 1995 |
| | | SK | 87596 | A3 | 05 November 1997 |
| | | AU | 1514695 | A | 01 August 1995 |
| | | HU | 9601805 | D0 | 28 August 1996 |
| | | LV | 11632 | A | 20 December 1996 |
| | | SI | 9420079 | A | 28 February 1997 |
| | | FR | 2714670 | B1 | 10 January 1997 |
| | | HU | T75657 | A | 28 May 1997 |
| | | CZ | 296130 | B6 | 11 January 2006 |
| | | IL | 112184 | D0 | 15 March 1995 |
| | | ES | 2114786 | A1 | 01 June 1998 |
| | | FI | 962723 | A | 03 September 1996 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 104873464 B
- CN 102625696 B
- US 9394234 B2
- WO 2010144740 A1
- US 8158601 B2
- US 8802644 B2
- US 20100324120 A1


### Non-patent literature cited in the description


- **SAMBROOK et al.** Molecular Cloning: Laboratory Manual. Cold Spring Harbor Laboratory Press, 1989
- **BARTLEY, T. D. ; J. BOGENBERGER ; P. HUNT ; Y. S. LI ; H. S. LU ; F. MARTIN ; M. S. CHANG ; B. SAMAL ; J. L. NICHOL ; S. SWIFT et al.** Identification and cloning of a megakaryocyte growth and development factor that is a ligand for the cytokine receptor Mpl. *Cell,* 1994, vol. 77, 1117-24
- **BASSER, R.** The impact of thrombopoietin on clinical practice. *Curr Pharm Des,* 2002, vol. 8, 369-77
- **DE SAUVAGE, F. J. ; P. E. HASS ; S. D. SPENCER ; B. E. MALLOY ; A. L. GURNEY ; S. A. SPENCER ; W. C. DARBONNE ; W. J. HENZEL ; S. C. WONG ; W. J. KUANG et al.** Stimulation of megakaryocytopoiesis and thrombopoiesis by the c-Mpl ligand. *Nature,* 1994, vol. 369, 533-8
- **HASSAN, M. N. ; E. K. WALLER.** Treating chemotherapy-induced thrombocytopenia: is it time for oncologists to use thrombopoietin agonists?. *Oncology (Williston Park),* 2015, vol. 29, 295-6
- **KAUSHANSKY, K.** Lineage-specific hematopoietic growth factors. *N Engl J Med,* 2006, vol. 354, 2034-45
- **KUTER, D. J.** Thrombopoietin and thrombopoietin mimetics in the treatment of thrombocytopenia. *Annu Rev Med,* 2009, vol. 60, 193-206
- **LAKI, K.** Our ancient heritage in blood clotting and some of its consequences. *Ann N Y Acad Sci,* 1972, vol. 202, 297-307
- **MACHLUS, K. R. ; J. N. THON ; J. E. ITALIANO, JR.** Interpreting the developmental dance of the megakaryocyte: a review of the cellular and molecular processes mediating platelet formation. *Br J Haematol,* 2014, vol. 165, 227-36
- **MIGNOTTE, V. ; I. VIGON ; E. BOUCHER DE CREVECOEUR ; P. H. ROMEO ; V. LEMARCHANDEL ; S. CHRETIEN.** Structure and transcription of the human c-mpl gene (MPL). *Genomics,* 1994, vol. 20, 5-12
- **PROVAN, D. ; R. STASI ; A. C. NEWLAND ; V. S. BLANCHETTE ; P. BOLTON-MAGGS ; J. B. BUSSEL ; B. H. CHONG ; D. B. CINES ; T. B. GERNSHEIMER ; B. GODEAU.** International consensus report on the investigation and management of primary immune thrombocytopenia. *Blood,* 2010, vol. 115, 168-86
- **SEO, A. ; M. BEN-HAROSH ; M. SIRIN ; J. STEIN ; O. DGANY ; J. KAPLELUSHNIK ; M. HOENIG ; U. PANNICKE ; M. LORENZ ; K. SCHWARZ.** Bone marrow failure unresponsive to bone marrow transplant is caused by mutations in thrombopoietin. *Blood,* 2017, vol. 130, 875-80
- **SOLBERG, L. A., JR.** Biologic aspects of thrombopoietins and the development of therapeutic agents. *Curr Hematol Rep,* 2005, vol. 4, 423-8